# EUROPEAN PATENT SPECIFICATION

(11) **EP 4 081 210 B1**
(45) Date of publication and mention of the grant of the patent: **15.04.2026**
(21) Application number: 20839398.3
(22) Date of filing: 26.12.2020
(51) Int. Cl.: A61K 31/353, A61K 31/5375, A61K 31/538, A61K 31/195, A61K 31/137, A61P 5/20, A61P 13/12

(54) **PHARMACEUTICAL COMPOSITION OF CASR MODULATORS AND METHODS AND USES THEREOF**
PHARMAZEUTISCHE ZUSAMMENSETZUNG VON CASR-MODULATOREN UND VERFAHREN UND IHRE VERWENDUNG
COMPOSITION PHARMACEUTIQUE DE MODULATEURS DE CASR ET PROCÉDÉS ET UTILISATIONS ASSOCIÉS

(30) Priority: 27.12.2019 IN 201921054138; 13.02.2020 IN 202021006208
(43) Date of publication of application: 02.11.2022
(73) Proprietor: Lupin Limited, Mumbai 400 055 (IN)
(72) Inventor: KAMBOJ, Rajender Kumar, Pune, Maharashtra 412115 (IN); BAKHLE, Dhananjay Sadashiv, Pune, Maharashtra 412115 (IN); SHAH, Chirag Anilkumar, Pune, Maharashtra 412115 (IN)
(74) Representative: Adamson Jones
(86) International application number: PCT/IN2020/051057
(87) International publication number: WO 2021/130779

(56) References cited:
- WO-A1-2012/120476
- WO-A1-2012/127385
- WO-A1-2013/124828
- WO-A1-2014/033604
- WO-A1-2015/022631
- WO-A1-2015/028938
- WO-A1-2015/136329

## Description

### CROSS REFERENCE TO THE RELATED APPLICATIONS

This PCT application claims the benefit in and to Indian Provisional Patent Application Nos. 201921054138, filed December 27, 2019, and 202021006208, filed February 13, 2020.

### FIELD

This disclosure relates to solid pharmaceutical compositions comprising a pharmaceutically effective amount of one or more CaSR agonists, wherein the one or more CaSR agonists is one or more compounds of Formula (I), Formula (II), Formula (III), Formula (IV), Formula (V), Formula (VI), including Compound 1 to Compound 6 disclosed below, pharmaceutically acceptable salts thereof, or a combination thereof, and methods of their use for the treatment of diseases or disorders associated with the modulation of calcium sensing receptors (CaSRs), including secondary hyperparathyroidism associated with chronic kidney disease in a subject in need thereof. The solid pharmaceutical compositions can be administered orally to a subject in need thereof. This disclosure further relates to a process for the preparation of said pharmaceutical compositions.

### BACKGROUND

The following includes information that may be useful in understanding the invention. It is not an admission that any of the information, publications or documents specifically or implicitly referenced herein is prior art, or essential, to the described or claimed invention.

Ca²⁺ is known to be an intracellular second messenger, with the molecular identification of an extracellular calcium sensing receptor (CaSR). The possibility that Ca2+ might also function as a messenger outside the cells has been further opened. Information about the local changes in extracellular concentration of Ca²⁺ is conveyed to the interior of many types of cells through this unique receptor.

Calcium-sensing receptor (CaSR) is a G-protein-coupled receptor (GPCR) that signals through the activation of phospholipase C, increasing levels of inositol 1,4,5-triphosphate and cytosolic calcium. The CaSR belongs to the subfamily C of the GPCR superfamily. Structurally, CaSR has an exceptionally large amino-terminal extracellular (ECD) domain (about 600 amino acids), a feature that is shared by all of the members of the family C GPCRs.

In mammals, the expression of CaSR is quite ubiquitous and its presence in the parathyroid gland plays an important role in the secretion of parathyroid hormone (PTH). PTH is involved in the homeostasis of bone metabolism by regulating the level of calcium in the blood, release of calcium from the bone, absorption of calcium from the intestine and excretion of calcium in the urine (Tomasello S., "Secondary hyperparathyroidism and chronic kidney disease. Diabetes Spectrum," 21:19-25 (2008)). Secondary hyperparathyroidism (SHPT) is characterized by excessive secretion of PTH (Silverberg SJ & Bilezikian JP, "The diagnosis and management of asymptomatic primary hyperparathyroidism," Nature Clin. Pract. Endocrinol. Metab., 2:494-503 (2006); Goodman WG, "Recent developments in the management of secondary hyperparathyroidism," Kidney Inter., 59:1187-1201 (2001)) and affects almost 90% of patients with end stage renal disease and 40% to 60% of patients with chronic kidney disease (CKD). This results in an increase in serum phosphate and decrease in vitamin D and calcium levels, ultimatelycausing high PTH levels which can cause bone disorders, calcification of soft tissues and blood vessels and significant risk for cardiovascular morbidity (OPKO HEALTH Renal Division, "Improving people's lives by treating and preventing the clinical consequences of vitamin D insufficiency, secondary hyperparathyroidism, and hyperphosphatemia," Fact sheet CY (2015)).

The reduction in serum calcium leads to the secretion of PTH. Consequently, PTH secretion leads to conservation of serum Ca²⁺ by increasing kidney retention and intestinal absorption of Ca²⁺. Also, increase in extracellular calcium levels activate the CaSR resulting in decreased PTH secretion. This happens indirectly through the PTH-induced synthesis of the active vitamin D metabolite, 25-dihydroxyvitamin D. An integrated hormonal system controls calcium transport in the gut, kidneys and bones to maintain calcium homeostasis (Peacock M., "Calcium metabolism in health and disease," Clin. J. Am .Soc. Nephrol., 5:S23-S30 (2010); Carmeliet et al., "Disorders of calcium homeostasis," Best Pract. Res. Clin. Endocrinol. Metab.. 17:529-546 (2003)). A decrease in serum calcium inactivates the CaSR in the parathyroid glands, increasing PTH secretion which acts on the kidneys to increase calcium reabsorption and in bones to release skeletal calcium. The PTH also causes the kidneys to synthesize 1,25-dihydroxy vitamin D3, which increases calcium absorption in the gut, while decreasing further PTH secretion and increasing bone resorption. This regulation of PTH secretion by calcium is altered in SHPT (Carmeliet et al., "Disorders of calcium homeostasis," Best Pract. Res. Clin. Endocrinol. Metab.. 17:529-546 (2003); Tfelt-Hansen et al., "The calcium-sensing receptor in human disease," Front Biosci., 8:s377-390 (2003)). Accordingly, in chronic kidney disease patients it is important to manage intact PTH (iPTH) levels as well as those of serum calcium and phosphorus.

In addition, the pulsatile action of PTH has anabolic effects on bone development and its sustained levels can lead to catabolic effects, in which the bones break down releasing Ca²⁺ as in the case of osteoporosis. All these systems converge in maintenance of baseline serum Ca²⁺ and it involves a tight regulation between serum PTH and extracellular calcium which is mediated by the remarkable receptor CaSR.

In conditions such as primary and secondary hyperparathyroidism, there is excessive secretion of parathyroid hormone due to hyperplasia of the glands. The most common cause of primary hyperparathyroidism (PHPT) is parathyroid adenoma resulting from clonal mutations (~97%) and associated hypercalcemia. In the case of secondary hyperparathyroidism (SHPT), it is most commonly seen in patients with chronic renal failure. The kidneys fail to convert enough vitamin D to its active form and also does not adequately excrete phosphorous. Excess phosphorous further depletes serum calcium forming calcium phosphate (kidney stones) leading to hypocalcemia.

Small molecules that are positive allosteric modulators referred to as calcimimetics modulate and improve the receptors sensitivity to the already existing milieu of extracellular ionic calcium. This would eventually translate in lowering plasma PTH levels thereby improving conditions of hyperparathyroidism, calcium homeostasis and bone metabolism.

PCT International Patent Application Publication Nos. WO 2012/127388, WO 2012/120476, WO 2012/127385, WO 2012/069421, WO 2012/069419, WO 2012/069402, US 2011/0028452, WO 2010/150837, WO 2010/136037, WO 2010/042642, WO 2010/038895, WO 2009/065406, WO 2008/059854, WO 2006/123725, WO 2004/106280, WO 2004/069793, WO 2002/012181 and US 2003/0199497 refer to compounds related to calcium sensing receptors (CaSR) for the treatment of various diseases mediated by CaSR. Kessler et al., "N1-Benzoyl-N2-[1-(1-naphthyl)ethyl]-trans-1,2-diaminocyclohexanes: Development of 4-Chlorophenylcarboxamide (Calhex 231) as a New Calcium Sensing Receptor Ligand Demonstrating Potent Calcilytic Activity," J. Med. Chem. (2006), 49, 5119-5128 also discloses compounds related CaSR

As stated above, small molecules, termed "calcimimetics," mimic (directly activate the receptor) or potentiate (positive allosteric modulators) the effects of extracellular calcium at the CaSR. Cinacalcet was the first United States Food and Drug Administration (FDA) approved calcimimetic for treating SHPT in patients with CKD receiving dialysis (Stage 5 CKD) and hypercalcaemia in patients with parathyroid carcinoma (SENSIPAR^{®} (Cinacalcet) Prescribing Information, Amgen Inc., revised March 2019; Padhi, D. & Harris R., "Clinical pharmacokinetic and pharmacodynamic profile of Cinacalcethydrochloride," Clin Pharmacokinet., 48:303-311 (2009); Quarles LD, "Cinacalcet HCl: A novel treatment for secondary hyperparathyroidism in stage 5 chronic kidney disease," Kidney Inter., 68:S24-S28 (2005)). Another calcimemetic, Etelcalcetide (PARSABIV^{®}), approved for the treatment of SHPT in 2017 in the United States and European Union, is a second-generation calcimimetic agent for intravenous use developed to improve adherence. Among other current therapies for SHPT, phosphate binders have a risk of cardiovascular diseases and newer vitamin D sterols have a risk of hypercalcaemia and provide inefficient control. Second generation calcimimetic agents suppress PTH levels with lower calcium and phosphorus product and have a risk of hypocalcaemia, which is thought to occur after decreased mobilisation of calcium from the bone caused by reduced PTH levels (Cunningham et al., "Review secondary hyperparathyroidism: pathogenesis, disease progression, and therapeutic options," Clin. J. Am. Soc. Nephrol., 6:913-921 (2011)).

Current treatments for SHPT differ in duration of PTH suppression and modes of administration, and have distinctly different side effect profiles that impact patient preferences and compliance. Cinacalcet requires daily oral therapy to control SHPT, and its major side effects are nausea and vomiting and are critically responsible for low adherence and insufficient dosage (Gincherman et al., "Assessment of adherence to cinacalcet by prescription refill rates in hemodialysis patients," Hemodialysis International,14:68-72 (2010)). Cinacalcet, has wide variation in doses (30mg, 60 mg, 90 mg, 120 mg and 180 mg) and require a dose titration strategy in which the dose is progressively increased to the therapeutic dose for treating or managing secondary hyperparathyroidism associated with CKD in a patient on dialysis. Dose titration is generally used to enhance tolerability and efficacy, but the process can also be associated with negative outcomes. For example, the incidence of relapse or recurrence are higher in patients whose doses are titrated since there is increased patient discontinuation rate associated with titration. Patients who participate in dose titration processes experience frustration and delayed therapeutic relief due to complicated schedules and/or delays in achieving the therapeutic dosage. Such dose titration also increases healthcare costs for patients undergoing titration because they required to make more physician visits and need more prescriptions, often leading to greater use of resources and higher costs associated with physicians and laboratory monitoring. Also, patients who are far from or have transportation, mobility or financial difficulties to visit healthcare providers and/or facilities find it difficult to participate and adhere to such dose titration processes. In addition, such patients who experience side effects during a dose titration process and/or are not given an exact time of when they will reach therapeutic dosage are more likely to abandon the process altogether. Further, patients are not guaranteed that they will reach therapeutic dosage via a dose titration process.

Thus, there is a need in the art to identify a novel drug for treating or controlling SHPT that can suppress PTH with minor effect on calcium and phosphate metabolism. Also, given the limitations of dose titration and observed side effects of current treatments available and the requirement of positive/favorable benefit: risk ratio therapy, there remains a significant need to develop methods of treating or managing, medicaments, compositions and kits having a simple dosage regimen that requires no dose titration, and causes fewer GI symptoms for the treatment or management of SHPT, primary hyperparathyroidism (PHPT) and hypercalcaemia in patients with parathyroid carcinoma in a patient in need thereof.

### SUMMARY

The present invention provides a solid pharmaceutical composition comprising a pharmaceutically effective amount of one or more compounds selected from compounds of formulae (I) - (VI) as set out in the appended claims. The present invention also provides the solid pharmaceutical compositions for use in treating secondary hyperparathyroidism associated with chronic kidney disease (CKD) and for use in lowering or suppressing one or more intact parathyroid hormone (iPTH) levels in a subject suffering from secondary hyperparathyroidism associated with CKD, as set out in the appended claims.

In accordance with a first aspect, the present invention provides a solid pharmaceutical composition comprising a pharmaceutically effective amount of one or more compounds, wherein the one or more compounds is selected from the group consisting of compounds having the structure of Formula (I), Formula (II), Formula (III), Formula (IV), Formula (V), or Formula (VI): 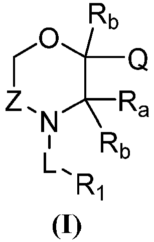 wherein
Q is 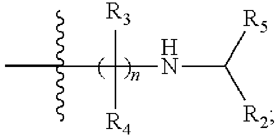
Rₐ is hydrogen;
R_{b} is hydrogen;
L is a bond or -(CR_{c}R_{d})ₘ;
R_{c} and R_{d} are independently selected from hydrogen or substituted or unsubstituted alkyl;
R₁ is 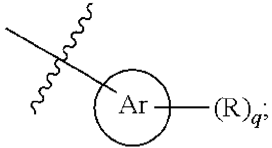
ring Ar is phenyl or naphthyl;
R, which may be same or different at each occurrence, is independently selected from halogen, nitro, cyano, substituted or unsubstituted alkyl, substituted or unsubstituted alkenyl, substituted or unsubstituted alkynyl, substituted or unsubstituted haloalkyl, -OR₆, -C(O)R₆, -(CRₑR_{f})₀₋₃-C(O)OR₆, -(CRₑR_{f})₁₋₂cycloalkylene-C(O)OR₆, - cycloalkylene (CRₑR_{f})₀₋₂-C(O)OR₆, -O(CRₑR_{f})₀₋₃-C(O)OR₆, -O-cycloalkylene-C(O)OR₆, -C(O)NR₇-(CRₑR_{f})₁₋₂-C(O)OR₆, -C(O)NR₇R₈, -S(O)₀₋₂R₆, and - S(O)₂NR₇R₈;
Rₑ and R_{f} are independently hydrogen or substituted or unsubstituted alkyl;
R₂ is substituted or unsubstituted aryl;
R₃ and R₄ are independently selected from hydrogen, halogen, and substituted or unsubstituted alkyl;
R₅ is substituted or unsubstituted alkyl or haloalkyl;
R₆, which may be same or different at each occurrence, is independently selected from hydrogen, substituted or unsubstituted alkyl and substituted or unsubstituted haloalkyl;
R₇ and R₈, which may be same or different at each occurrence, are independently selected from hydrogen, substituted or unsubstituted alkyl, substituted or unsubstituted cycloalkyl, and substituted or unsubstituted aryl;
Z is -CR_{g}Rₕ-;
R_{g} and Rₕ are hydrogen;
"m" is an integer ranging from 1 to 3, both inclusive; "n" is an integer ranging from 1 to 3, both inclusive; and "q" is an integer ranging from 0 to 4, both inclusive; 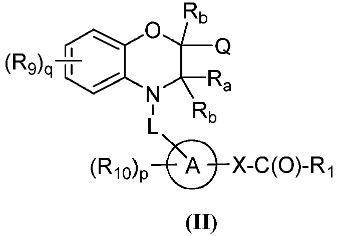
wherein
Q is 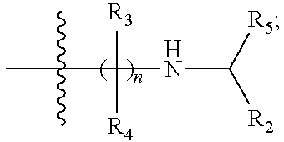
Rₐ is hydrogen, halogen or alkyl;
R_{b} is selected from hydrogen, alkyl, and haloalkyl;
or Rₐ and R_{b} together attached on the same carbon form C(O);
L is a bond or -CR_{c}R_{d}-;
ring A is phenyl;
R_{c} and R_{d} are independently selected from hydrogen, halogen, and alkyl;
X is selected from a bond, -(CRₑR_{f})ₘ, -O-, -O(CRₑR_{f})ₘ-, -(CRₑR_{f})ₘO-, - C(O)(CRₑR_{f})ₘ-, -C(O)NR₇-, -C(O)NR₇(CRₑR_{f})ₘ-, -cycloalkylene-, and -O-cycloalkylene-;
Rₑ and R_{f}, which may be same or different at each occurrence, are independently selected from hydrogen, halogen, alkyl, haloalkyl and cycloalkyl; or Rₑ and R_{f}, together with the carbon atom to which they are attached, may form a 3 to 7 membered saturated carbocyclic ring;
R₁ is -OR₆ or -NR₇R₈;
R₂ is substituted or unsubstituted aryl;
R₃ and R₄ are independently selected from hydrogen, halogen, alkyl, haloalkyl, and cycloalkyl;
R₅ is alkyl or haloalkyl;
R₆ is hydrogen or alkyl;
R₇ and R₈ are independently selected from hydrogen, alkyl, cycloalkyl cycloalkylalkyl and aryl;
R₉, which may be same or different at each occurrence, is independently selected from halogen, cyano, alkyl, haloalkyl, and cycloalkyl;
R₁₀, which may be same or different at each occurrence, is independently selected from halogen, cyano, alkyl, alkenyl, alkynyl, haloalkyl, hydroxyalkyl, -OR₆, - C(O)R₆, -NR₇C(O)R₆, -S(O)₀₋₂R₆, -S(O)₂NR₇R₈, and -NR₇S(O)₂R₆;
"m" is an integer ranging from 1 to 3, both inclusive; "n" is an integer ranging from 1 to 3, both inclusive; "p" is an integer ranging from 0 to 2, both inclusive; and "q" is an integer ranging from 0 to 1, both inclusive; 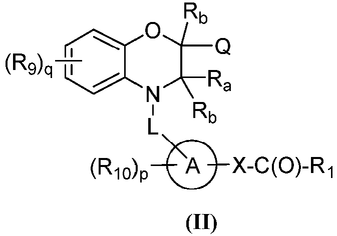
wherein Q is 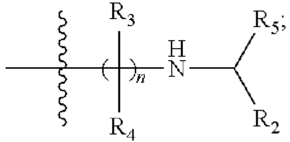
Rₐ is hydrogen; R_{b} is hydrogen or alkyl;
L is a bond, or -CR_{c}R_{d};
ring A is phenyl;
R_{c} and R_{d} are independently selected from hydrogen, halogen, and alkyl;
X is selected from a bond, -(CRₑR_{f})ₘ, -O-, -O(CRₑR_{f})ₘ-, -(CRₑR_{f})ₘO-, - C(O)(CRₑR_{f})ₘ-, -C(O)NR₇-, and -C(O)NR₇(CRₑR_{f})ₘ-;
Rₑ and R_{f}, which may be same or different at each occurrence, are independently selected from hydrogen, halogen, alkyl, haloalkyl and cycloalkyl; or Rₑ and R_{f}, together with the carbon atom to which they are attached, form a 3 to 7 membered saturated carbocyclic ring;
R₁ is -OR₆ or -NR₇R₈;
R₂ is phenyl or naphthyl, wherein the phenyl is substituted with halogen, alkyl, haloalkyl, alkoxy or haloalkoxy;
R₃ and R₄ are hydrogen;
R₅ is alkyl;
R₆ is hydrogen or alkyl;
R₇ and R₈ are hydrogen or alkyl;
R₉ is independently selected from halogen, cyano, alkyl, haloalkyl, and cycloalkyl;
R₁₀, which may be same or different at each occurrence, is independently selected from halogen, cyano, alkyl, haloalkyl, hydroxyalkyl, -OR₆, -C(O)R₆, -NR₇R₈, - NR₇C(O)R₆, and -(O)₂NR₇R₈;
"m" is an integer ranging from 1 to 3, both inclusive; "n" is an integer ranging from 1 to 3, both inclusive; "p" is an integer ranging from 0 to 2, both inclusive; "q" is an integer ranging from 0 to 1, both inclusive; 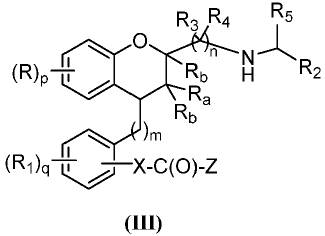
wherein
Rₐ is selected from hydrogen, halogen, substituted or unsubstituted alkyl, cyano, substituted or unsubstituted cycloalkyl and substituted or unsubstituted haloalkyl;
R_{b}, which may be same or different at each occurrence, is independently selected from hydrogen, halogen, substituted or unsubstituted alkyl, substituted or unsubstituted cycloalkyl and substituted or unsubstituted haloalkyl;
R_{c} which may be same or different at each occurrence, is independently selected from halogen, hydroxy, substituted or unsubstituted alkyl, substituted or unsubstituted haloalkyl, substituted or unsubstituted alkenyl, substituted or unsubstituted alkynyl, substituted or unsubstituted cycloalkyl, OR₆, nitro, cyano, -C(O)OR₆, -(CH₂)ᵣ-C(O)OR₆, -O-C(O)OR₆, -O(CH₂)ᵣ-C(O)OR₆, -NR₇R₈, -(CH₂)ᵣNR₇R₈-, - C(O)R₉, -C(O)NR₇R₈, -(CH₂)ᵣ-C(O)NR₇R₈, -NR₇C(O)R₉, -S(O)₀₋₂R₆, - S(O)₂NR₇R₈, and -NR₇S(O)₂R₉;
X is selected from a bond, -(CR_{c}R_{d})ᵣ-, -O-, -NR₇-, -NR₇(CR_{c}R_{d})ᵣ-, - O(CR_{c}R_{d})ᵣ, -C(O)NR₇-, -C(O)NR₇(CRₑR_{d})ᵣ, -(CR_{c}R_{d})ᵣNR₇(CR_{c}R_{d})ᵣ, - (CR_{c}R_{d})ᵣcycloalkylene-, cycloalkylene, -cycloalkylene(CR_{c}R_{d})ᵣ- and -O-cycloalkylene where cycloalkylene may be substituted or unsubstituted;
R_{c} and R_{d}, which may be same or different at each occurrence, are independently selected from hydrogen, halogen, hydroxy, cyano, nitro, substituted or unsubstituted alkyl, substituted or unsubstituted haloalkyl and substituted or unsubstituted cycloalkyl; or R_{c} and R_{d}, together with the carbon atom to which they are attached, may form a substituted or unsubstituted 3 to 7 membered saturated carbocyclic ring;
Z is -OR₆ or -NR₁₀R₁₁;
R₁, which may be same or different at each occurrence, is independently selected from halogen, nitro, cyano, substituted or unsubstituted alkyl, substituted or unsubstituted alkenyl, substituted or unsubstituted alkynyl, substituted or unsubstituted haloalkyl, substituted or unsubstituted cycloalkyl, -OR₆, -C(O)R₉, - NR₇R₈, -(CH₂)ᵣNR₇R₈-, -(CH₂)ᵣ-C(O)OR₆, -O-C(O)OR₆, -O(CH₂)ᵣ-C(O)OR₆, -C(O)NR₇R₈, -(CH₂)ᵣ-C(O)NR₇R₈, -NR₇C(O)R₉, -S(O)₀₋₂R₇, - S(O)₂NR₇R₈ and -NR₇S(O)₂R₉;
R₂ is selected from substituted or unsubstituted aryl, substituted or unsubstituted heteroaryl and substituted or unsubstituted heterocyclyl;
R₃ and R₄ may be same or different and are independently selected from hydrogen, halogen, substituted or unsubstituted alkyl, substituted or unsubstituted haloalkyl, substituted or unsubstituted alkenyl, substituted or unsubstituted alkynyl, substituted or unsubstituted alkoxy, substituted or unsubstituted haloalkoxy and substituted or unsubstituted cycloalkyl;
R₅ is substituted or unsubstituted alkyl;
R₆, which may be same or different at each occurrence, is independently selected from hydrogen, substituted or unsubstituted alkyl, substituted or unsubstituted haloalkyl, substituted or unsubstituted alkenyl, substituted or unsubstituted alkynyl, and substituted or unsubstituted aryl;
R₇ and R₈, which may be same or different at each occurrence, are independently selected from hydrogen, substituted or unsubstituted alkyl, substituted or unsubstituted alkenyl, substituted or unsubstituted alkynyl, substituted or unsubstituted cycloalkyl, substituted or unsubstituted cycloalkylalkyl, substituted or unsubstituted aryl, substituted or unsubstituted arylalkyl, substituted or unsubstituted heteroaryl, substituted or unsubstituted heteroarylalkyl, substituted or unsubstituted heterocyclyl, and substituted or unsubstituted heterocyclylalkyl; or R₇ and R₈, together with the nitrogen atom to which they are attached, may form a substituted or unsubstituted, saturated or unsaturated 3 to 12 membered cyclic ring, wherein the unsaturated cyclic ring may have one or two double bonds;
at each occurrence, R₉ is substituted or unsubstituted alkyl or substituted or unsubstituted aryl;
R₁₀ and R₁₁ may be same or different and are independently selected from hydrogen, substituted or unsubstituted alkyl, substituted or unsubstituted alkenyl, substituted or unsubstituted alkynyl, -(CR_{c}R_{d})ᵣ-C(O)OR₆, substituted or unsubstituted cycloalkyl, substituted or unsubstituted cycloalkylalkyl, substituted or unsubstituted aryl, substituted or unsubstituted arylalkyl, substituted or unsubstituted heteroaryl, substituted or unsubstituted heteroarylalkyl, substituted or unsubstituted heterocyclyl, and substituted or unsubstituted heterocyclylalkyl; or R₁₀ and R₁₁, together with the nitrogen atom to which they are attached, may form a substituted or unsubstituted, saturated or unsaturated 3 to 12 membered cyclic ring, wherein the unsaturated cyclic ring may have one or two double bonds;
"n" is an integer ranging from 1 to 3, both inclusive; "m" is an integer ranging from 0 to 3, both inclusive; "p" is an integer ranging from 0 to 4, both inclusive; "q" is an integer ranging from 0 to 3, both inclusive; and "r" is an integer ranging from 1 to 3, both inclusive; 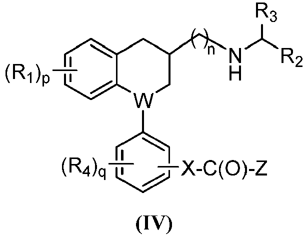
wherein
W is CH or N;
R₁, which may be same or different at each occurrence, is independently selected from halogen, nitro, cyano, substituted or unsubstituted alkyl, substituted or unsubstituted haloalkyl, substituted or unsubstituted cycloalkyl, -C(O)OR₅, - (CRₐR_{b})ᵣ-C(O)OR₅, -O-C(O)OR₅, -O(CRₐR_{b})ᵣ-C(O)OR₅, -NR₆R₇, - C(O)R₈, -C(O)NR₆R₇, -NR₆C(O)R₈, -S(O)₀₋₂R₅, -S(O)₂NR₆R₇ and - NR₆S(O)₂R₈;
R₂ is substituted or unsubstituted aryl;
R₃ is substituted or unsubstituted alkyl;
R₄, which may be same or different at each occurrence, is independently selected from halogen, cyano, substituted or unsubstituted alkyl, substituted or unsubstituted haloalkyl, -OR₅, -NR₆R₇, -C(O)R₈, -C(O)NR₆R₇, -NR₆C(O)R₈, -S(O)₀₋₂R₅, -S(O)₂NR₆R₇ and -NR₅S(O)₂R₈;
X is selected from a bond, -(CRₐR_{b})ᵣ-, -O-, -NR₇-, -O(CRₐR_{b})ᵣ-, - C(O)NR₇-, -C(O)NR₇(CRₐR_{b})ᵣ-, -(CRₐR_{b})ᵣcycloalkylene-, cycloalkylene, cycloalkylene-(CRₐR_{b})ᵣ- and -O-cycloalkylene;
Rₐ and R_{b}, which may be same or different at each occurrence, are independently selected from hydrogen, halogen, hydroxy, cyano, substituted or unsubstituted alkyl, substituted or unsubstituted haloalkyl and substituted or unsubstituted cycloalkyl; or Rₐ and R_{b}, together with the carbon atom to which they are attached, may form a substituted or unsubstituted 3 to 7 membered saturated carbocyclic ring;
Z is -OR₅ or -NR₆R₇;
R₈, which may be same or different at each occurrence, is independently selected from hydrogen, substituted or unsubstituted alkyl, substituted or unsubstituted haloalkyl and substituted or unsubstituted aryl;
R₆ and R₇, which may be same or different at each occurrence, are independently selected from hydrogen, substituted or unsubstituted alkyl, -(CRₐR_{b})ᵣ-C(O)OR₅, substituted or unsubstituted cycloalkyl, substituted or unsubstituted cycloalkylalkyl, substituted or unsubstituted aryl, substituted or unsubstituted arylalkyl, substituted or unsubstituted heteroaryl, substituted or unsubstituted heteroarylalkyl, substituted or unsubstituted heterocyclyl and substituted or unsubstituted heterocyclylalkyl; or R₆ and R₇, together with the nitrogen atom to which they are attached, may form a substituted or unsubstituted, saturated or unsaturated 3 to 10 membered cyclic ring, wherein the unsaturated cyclic ring may have one or two double bonds;
R₅ is substituted or unsubstituted alkyl or substituted or unsubstituted aryl;
"n" is an integer ranging from 0 to 3, both inclusive; "p" is an integer ranging from 0 to 3, both inclusive; "q" is an integer ranging from 0 to 3, both inclusive; and "r" is an integer ranging from 1 to 3, both inclusive; 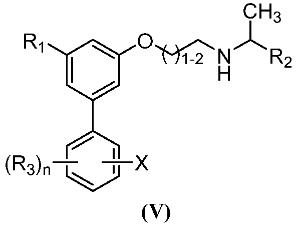
wherein
R₁ is selected from halogen, substituted or unsubstituted alkyl, substituted or unsubstituted haloalkyl, substituted or unsubstituted cycloalkyl, substituted or unsubstituted alkoxy, substituted or unsubstituted haloalkoxy, -(CRₐR_{b})₁-₃OH, - C(O)NH-alkyl, -S(O)₂- alkyl, -S(O)₂NH-alkyl, -C(O)OH, -C(O)O-alkyl, -(CRₐR_{b})₁₋₃C(O)OH and -(CRₐR_{b})₁₋₃C(O)O- alkyl;
R₂ is substituted or unsubstituted phenyl or substituted or unsubstituted naphthyl, wherein the substituents may be one or more and are independently selected from halogen, substituted or unsubstituted alkyl, substituted or unsubstituted haloalkyl, - OR₄, -NR₅R₆ and substituted or unsubstituted cycloalkyl;
X is selected from -C(O)OH, -C(O)O-alkyl, -C(O)NR₅R₆, -(CRₐR_{b})₁₋₃C(O)OH, - C(O)O-alkyl, -O-(CRₐR_{b})1₋₃C(O)OH, -O-(CRₐR_{b})₁₋₃C(O)O-alkyl, -CR_{C}=CR_{C}- - C(O)OH, halogen, substituted or unsubstituted alkyl, substituted or unsubstituted haloalkyl,- OR₄ and -S(O)₂-alkyl;
Rₐ and R_{b} are independently selected from hydrogen, halogen, hydroxy, substituted or unsubstituted alkyl, substituted or unsubstituted haloalkyl and substituted or unsubstituted cycloalkyl; or Rₐ and R_{b ,} together with the carbon atom to which they are attached, may form a substituted or unsubstituted 3 to 6 membered saturated carbocyclic ring;
R_{c} is independently selected from hydrogen, halogen and substituted or unsubstituted alkyl;
R₃, which may be same or different at each occurrence, is independently selected from halogen, cyano, substituted or unsubstituted alkyl, substituted or unsubstituted haloalkyl and -OR₄;
R₄ is selected from hydrogen, substituted or unsubstituted alkyl, substituted or unsubstituted haloalkyl and substituted or unsubstituted cycloalkyl;
R₅ and R₆ are independently selected from hydrogen, substituted or unsubstituted alkyl and substituted or unsubstituted cycloalkyl; and "n" is an integer ranging from 0 to 2, both inclusive; 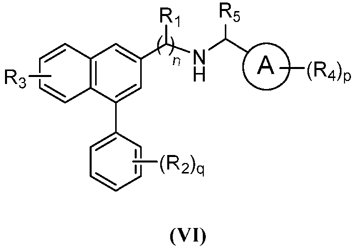
wherein
ring A is phenyl or naphthyl;
R₁ is hydrogen or substituted or unsubstituted (C₁-C₆)alkyl;
R₂, which may be same or different at each occurrence, is independently selected from halogen, cyano, substituted or unsubstituted (C₁-C₆)alkyl, substituted or unsubstituted (C₁-C₆)haloalkyl, substituted or unsubstituted (C₁-C₆)hydroxyalkyl, - X-C(O)-Z, -OR₉, -NR₇R₈, -NR₇C(O)R₆, -S(O)₀₋₂R₆, -S(O)₂NR₇R₈, - NR₇S(O)₂R₆, substituted or unsubstituted (C₃-C₁₂) cycloalkyl, substituted or unsubstituted phenyl, substituted or unsubstituted 5- to 6-membered heteroaryl, substituted or unsubstituted 5- to 6-membered heterocyclyl and ring D;
ring D is 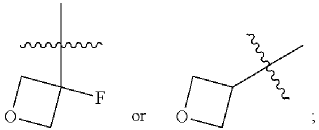
X is selected from a bond, -(CRₐR_{b})ₘ-, -NR₂-, -O(CRₐR_{b})ₘ-, - (CRₐR_{b})ₘO-, -C(O)NR₁₂-, -(CRₐR_{b})ₘO-(CRₐR_{b})ₘ- and - C(O)NR₁₂(CRₐR_{b})ₘ-;
Rₐ and R_{b} which may be same or different at each occurrence, are independently selected from hydrogen, halogen, hydroxy, substituted or unsubstituted (C₁-C₆)alkyl, substituted or unsubstituted (C₁-C₆)haloalkyl and substituted or unsubstituted (C₃-C₆)cycloalkyl; or Rₐ and R_{b}, together with the carbon atom to which they are attached, form a substituted or unsubstituted 3 to 6 membered saturated carbocyclic ring;
Z is -OR₁₀ or -NR₇R₈;
R₃ is selected from hydrogen, halogen, substituted or unsubstituted (C₁-C₆)alkyl, substituted or unsubstituted (C₁-C₆)haloalkyl, -OR₉, and substituted or unsubstituted (C₃-C₁₂)cycloalkyl;
R₄, which may be same or different at each occurrence, is independently selected from halogen, substituted or unsubstituted (C₁-C₆)alkyl, substituted or unsubstituted (C₁-C₆)haloalkyl, substituted or unsubstituted (C₁-C₆)alkoxyalkyl, -SF₅ and -OR₉;
R₅ is substituted or unsubstituted (C₁-C₆)alkyl;
R₆ is selected from substituted or unsubstituted (C₁-C₆)alkyl, substituted or unsubstituted (C₃-C₁₂)cycloalkyl and substituted or unsubstituted (C₆-C₁₄)aryl;
R₇ and R₈, which may be same or different at each occurrence, are independently selected from hydrogen, substituted or unsubstituted (C₁-C₆)alkyl, -(CRₐR_{b})₁₋₂R₁₁, -(CR_{c}R_{d})ₘ-OH and substituted or unsubstituted (C₃-C₁₂)cycloalkyl;
R_{c} and R_{d} which may be same or different at each occurrence, are independently hydrogen or substituted or unsubstituted (C₁-C₆)alkyl;
R₉ is independently selected from hydrogen, substituted or unsubstituted (C₁-C₆)alkyl, substituted or unsubstituted (C₁-C₆)haloalkyl, substituted or unsubstituted (C₁-C₆)alkoxyalkyl and substituted or unsubstituted (C₃-C₁₂)cycloalkyl;
R₁₀ is selected from hydrogen, substituted or unsubstituted (C₁-C₆)alkyl and - (CRₐR_{b})₁₋₂phenyl;
R₁₁ is substituted or unsubstituted phenyl, wherein the substituents are selected from halogen, (C₁-C₆)alkyl and -OR₉;
R₁₂ is hydrogen or substituted or unsubstituted (C₁-C₆)alkyl;
"m" is an integer ranging from 1 to 3, both inclusive; "n" is an integer ranging from 1 to 3, both inclusive; "p" is an integer ranging from 0 to 3, both inclusive; and "q" is an integer ranging from 1 to 3, both inclusive;
or pharmaceutically acceptable salts thereof, or a combination thereof, and at least two pharmaceutically acceptable excipients, wherein the at least two pharmaceutically acceptable excipients is microcrystalline cellulose, crospovidone, starch, magnesium stearate, sodium lauryl sulfate, colloidal silicon dioxide, or a combination thereof; and
wherein the solid pharmaceutical composition is suitable for oral administration and is in the form of a tablet or capsule.

In embodiments of the present invention, the solid pharmaceutical composition comprises a pharmaceutically effective amount of one or more CaSR agonists, wherein the one or more CaSR agonists is one or more compounds having a structure of Formula (I), Formula (II), Formula (III), Formula (IV), Formula (V), or Formula (VI) which has the structure of Compound 1, Compound 2, Compound 3, Compound 4, Compound 5, or Compound 6, respectively, as defined below:
(i) the compound of 3-((S)-2-((((R)-1-(naphthalen-1-yl)ethyl)amino)methyl) morpholino)-5-(trifluoromethyl)benzoic acid, or an internal salt or hydrochloride salt thereof ("Compound 1") 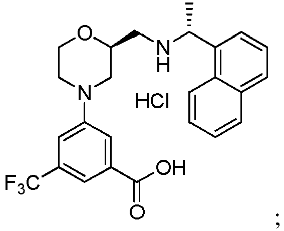
(ii) the compound of 2-methyl-5-((S)-2-(2-(((R)-1-(naphthalen-1-yl)ethyl)amino) ethyl)-2H-benzo[b][1,4]oxazin-4(3H)-yl)benzoic acid, or an internal salt or hydrochloride salt thereof ("Compound 2") 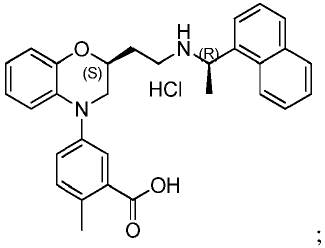
(iii) the compound of 2-methyl-5-((2R,4S)-2-((((R)-1-(naphthalen-1-yl)ethyl)amino) methyl)chroman-4-yl)benzoic acid, or an internal salt or hydrochloride salt thereof (such as 2-methyl-5-((2R,4S)-2-((((R)-1-(naphthalen-1-yl)ethyl)amino) methyl)chroman-4-yl)benzoic acid hydrochloride ("Compound 3")) 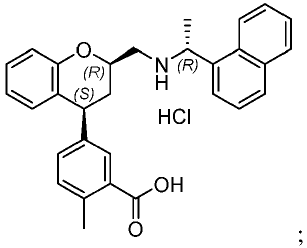
(iv) the compound of 3-((1R,3S)-3-((((R)-1-(4-fluoro-3-methoxyphenyl)ethyl)amino) methyl)-1,2,3,4-tetrahydronaphthalen-1-yl)-2,6-dimethylbenzoic acid, or an internal salt or hydrochloride salt thereof ("Compound 4") 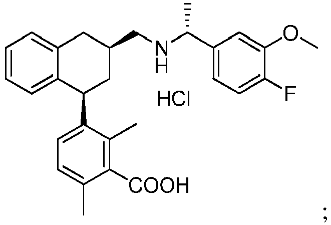
(v) the compound of (R)-3-(4-fluoro-3'-(2-((1-(3-methoxy phenyl)ethyl)amino) ethoxy)-5'-(trifluoromethyl)-[1,1'-biphenyl]-3-yl)propanoic acid, or an internal salt or hydrochloride salt thereof ("Compound 5") 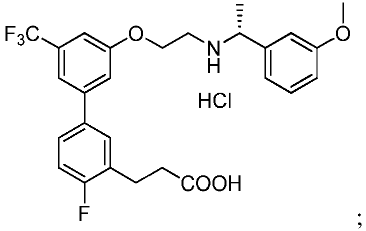 and
(vi) the compound of (R)-N-((R)-1-(3-methoxyphenyl)ethyl)-1-(4-(4-(trifluoromethyl) phenyl)naphthalen-2-yl)propan-1-amine, or hydrochloride salt thereof ("Compound 6")

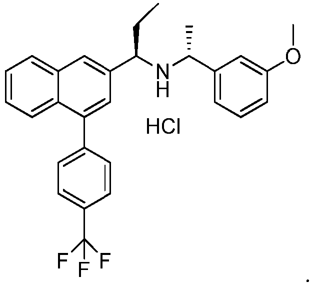

In some embodiments, the compound of Formula (I) is Compound 1, 3-((S)-2-((((R)-1-(naphthalen-1-yl)ethyl)amino)methyl) morpholino)-5-(trifluoromethyl)benzoic acid, or an internal salt or hydrochloride salt thereof.

In some embodiments, the compound of Formula (II) is Compound 2, 2-methyl-5-((S)-2-(2-(((R)-1-(naphthalen-1-yl)ethyl)amino) ethyl)-2H-benzo[b][1,4]oxazin-4(3H)-yl)benzoic acid, or an internal salt or hydrochloride salt thereof.

In some embodiments, the compound of Formula (III) is Compound 3, 2-methyl-5-((2R,4S)-2-((((R)-1-(naphthalen-1-yl)ethyl)amino) methyl)chroman-4-yl)benzoic acid hydrochloride.

In some embodiments, the compound of Formula (IV) is Compound 4, 3-((1R,3S)-3-((((R)-1-(4-fluoro-3-methoxyphenyl)ethyl)amino) methyl)-1,2,3,4-tetrahydronaphthalen-1-yl)-2,6-dimethylbenzoic acid, or an internal salt or hydrochloride salt thereof.

In some embodiments, the compound of Formula (V) is Compound 5, (R)-3-(4-fluoro-3'-(2-((1-(3-methoxy phenyl)ethyl)amino) ethoxy)-5'-(trifluoromethyl)-[1,1'-biphenyl]-3-yl)propanoic acid, or an internal salt or hydrochloride salt thereof.

In some embodiments, the compound of Formula (VI) is Compound 6, (R)-N-((R)-1-(3-methoxyphenyl)ethyl)-1-(4-(4-(trifluoromethyl) phenyl)naphthalen-2-yl)propan-1-amine, or an internal salt or hydrochloride salt thereof.

In some embodiments, the solid pharmaceutical composition of the present invention is in the form of a tablet.

In some embodiments, the solid pharmaceutical composition of the present invention is in the form of a coated tablet.

In some embodiments, the solid pharmaceutical composition of the present invention is in the form of a film coated tablet. In some embodiments, the thickness of the film coating is from about 10 microns to about 500 microns. In some embodiments, the thickness of the film coating is from about 20 microns to about 100 microns. In some aspects, the film coating comprises a resistant starch, a high-amylose maize starch, Eudragit^{®} S polymer (Poly(methacrylic acid-co-methyl methacrylate) 1:2), Eudragit^{®} RS (Poly(ethyl acrylate-co-methyl methacrylate-co-trimethylammonioethyl methacrylate chloride) 1:2:0.1), Eudragit^{®} RL (Poly(ethyl acrylate-co-methyl methacrylate-co-trimethylammonioethyl methacrylate chloride) 1:2:0.2), starch acetate, ethyl cellulose, Ac-Di-Sol^{®} (croscarmellose sodium), sodium starch glycolate, cellulose ether (e.g., hydroxypropyl methylcellulose (HPMC), hydroxypropyl cellulose (HPC), ethyl cellulose (EC), methylcellulose, methylhydroxycellulose, methylhydroxyethylcellulose, ethylcellulose, sodium carboxymethylcellulose, or a combination thereof), vinyl polymer (e.g., polyvinyl pyrrolidone (PVP), polyvinyl alcohol (PVA), PVP-polyvinyl acetate copolymer, PVA-PEG copolymer), glycol (e.g., high molecular weight polyethylene glycol (PEG), polypropylene glycol), acrylic polymer (e.g., methacrylate aminoester copolymer, ethylacrylate-methylmethacrylate copolymer), maltodextrin, polydextrose, cellulose, acetate phthalate, cellulose acetate trimellitate, hydroxypropyl methylcellulose acetate succinate-hypromellose acetate succinate, hydroxypropyl methylcellulose phthalate, polyvinyl acetate phthalate, poly(methacrylic acid-co-methyl methacrylate), shellac (e.g., ester of aleurtic acid), and a combination thereof.

In accordance with a second aspect, the present invention provides a solid pharmaceutical composition as described herein, for use in treating secondary hyperparathyroidism associated with chronic kidney disease (CKD) in asubject in need thereof. In accordance with a third aspect, the present invention provides a solid pharmaceutical composition as described herein, for use in lowering or suppressing one or more intact parathyroid hormone (iPTH) levels in a subject suffering from secondary hyperparathyroidism associated with CKD. In further aspects, the present invention provides a solid pharmaceutical composition of 2-methyl-5-((2R,4S)-2-((((R)-1-(naphthalen-1-yl)ethyl)amino) methyl)chroman-4-yl)benzoic acid, hydrochloride salt for use in the treatmentor management of secondary hyperparathyroidism associated with CKD; and asolid pharmaceutical composition of 2-methyl-5-((2R,4S)-2-((((R)-1-(naphthalen-1-yl)ethyl)amino) methyl)chroman-4-yl)benzoic acid, hydrochloride salt for use in the treatmentor management of secondary hyperparathyroidism associated with CKD by lowering orsuppressing one for more iPTH levels.

### BRIEF DESCRIPTION OF THE DRAWINGS

FIG. 1 is a bar graph showing mean % change in iPTH from baseline iPTH concentration levels against each dose (i.e., placebo; 10 mg QD; 25 mg QD; 20mg BID) of a representative compound of this disclosure, 2-methyl-5-((2R,4S)-2-((((R)-1-(naphthalen-1-yl)ethyl)amino) methyl)chroman-4-yl)benzoic acid hydrochloride (Compound 3) for patients on dialysis. This FIG. 1 also shows that iPTH levels in the patients are reduced more than 30% for the 20 mg BID and 25 mg QD dose groups and 25.65% for the 10 mg QD dose group. "N" indicates number of patients.
FIG. 2 is a bar graph showing mean % change in iPTH baseline iPTH concentration levels against each dose (i.e., placebo; 5 mg BID; 10 mg QD) of Compound 3 for patients not on dialysis. This FIG. 2 also shows that iPTH levels in the patients are reduced more than 30% for 5 mg BID and 10 mg QD dose groups.
FIG. 3 is a line graph showing mean iPTH levels at different time points (e.g., baseline (t=0); 30 days; 60 days; 90 days) and at different daily doses (i.e., placebo; 10 mg QD; 20 mg BID; 25 mg QD) of Compound 3 for patients on dialysis.
FIG. 4 is a line graph showing mean iPTH levels at different time points (e.g., baseline (t=0); 30 days; 60 days; 90 days) and at different daily doses (i.e., placebo; 5 mg BID; 10 mg QD) of Compound 3 for patients not on dialysis.
FIG. 5 is a bar graph showing % of patients in each dose arm (or group) (i.e., placebo; 10 mg QD; 20 mg BID; 25 mg QD) using Compound 3 who achieved equal to or more than 30% reduction at D90 from baseline iPTH concentration levels.
FIG. 6 is a bar graph showing mean % change in iPTH at day 90, 2-4 hours post dose (i.e., placebo; 10 mg QD; 20 mg BID; 25 mg QD) using Compound 3 during exploratory analysis.

### DETAILED DESCRIPTION

The invention is not limited to particular compositions, methods, uses, compounds, processes, or methodologies described, as these may vary. The terminology used in this Detailed Description section is for the purpose of describing particular versions or embodiments only, and is not intended to limit the scope of the invention. Unless defined otherwise, all technical and scientific terms used herein have the same meanings as commonly understood by one of ordinary skill in the art. Any methods and materials similar or equivalent to those described herein can be used in the practice or testing of embodiments of the invention, the preferred methods, devices, and materials described herein.

The pharmaceutical composition, as disclosed herein, is useful in tablet form, which is stable during long-term preservation, and further provides good oral bioavailability of the disclosed compounds.

### Definitions

For purposes of interpreting the specification, the following definitions will apply and whenever appropriate, terms used in the singular will also include the plural and vice versa.

As used herein, the singular forms "a," "an" and "the" include plural referents unless the context clearly dictates otherwise.

As used herein, the term "about" is intended to qualify the numerical values that it modifies, denoting such a value as variable within a margin of error. When no particular margin of error (such as, for example, standard deviation to a mean value) is recited, the term "about" means plus or minus 10% of the numerical value of the number with which it is being used. For example, "about 50%" means in the range of 45% to 55%. Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

Unless otherwise stated, the term "oxo" as used herein is a C(=O) group. Such an oxo group may be a part of either a cycle or a chain in the compounds disclosed in this application.

The term "acyl" as used herein is a -COR radical where "R" is hydrogen, alkyl, haloalkyl, cycloalkyl, aryl, aralkyl, heteroaryl, heteroaralkyl, or heterocyclyl as defined herein, e.g., formyl, acetyl, trifluoroacetyl, benzoyl, piperazin-1-ylcarbonyl, and the like. When R is alkyl it is referred to in this application as alkylcarbonyl. When R is aryl it is referred to in this application as arylcarbonyl. When R is heteroaryl it is referred to in this application as heteroarylcarbonyl. When R is heterocyclyl it is referred to in this application as heterocyclylcarbonyl.

The term "alkenyl" as used herein is a hydrocarbon radical containing from 2 to 10 carbon atoms and including at least one carbon-carbon double bond. Non-limiting examples of alkenyl groups include ethenyl, 1-propenyl, 2-propenyl (allyl), iso-propenyl, 2-methyl-l- propenyl, 1- butenyl, 2-butenyl and the like. Unless set forth or recited to the contrary, all alkenyl groups described or claimed herein may be straight chain or branched.

The term "alkoxy" as used herein is an alkyl group attached via an oxygen linkage. Non-limiting examples of alkoxy groups include methoxy, ethoxy, propoxy and the like. Unless set forth or recited to the contrary, all alkoxy groups described or claimed herein may be straight chain or branched.

The term "alkoxyalkyl" as used herein is a linear monovalent hydrocarbon radical of one to six carbon atoms or a branched monovalent hydrocarbon radical of three to six carbons substituted with at least one alkoxy group, preferably one or two alkoxy groups, as defined above, e.g., 2-methoxy-ethyl, 1-, 2-, or 3-methoxypropyl, 2-ethoxyethyl, and the like.

The term "alkoxycarbonyl" as used herein is a -C(O)OR radical, wherein "R" is an alkyl group as defined above, e.g., methoxycarbonyl, ethoxycarbonyl, and the like.

The term "alkyl" as used herein is an alkane-derived hydrocarbon radical that includes solely carbon and hydrogen atoms in the backbone, contains no unsaturation, has from one to six carbon atoms, and is attached to the remainder of the molecule by a single bond, e.g., methyl, ethyl, n-propyl, 1 -methylethyl (isopropyl), n-butyl, n-pentyl, 1, 1-dimethylethyl (t-butyl) and the like. Unless set forth or recited to the contrary, all alkyl groups described or claimed herein may be straight chain or branched.

The term "alkylamino" as used herein is a radical -NHR, wherein "R" is alkyl as defined herein, e.g., methylamino, ethylamino, n-, iso-propylamino, n-, iso-, tert-butylamino, and the like.

The term "alkylsulfonyl" as used herein is a -SO₂R radical, wherein "R" is alkyl as defined herein, e.g., methylsulfonyl, ethylsulfonyl, and the like.

The term "alkylthio" as used herein is a -SR radical, wherein "R" is alkyl as defined herein, e.g., methylthio, ethylthio, propylthio, or butylthio, and the like.

The term "alkynyl" as used herein is a hydrocarbon radical containing 2 to 10 carbon atoms and including at least one carbon-carbon triple bond. Non-limiting examples of alkynyl groups include ethynyl, propynyl, butynyl and the like. Unless set forth or recited to the contrary, all alkynyl groups described or claimed herein may be straight chain or branched.

The term "amino" as used herein is an -NH₂ radical.

The term "aminoalkyl" as used herein is a linear monovalent hydrocarbon radical of one to six carbon atoms or a branched monovalent hydrocarbon radical of three to six carbons substituted with at least one, preferably one or two, -NRR' where "R" is hydrogen, alkyl, acyl, hydroxyalkyl, alkoxyalkyl, aryl, aralkyl, heteroaryl, heteroaralkyl or heterocyclylalkyl and R' is hydrogen, alkyl, hydroxyalkyl, alkoxyalkyl, aryl, aralkyl, heteroaryl, heteroaralkyl, heterocyclylalkyl, cycloalkyl, cycloalkylalkyl, aminocarbonyl, aminosulfonyl as defined herein, e.g., aminomethyl, methylaminoethyl, dimethylaminoethyl, 1,3-diaminopropyl, acetylaminopropyl, and the like.

The term "aryl" as used herein is an aromatic radical having 6- to 14- carbon atoms, including monocyclic, bicyclic and tricyclic aromatic systems, such as phenyl, naphthyl, tetrahydronaphthyl, indanyl, and biphenyl and the like.

The term "arylalkyl" as used herein is an aryl group as defined above directly bonded to an alkyl group as defined above, e.g., -CH₂C₆H₅ and -C₂H₄C₆H₅.

The term "aromatic" as used herein is a moiety wherein the constituent atoms make up an unsaturated ring system, all atoms in the ring system are sp² hybridized and the total number of pi electrons is equal to 4n+2.

The terms "carbocyclic ring" or "carbocycle" as used herein refers to a 3- to 6 membered saturated or partially unsaturated, monocyclic fused bicyclic, spirocyclic ring containing carbon atoms, which may optionally be substituted, for Example, carbocyclic rings include but are not limited to cyclopropyl, cyclobutyl, cyclopentyl, cyclohexyl, cyclopropylene, cyclohexanone, etc.

The term "carboxy" as used herein is a -C(O)OH radical.

The term "cycloalkyl" refers to a non-aromatic mono or multicyclic ring system having 3 to 12 carbon atoms, such as cyclopropyl, cyclobutyl, cyclopentyl, cyclohexyl and the like. Examples of multicycliccycloalkyl groups include, but are not limited to, perhydronaphththyl, adamantyl and norbornyl groups, bridged cyclic groups or spirobicyclic groups, e.g. spiro(4,4)non-2-yl and the like.

The term "cycloalkylalkyl" refers to a cycloalkyl group as defined above, directly bonded to an alkyl group as defined above, e.g., cyclopropylmethyl, cyclobutylmethyl, cyclopentylmethyl, cyclohexylmethyl, cyclohexylethyl, etc.

The term "3 to 6 membered saturated carbocyclic ring" as used herein is a carbocyclic ring which is monocyclic and non-aromatic carbocyclic ring as defined herein.

The term "haloalkyl" as used herein is an alkyl group as defined above that is substituted by one or more halogen atoms as defined above. Preferably, the haloalkyl may be monohaloalkyl, dihaloalkyl or polyhaloalkyl, including perhaloalkyl. A monohaloalkyl can have one iodine, bromine, chlorine or fluorine atom. Dihaloalkyl and polyhaloalkyl groups can be substituted with two or more of the same halogen atoms or a combination of different halogen atoms. Preferably, a polyhaloalkyl is substituted with up to 12 halogen atoms. Non-limiting examples of a haloalkyl include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl,dichloropropyl and the like. A perhaloalkyl as used herein is an alkyl having all hydrogen atoms replaced with halogen atoms. Unless set forth or recited to the contrary, all haloalkyl groups described or claimed herein may be straight chain or branched.

The term "haloalkoxy" refers to a haloalkyl, defined herein, group attached via an oxygen linkage. Non-limiting Examples of such groups are monohaloalkoxy, dihaloalkoxy or polyhaloalkoxy including perhaloalkoxy. Unless set forth or recited to the contrary, all haloalkoxy group described or claimed herein may be straight chain or branched.

The terms "halogen" or "halo" as used herein is the group that can include, but not limited to fluorine, chlorine, bromine, or iodine.

The terms "heterocyclic ring" or "heterocyclyl ring" or "heterocyclyl", unless otherwise specified, as used herein is substituted or unsubstituted non-aromatic 3- to 15-membered ring which consists of carbon atoms and with one or more heteroatom(s) independently selected from N, O or S. The heterocyclic ring may be a mono-, bi- or tricyclic ring system, which may include fused, bridged or spiro ring systems and the nitrogen, carbon, oxygen or sulfur atoms in the heterocyclic ring may be optionally oxidized to various oxidation states. In addition, the nitrogen atom may be optionally quaternized, the heterocyclic ring or heterocyclyl may optionally contain one or more olefinic bond(s), and one or two carbon atoms(s) in the heterocyclic ring or heterocyclyl may be interrupted with - CF₂-, -C(O)-, -S(O)-, S(O)₂, - C(=N-alkyl)-, or -C(=N-cycloalkyl), etc. In addition, heterocyclic ring may also be fused with aromatic ring. Non-limiting examples of heterocyclic rings include azetidinyl, benzopyranyl, chromanyl, decahydroisoquinolyl, indanyl, indolinyl, isoindolinyl, isochromanyl, isothiazolidinyl, isoxazolidinyl, morpholinyl, oxazolinyl, oxazolidinyl, 2- oxopiperazinyl, 2-oxopiperidinyl, 2-oxopyrrolidinyl, 2-oxoazepinyl, octahydroindolyl, octahydroisoindolyl, perhydroazepinyl, piperazinyl, 4-piperidonyl, pyrrolidinyl, piperidinyl, phenothiazinyl, phenoxazinyl, quinuclidinyl, tetrahydroisquinolyl, tetrahydrofuryl, tetrahydropyranyl, thiazolinyl, thiazolidinyl, thiamorpholinyl, thiamorpholinylsulfoxide, thiamorpholinylsulfoneindoline, benzodioxole, tetrahydroquinoline, tetrahydrobenzopyran and the like. The heterocyclic ring may be attached by any atom of the heterocyclic ring that results in the creation of a stable structure.

The term "heteroaryl," unless otherwise specified, as used herein is a substituted or unsubstituted 5- to 14- membered aromatic heterocyclic ring with one or more heteroatom(s) independently selected from N, O or S. The heteroaryl may be a mono-, bi- or tricyclic ring system. The heteroaryl ring may be attached by any atom of the heteroaryl ring that results in the creation of a stable structure. Non-limiting examples of a heteroaryl ring include oxazolyl, isoxazolyl, imidazolyl, furyl, indolyl, isoindolyl, pyrrolyl, triazolyl, triazinyl, tetrazolyl, thienyl, thiazolyl, isothiazolyl, pyridyl, pyrimidinyl, pyrazinyl, pyridazinyl, benzofuranyl, benzothiazolyl, benzoxazolyl, benzimidazolyl, benzothienyl, carbazolyl, quinolinyl, isoquinolinyl, quinazolinyl, cinnolinyl, naphthyridinyl, pteridinyl, purinyl, quinoxalinyl, quinolyl, isoquinolyl, thiadiazolyl, indolizinyl, acridinyl, phenazinyl, phthalazinyl and the like.

The term "heterocyclylalkyl" as used herein is a -(alkylene)-R radical, wherein R is heterocyclyl as defined above, e.g., pyrrolidinylmethyl, tetrahydrofuranylethyl, pyridinylmethylpiperidinylmethyl, and the like.

The term "heteroarylalkyl" as used herein is a heteroaryl ring radical directly bonded to an alkyl group. The heteroarylalkyl radical may be attached to the main structure at any carbon atom in the alkyl group that results in the creation of a stable structure. Unless otherwise specified, the term "substituted" as used herein refers to a group or moiety having one or more substituents attached to the structural skeleton of the group or moiety. Such substituents include, but are not limited to, hydroxy, halogen, carboxyl, cyano, nitro, oxo (=O), thio (=S), alkyl, haloalkyl, alkenyl, alkynyl, aryl, arylalkyl, cycloalkyl, cycloalkylalkyl, heteroaryl, heterocyclic ring, heterocyclylalkyl, heteroarylalkyl, -C(O)ORₓ, - C(O)Rₓ, - C(S)R_{X}, - C(O)NRₓR_{y}, -NRₓC(O)NR_{y}R_{z}, -N(Rₓ)S(O)R_{y}, -N(Rₓ)S(O)₂R_{y}, -NRₓR_{y}, - NRₓC(O)R_{y}, -NRₓC(S)R_{y}, -NRₓC(S)NR_{y}R_{z}, - S(O)₂NRₓR_{y}, -ORₓ, -OC(O)Rₓ, -OC(O)NRₓR_{y}, -RₓC(O)OR_{y}, -RₓC(O)NR_{y}R_{z}, -RₓC(O)R_{y}, -SR_{X}, and -S(O)₂Rₓ; wherein each occurrence of Rₓ, R_{y} and R_{z} are independently selected from hydrogen, halogen, alkyl, haloalkyl, alkenyl, alkynyl, cycloalkyl and aryl. The aforementioned "substituted" groups cannot be further substituted.

The compounds of the invention may have one or more chiral centers. The absolute stereochemistry at each chiral center may be 'R' or 'S'. The compounds of the invention include all diastereomers and enantiomers and mixtures thereof. Unless specifically mentioned otherwise, reference to one stereoisomer applies to any of the possible stereoisomers. Whenever the stereoisomeric composition is unspecified, it is to be understood that all possible stereoisomers are included.

The term "stereoisomer" as used herein is a compound made up of the same atoms bonded by the same bonds but having different three-dimensional structures that are not interchangeable. The three-dimensional structures are called configurations.

The term "enantiomer" as used herein is two stereoisomers whose molecules are nonsuperimposable mirror images of one another.

The term "chiral center" as used herein is a carbon atom to which four different groups are attached.

The term "diastereomers" as used herein is stereoisomers which are not enantiomers.

The term "racemate" or "racemic mixture" as used herein is a mixture of equal parts of enantiomers.

The term "tautomer" as used herein is a compound that undergoes rapid proton shifts from one atom of the compound to another atom of the compound. Some of the compounds described herein may exist as tautomers with different points of attachment of hydrogen. The individual tautomers as well as mixture thereof are encompassed with compounds of Formula (I)-(VI).

The term "pharmaceutically acceptable" as used herein is that which is useful in preparing a pharmaceutical composition that is generally safe, non-toxic and neither biologically nor otherwise undesirable and includes that which is acceptable for veterinary use as well as human pharmaceutical use.

The term "pharmaceutically acceptable salts" as used herein refers to salts of compounds of this disclosure that are pharmaceutically acceptable, as defined above, and that possess the desired pharmacological activity. Such salts include acid addition salts formed with inorganic acids, such as hydrochloric acid, hydrobromic acid, sulfuric acid, nitric acid, phosphoric acid, and the like; or with organic acids, such as acetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, o-(4-hydroxybenzoyl)benzoic acid, cinnamic acid, mandelic acid, methylsulfonic acid, ethanesulfonic acid, 1,2-ethanedisulfonic acid, 2-hydroxy-ethanesulfonic acid, benzenesulfonic acid, p-chlorobenzenesulfonic acid, 2-naphthalenesulfonic acid, p-toluenesulfonic acid, camphorsulfonic acid, 4-methylbicyclo[2.2.2]oct-2-ene-1-carboxylic acid, glucoheptonic acid, 4,4'-methylenebis(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like.

Pharmaceutically acceptable salts also include base addition salts that may be formed when acidic protons present are capable of reacting with inorganic or organic bases. Acceptable inorganic bases include, but not limited to, sodium hydroxide, sodium carbonate, potassium hydroxide, aluminum hydroxide, calcium hydroxide, and the like. Acceptable organic bases include ethanolamine, diethanolamine, triethanolamine, tromethamine, N-methylglucamine, and the like.

As used herein, the terms "subject" or "patient" are interchangeable and may be taken to mean any living organism that may be treated with the methods/uses/compounds/compositions of the invention. As such, the term "subject" or "patient" may include, but is not limited to, mammal (non-human and human), primate, and other animals, such as domesticated animals (e.g., household pets, including cats and dogs) and non-domesticated animals (e.g., wildlife). In some embodiments, the "subject" or "patient" is an adult, child, infant, or fetus. In some embodiments, the term "subject" or "patient" is a human. In some embodiments, the term "subject" or "patient" is mammal, such as mice, rats, other rodents, rabbits, dogs, cats, swine, cattle, sheep, horses, primates, or humans. The disclosed methods/uses/compounds/compositions of the invention may be utilized to treat and/or prevent secondary hyperthyroidism of the symptoms thereof in a subject in need thereof.

As used herein, the term "subject in need thereof," includes, but not limited to, a subject having or at risk for developing a disease, condition or disorder, such as, for example, secondary hyperthyroidism. A subject in need thereof may include a subject on dialysis, or a subject not on dialysis.

As used herein, the terms "treat," "treating" or "treatment" of a disease, condition or disorder includes: (a) preventing or delaying the appearance of clinical symptoms of the disease, condition or disorder developing in a subject that may be afflicted with or predisposed to the disease, condition or disorder but does not yet experience or display clinical or subclinical symptoms of the disease, condition or disorder; (b) inhibiting the disease, condition or disorder, i.e., arresting or reducing the development of the disease, condition or disorder or at least one clinical or subclinical symptom thereof; c) ameliorating or lessening the severity of the disease, condition or disorder or at least one of its clinical or subclinical symptoms; or (d) relieving the disease, condition or disorder, i.e., causing regression of the disease, condition or disorder or at least one of its clinical or subclinical symptoms.

As used herein, the term "therapeutic" means an agent utilized to treat, combat, ameliorate or prevent an unwanted disease, condition or disorder in a subject.

The terms "pharmaceutically effective amount" or "therapeutically effective amount" or "therapeutic dose" as used herein are used interchangeably and refer to the amount of an active agent or pharmaceutical compound or composition that elicits a clinical, biological or medicinal response in a tissue, system, animal, individual or human that is being sought by a researcher, veterinarian, medical doctor, or other clinical professional. A clinical, biological or medical response may include, for example, one or more of the following: (1) preventing ordelaying a disease, condition or disorder in an individual that may be predisposed to the disease, condition or disorder but does not yet experience or display pathology or symptoms of the disease, condition or disorder, (2) inhibiting a disease, condition or disorder in an individual that is experiencing or displaying the pathology or symptoms of the disease, condition or disorder or arresting further development of the pathology and/or symptoms of the disease, condition or disorder, (3) ameliorating or lessening the severity of a disease, condition or disorder in an individual that is experiencing or exhibiting the pathology or symptoms of the disease, condition or disorder or reversing the pathology and/or symptoms experienced or exhibited by the individual, or (4) relieving the disease, condition or disorder, i.e., causing regression of the disease, condition or disorder or at least one of its clinical or subclinical symptoms, in the individual. The "therapeutically effective amount" will vary depending on the compound, the disease and its severity and the age, weight, physical condition and responsiveness of the subject to be treated.

As used herein, the term "manage" or "managing" or "management" of a disease, condition or disorder refers to the beneficial effects that a subject derives from a therapy comprising one or more prophylactic and/or therapeutic agents, which does not result in a cure of the disease, condition or disorder. In certain embodiments, a subject is administered a therapy comprising one or more prophylactic and/or therapeutic agents to "manage" a disease, condition or disorder so as to prevent the progression or worsening of the disease, condition or disorder.

As used herein, the term "Cmax" refers to the maximum concentration that a compound or drug achieves in a tested area in a subject after the drug has been administered and prior to the administration of the next dose. The tested area can be, for example, plasma.

As used herein, the term "Tmax" refers to a period of time between the administration of a given dose of a compound or drug to a subject and the point in time when Cmax is reached.

As used herein, the terms "modulate" or "modulating" or "modulation" or "modulator" refer to an increase in the amount, quality, or effect of a particular activity or function of a receptor. By way of illustration and not limitation, it includes agonists, partial agonists, and allosteric modulators of calcium sensing receptor (CaSR) of the invention. Such modulation may be contingent on the occurrence of a specific event, such as activation of a signal transduction pathway.

As used herein, the term "pharmaceutically acceptable excipient" refers to any of the components of a formulation or pharmaceutical composition other than the Compound 1 to Compound 6, and which are approved by regulatory authorities or are generally regarded as safe for human or animal use. Pharmaceutically acceptable excipient includes, but not limited to, one or more of diluents, disintegrants, binders, lubricants, surfactants, glidants, preservatives, buffering agents, chelating agents, polymers, opacifiers, colorants, gelling agents/viscosifying agents, antioxidants, solvents and the like.

Non-limiting examples of "binders" include one or more of alginic acid and salts thereof; cellulose derivatives, such as carboxymethylcellulose, methylcellulose (e.g., Methocel^{®}), hydroxypropylmethylcellulose, hydroxyethylcellulose, hydroxypropylcellulose (e.g., Kiucel^{®}), ethylcellulose (e.g., Ethocel^{®}), and microcrystalline cellulose (e.g., Avicel^{®}); microcrystalline dextrose; amylose; magnesium aluminum silicate; polysaccharide acids; bentonites; gelatin; polyvinyl-pyrrolidone/vinyl acetate copolymer; crosspovidone; povidone; starch; pregelatinized starch; dextrin; a sugar, such as sucrose (e.g., Dipac^{®}), glucose, dextrose, molasses, mannitol, sorbitol, xylitol (e.g., Xylitab^{®}), and lactose; a natural or synthetic gum such as acacia, tragacanth, ghatti gum mucilage of isapol husks, polyvinylpyrrolidone (e.g., Polyvidone^{®} CL, Kollidon^{®} CL, Polyplasdone^{®} XL-10), larch arabogalactan, Veegum^{®}, polyethylene glycol, polyethylene oxide, waxes, sodium alginate, and the like. Binder usage level in tablet formulations varies on whether direct compression, wet granulation, or roller compaction process is used to make the tablet, and/or on types of other excipients used to make the formulation e.g., fillers which itself can act as moderate binder.

Non-limiting examples of "diluents" include one or more of lactose, starch, mannitol, sorbitol, dextrose, microcrystalline cellulose such as Avicel^{®}; dibasic calcium phosphate, dicalcium phosphate dihydrate; tricalcium phosphate, calcium phosphate; anhydrous lactose, spray-dried lactose; pregelatinized starch, compressible sugar, such as DiPac^{®} (Amstar); hydroxypropyl-methylcellulose, hydroxypropylmethylcellulose acetate stearate, sucrose-based diluents, confectioner's sugar; monobasic calcium sulfate monohydrate, calcium sulfate dihydrate; calcium lactate trihydrate, dextrates; hydrolyzed cereal solids, amylose; powdered cellulose, calcium carbonate; glycine, kaolin; mannitol, sodium chloride; inositol, bentonite, and the like. Diluent(s) may be used in the pharmaceutical composition to dilute the compound of interest prior to delivery. Diluents may also be used to stabilize the compounds because they can provide a more stable environment.

Non-limiting examples of "disintegrants" or "disintegration agents" include one or more of a starch, e.g., a natural starch such as corn starch or potato starch, a pregelatinized starch such as National 1551 or sodium starch glycolate such as Promogel^{®}. or Explotab^{®}, a cellulose such as a wood product, methylcrystalline cellulose, e.g., Avicel^{®}, Avicel^{®} PH101, Avicel^{®} PH 102, Avicel^{®} PH105, Elceme^{®} P100, Emcocel^{®}, Vivacel^{®}, and Solka-Floc^{®}, methylcellulose, croscarmellose, or a cross-linked cellulose, such as cross-linked sodium carboxymethyl-cellulose (Ac-Di-Sol^{®}), cross-linked carboxymethylcellulose, or cross-linked croscarmellose, a cross-linked starch such as sodium starch glycolate, a cross-linked polymer such as crosspovidone, a cross-linked polyvinylpyrrolidone, alginate such as alginic acid or a salt of alginic acid such as sodium alginate, a clay such as Veegum^{®} HV (magnesium aluminum silicate), a gum such as agar, guar, locust bean, Karaya, pectin, or tragacanth, sodium starch glycolate, bentonite, a natural sponge, a surfactant, a resin such as a cation-exchange resin, citrus pulp, sodium lauryl sulfate, sodium lauryl sulfate in combination starch, and the like. Disintegrants may be used in a pharmaceutical composition to facilitate the breakup or disintegration of a dosage form when it comes in contact with the gastrointestinal fluid.

Non-limiting examples of "glidants" and "lubricants" includes one or more of stearic acid, magnesium stearate, talc, colloidal silicon dioxide, sodium stearyl fumarate, and the like.

Non-limiting examples of "surfactants" include one or more of sodium lauryl sulfate, sodium docusate, Tween 60 or 80, triacetin, vitamin E TPGS, sorbitan monooleate, polyoxyethylene sorbitan monooleate, polysorbates, polaxomers, bile salts, glyceryl monostearate, copolymers of ethylene oxide and propylene oxide, e.g., Pluronic^{®} (BASF), and the like. Some other surfactants include polyoxyethylene fatty acid glycerides and vegetable oils, e.g., polyoxyethylene (60) hydrogenated castor oil; and polyoxyethylene alkylethers and alkylphenyl ethers, e.g. octoxynol 10, octoxynol 40. Surfactants may be used in a pharmaceutical composition to enhance physical stability or for other purposes.

Non-limiting examples of "solubilizers" includes one or more of triacetin; triethylcitrate; ethyl oleate; ethyl caprylate; sodium lauryl sulfate; sodium docusate; vitamin E TPGS; dimethylacetamide; N-hydroxyethylpyrrolidone; polyvinylpyrrolidone; organic alcohols such as ethanol, n-butanol, isopropyl alcohol, and the like; hydroxypropylmethyl cellulose; hydroxypropyl beta cyclodextrins, such as Captisol^{®}; cholesterol; bile salts; propylene glycol; polyethylene glycol (e.g., PEG 200-600); glycofurol; transcutol; dimethyl isosorbide and the like.

As used herein, "hyperphosphatemia" means a condition in which there is an elevated level of phosphate in the blood. Average serum phosphorus mass in a human adult typically range from about 2.5-4.5 mg/dL (about 0.81-1.45 mmol/L). In patients with advanced kidney disease, the body phosphorus overload manifests itself by serum phosphorus concentration above normal levels, i.e., hyperphosphatemia having serum phosphate >4.5 mg/dL (>1.44 mmol/L).

As used herein, "hypocalcemia" means decreased calcium levels in the blood. Calcium levels less than about 9 mg/dL or 2.2 mmol/L may be indicative of hypocalcemia. Hypocalcemia is a contraindication of calcimimetics and calcimimetics should not be administered to subjects having a calcium level of 8 mg/dL or less.

As used herein, the terms "not on dialysis subject" or "subject not on dialysis" refer to subjects with 1 of 2 corrected total serum calcium values obtained during the screening period of ≥8.8 mg/dL (2.20 mmol/L), provided the lower value was ≥8.4 mg/dL (2.10 mmol/L). The subject is not undergoing or has not been diagnosed to undergo renal dialysis.

As used herein, the terms "on dialysis subject" or "subject on dialysis" refer to subjects with 1 of 2 corrected total serum calcium values obtained during the screening period of ≥8.4 mg/dL (2.10 mmol/L), provided the lower value was ≥8.0 mg/dL (2 mmol/L). The subject undergoing or has been diagnosed to undergo renal dialysis.

As used herein, the term "administering" when used in conjunction with a therapeutic refers to administer a therapeutic directly or indirectly into or onto a target issue or to a subject, whereby the therapeutic locally or systemically affects the tissue or the subject. "Administering" a composition or pharmaceutical composition may be accomplished by oral administration, injection, infusion, inhalation, absorption or by any method in combination with other known techniques. "Administering" may include the act of self-administration or administration by another person, such as by a health care provider.

### Example Compounds and Pharmaceutical Compositions Thereof

In some aspects, this disclosure provides for pharmaceutical compositions that provide for unexpectedly high oral bioavailability of one or more compounds of Formula (I), Formula (II), Formula (III), Formula (IV), Formula (V), Formula (VI), pharmaceutically acceptable salts thereof, or a combination thereof. The pharmaceutical compositions can be solid.

In some aspects, compounds of Formula (I) have the following structure: 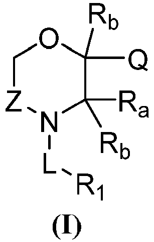 wherein
Q is 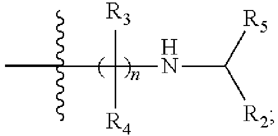
Rₐ is hydrogen;
R_{b} is hydrogen;
L is a bond or -(CR_{c}R_{d})ₘ;
R_{c} and R_{d} are independently selected from hydrogen or substituted or unsubstituted alkyl;
R₁ is 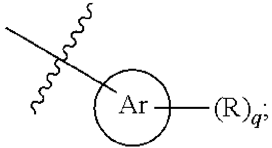
ring Ar is phenyl or naphthyl;
R, which may be same or different at each occurrence, is independently selected from halogen, nitro, cyano, substituted or unsubstituted alkyl, substituted or unsubstituted alkenyl, substituted or unsubstituted alkynyl, substituted or unsubstituted haloalkyl, -OR₆, - C(O)R₆, -(CRₑR_{f})₀₋₃-C(O)OR₆, -(CRₑR_{f})₁₋₂cycloalkylene-C(O)OR₆, -cycloalkylene (CRₑR_{f})₀₋₂-C(O)OR₆, -O(CRₑR_{f})₀₋₃-C(O)OR₆, -O-cycloalkylene-C(O)OR₆, - C(O)NR₇-(CRₑR_{f})₁₋₂-C(O)OR₆, -C(O)NR₇R₈, -S(O)₀₋₂R₆, and -S(O)₂NR₇R₈;
Rₑ and R_{f} are independently hydrogen or substituted or unsubstituted alkyl;
R₂ is substituted or unsubstituted aryl;
R₃ and R₄ are independently selected from hydrogen, halogen, and substituted or unsubstituted alkyl;
R₅ is substituted or unsubstituted alkyl or haloalkyl;
R₆, which may be same or different at each occurrence, is independently selected from hydrogen, substituted or unsubstituted alkyl and substituted or unsubstituted haloalkyl;
R₇ and R₈, which may be same or different at each occurrence, are independently selected from hydrogen, substituted or unsubstituted alkyl, substituted or unsubstituted cycloalkyl, and substituted or unsubstituted aryl;
Z is -CR_{g}Rₕ-;
R_{g} and Rₕ are hydrogen;
"m" is an integer ranging from 1 to 3, both inclusive; "n" is an integer ranging from 1 to 3, both inclusive; and "q" is an integer ranging from 0 to 4, both inclusive;
or its pharmaceutically acceptable salt thereof.

In some aspects, the compound of Formula (II) has the following structure: 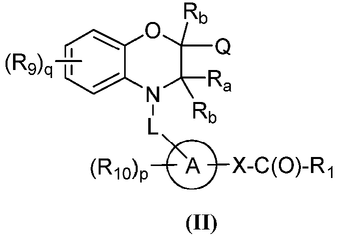 wherein
Q is 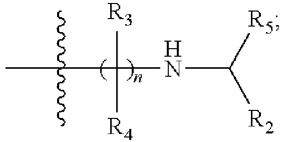
Rₐ is hydrogen, halogen or alkyl;
R_{b} is selected from hydrogen, alkyl, and haloalkyl;
or Rₐ and R_{b} together attached on the same carbon form C(O);
L is a bond or -CR_{c}R_{d}-;
ring A is phenyl;
R_{c} and R_{d} are independently selected from hydrogen, halogen, and alkyl;
X is selected from a bond, -(CRₑR_{f})ₘ, -O-, -O(CRₑR_{f})ₘ-, -(CRₑR_{f})ₘO-, - C(O)(CRₑR_{f})ₘ-, -C(O)NR₇-, -C(O)NR₇(CRₑR_{f})ₘ₋, -cycloalkylene-, and -O-cycloalkylene-;
Rₑ and R_{f}, which may be same or different at each occurrence, are independently selected from hydrogen, halogen, alkyl, haloalkyl and cycloalkyl; or Rₑ and R_{f}, together with the carbon atom to which they are attached, may form a 3 to 7 membered saturated carbocyclic ring;
R₁ is -OR₆ or -NR₇R₈;
R₂ is substituted or unsubstituted aryl;
R₃ and R₄ are independently selected from hydrogen, halogen, alkyl, haloalkyl, and cycloalkyl;
R₅ is alkyl or haloalkyl;
R₆ is hydrogen or alkyl;
R₇ and R₈ are independently selected from hydrogen, alkyl, cycloalkyl cycloalkylalkyl and aryl;
R₉, which may be same or different at each occurrence, is independently selected from halogen, cyano, alkyl, haloalkyl, and cycloalkyl;
R₁₀, which may be same or different at each occurrence, is independently selected from halogen, cyano, alkyl, alkenyl, alkynyl, haloalkyl, hydroxyalkyl, -OR₆, -C(O)R₆, - NR₇C(O)R₆, -S(O)₀₋₂R₆, -S(O)₂NR₇R₈, and -NR₇S(O)₂R₆;
"m" is an integer ranging from 1 to 3, both inclusive; "n" is an integer ranging from 1 to 3, both inclusive; "p" is an integer ranging from 0 to 2, both inclusive; and "q" is an integer ranging from 0 to 1, both inclusive;
or pharmaceutically acceptable salt thereof.

In some aspects, the compound of Formula (II) has the following structure: 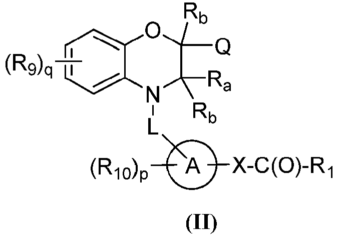
wherein Q is 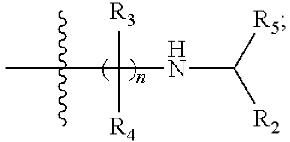
Rₐ is hydrogen; R_{b} is hydrogen or alkyl;
L is a bond, or -CR_{c}R_{d};
ring A is phenyl;
R_{c} and R_{d} are independently selected from hydrogen, halogen, and alkyl;
X is selected from a bond, -(CRₑR_{f})ₘ, -O-, -O(CRₑR_{f})ₘ-, -(CRₑR_{f})ₘO-, - C(O)(CRₑR_{f})ₘ-, -C(O)NR₇-, and -C(O)NR₇(CRₑR_{f})ₘ-;
Rₑ and R_{f}, which may be same or different at each occurrence, are independently selected from hydrogen, halogen, alkyl, haloalkyl and cycloalkyl; or Rₑ and R_{f}, together with the carbon atom to which they are attached, form a 3 to 7 membered saturated carbocyclic ring;
R₁ is -OR₆ or -NR₇R₈;
R₂ is phenyl or naphthyl, wherein the phenyl is substituted with halogen, alkyl, haloalkyl, alkoxy or haloalkoxy;
R₃ and R₄ are hydrogen;
R₅ is alkyl;
R₆ is hydrogen or alkyl;
R₇ and R₈ are hydrogen or alkyl;
R₉ is independently selected from halogen, cyano, alkyl, haloalkyl, and cycloalkyl;
R₁₀, which may be same or different at each occurrence, is independently selected from halogen, cyano, alkyl, haloalkyl, hydroxyalkyl, -OR₆, -C(O)R₆, -NR₇R₈, -NR₇C(O)R₆, and -(O)₂NR₇R₈;
"m" is an integer ranging from 1 to 3, both inclusive; "n" is an integer ranging from 1 to 3, both inclusive; "p" is an integer ranging from 0 to 2, both inclusive; "q" is an integer ranging from 0 to 1, both inclusive; or pharmaceutically acceptable salt thereof.

In some aspects, the compound of Formula (III) has the following structure: 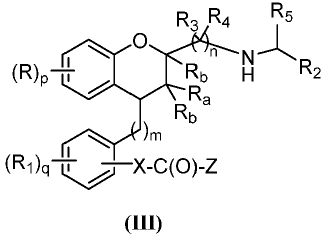 wherein
Rₐ is selected from hydrogen, halogen, substituted or unsubstituted alkyl, cyano, substituted or unsubstituted cycloalkyl and substituted or unsubstituted haloalkyl;
R_{b}, which may be same or different at each occurrence, is independently selected from hydrogen, halogen, substituted or unsubstituted alkyl, substituted or unsubstituted cycloalkyl and substituted or unsubstituted haloalkyl;
R_{c} which may be same or different at each occurrence, is independently selected from halogen, hydroxy, substituted or unsubstituted alkyl, substituted or unsubstituted haloalkyl, substituted or unsubstituted alkenyl, substituted or unsubstituted alkynyl, substituted or unsubstituted cycloalkyl, OR₆, nitro, cyano, -C(O)OR₆, -(CH₂)ᵣ-C(O)OR₆, -O-C(O)OR₆, -O(CH₂)ᵣ-C(O)OR₆, -NR₇R₈, -(CH₂)ᵣNR₇R₈-, -C(O)R₉, -C(O)NR₇R₈, -(CH₂)ᵣ-C(O)NR₇R₈, -NR₇C(O)R₉, -S(O)₀₋₂R₆, -S(O)₂NR₇R₈, and -NR₇S(O)₂R₉;
X is selected from a bond, -(CR_{c}R_{d})ᵣ-, -O-, -NR₇-, -NR₇(CR_{c}R_{d})ᵣ-, -O(CR_{c}R_{d})ᵣ, -C(O)NR₇-, -C(O)NR₇(CR_{c}R_{d})ᵣ, -(CR_{c}R_{d})ᵣNR₇(CR_{c}R_{d})ᵣ, -(CR_{c}R_{d})ᵣcycloalkylene-, cycloalkylene, -cycloalkylene(CR_{c}R_{d})ᵣ- and -O-cycloalkylene where cycloalkylene may be substituted or unsubstituted;
R_{c} and R_{d}, which may be same or different at each occurrence, are independently selected from hydrogen, halogen, hydroxy, cyano, nitro, substituted or unsubstituted alkyl, substituted or unsubstituted haloalkyl and substituted or unsubstituted cycloalkyl; or R_{c} and R_{d}, together with the carbon atom to which they are attached, may form a substituted or unsubstituted 3 to 7 membered saturated carbocyclic ring;
Z is -OR₆ or -NR₁₀R₁₁;
R₁, which may be same or different at each occurrence, is independently selected from halogen, nitro, cyano, substituted or unsubstituted alkyl, substituted or unsubstituted alkenyl, substituted or unsubstituted alkynyl, substituted or unsubstituted haloalkyl, substituted or unsubstituted cycloalkyl, -OR₆, -C(O)R₉, -NR₇R₈, -(CH₂)ᵣNR₇R₈-, -(CH₂)ᵣ-C(O)OR₆, -O-C(O)OR₆, -O(CH₂)ᵣ-C(O)OR₆, -C(O)NR₇R₈, -(CH₂)ᵣ-C(O)NR₇R₈, -NR₇C(O)R₉, -S(O)₀₋₂R₇, -S(O)₂NR₇R₈ and -NR₇S(O)₂R₉;
R₂ is selected from substituted or unsubstituted aryl, substituted or unsubstituted heteroaryl and substituted or unsubstituted heterocyclyl;
R₃ and R₄ may be same or different and are independently selected from hydrogen, halogen, substituted or unsubstituted alkyl, substituted or unsubstituted haloalkyl, substituted or unsubstituted alkenyl, substituted or unsubstituted alkynyl, substituted or unsubstituted alkoxy, substituted or unsubstituted haloalkoxy and substituted or unsubstituted cycloalkyl;
R₅ is substituted or unsubstituted alkyl;
R₆, which may be same or different at each occurrence, is independently selected from hydrogen, substituted or unsubstituted alkyl, substituted or unsubstituted haloalkyl, substituted or unsubstituted alkenyl, substituted or unsubstituted alkynyl, and substituted or unsubstituted aryl;
R₇ and R₈, which may be same or different at each occurrence, are independently selected from hydrogen, substituted or unsubstituted alkyl, substituted or unsubstituted alkenyl, substituted or unsubstituted alkynyl, substituted or unsubstituted cycloalkyl, substituted or unsubstituted cycloalkylalkyl, substituted or unsubstituted aryl, substituted or unsubstituted arylalkyl, substituted or unsubstituted heteroaryl, substituted or unsubstituted heteroarylalkyl, substituted or unsubstituted heterocyclyl, and substituted or unsubstituted heterocyclylalkyl; or R₇ and R₈, together with the nitrogen atom to which they are attached, may form a substituted or unsubstituted, saturated or unsaturated 3 to 12 membered cyclic ring, wherein the unsaturated cyclic ring may have one or two double bonds;
at each occurrence, R₉ is substituted or unsubstituted alkyl or substituted or unsubstituted aryl;
R₁₀ and R₁₁ may be same or different and are independently selected from hydrogen, substituted or unsubstituted alkyl, substituted or unsubstituted alkenyl, substituted or unsubstituted alkynyl, -(CR_{c}R_{d})ᵣ-C(O)OR₆, substituted or unsubstituted cycloalkyl, substituted or unsubstituted cycloalkylalkyl, substituted or unsubstituted aryl, substituted or unsubstituted arylalkyl, substituted or unsubstituted heteroaryl, substituted or unsubstituted heteroarylalkyl, substituted or unsubstituted heterocyclyl, and substituted or unsubstituted heterocyclylalkyl; or R₁₀ and R₁₁, together with the nitrogen atom to which they are attached, may form a substituted or unsubstituted, saturated or unsaturated 3 to 12 membered cyclic ring, wherein the unsaturated cyclic ring may have one or two double bonds;
"n" is an integer ranging from 1 to 3, both inclusive; "m" is an integer ranging from 0 to 3, both inclusive; "p" is an integer ranging from 0 to 4, both inclusive; "q" is an integer ranging from 0 to 3, both inclusive; and "r" is an integer ranging from 1 to 3, both inclusive;
or its pharmaceutically acceptable salt thereof.

In some aspects, the compound of Formula (IV) has the following structure: 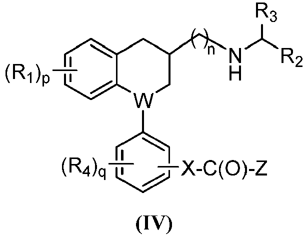 wherein
W is CH or N;
R₁, which may be same or different at each occurrence, is independently selected from halogen, nitro, cyano, substituted or unsubstituted alkyl, substituted or unsubstituted haloalkyl, substituted or unsubstituted cycloalkyl, -C(O)OR₅, -(CRₐR_{b})ᵣ-C(O)OR₅, - O-C(O)OR₅, -O(CRₐR_{b})ᵣ-C(O)OR₅, -NR₆R₇, -C(O)R₈, -C(O)NR₆R₇, -NR₆C(O)R₈, -S(O)₀₋₂R₅, -S(O)₂NR₆R₇ and -NR₆S(O)₂R₈;
R₂ is substituted or unsubstituted aryl;
R₃ is substituted or unsubstituted alkyl;
R₄, which may be same or different at each occurrence, is independently selected from halogen, cyano, substituted or unsubstituted alkyl, substituted or unsubstituted haloalkyl, - OR₅, -NR₆R₇, -C(O)R₈, -C(O)NR₆R₇, -NR₆C(O)R₈, -S(O)₀₋₂R₅, -S(O)₂NR₆R₇ and -NR₅S(O)₂R₈;
X is selected from a bond, -(CRₐR_{b})ᵣ-, -O-, -NR₇-, -O(CRₐR_{b})ᵣ-, -C(O)NR₇-, -C(O)NR₇(CRₐR_{b})ᵣ-, -(CRₐR_{b})ᵣcycloalkylene-, cycloalkylene, cycloalkylene-(CRₐR_{b})ᵣ- and -O-cycloalkylene;
Rₐ and R_{b}, which may be same or different at each occurrence, are independently selected from hydrogen, halogen, hydroxy, cyano, substituted or unsubstituted alkyl, substituted or unsubstituted haloalkyl and substituted or unsubstituted cycloalkyl; or Rₐ and R_{b}, together with the carbon atom to which they are attached, may form a substituted or unsubstituted 3 to 7 membered saturated carbocyclic ring;
Z is -OR₅ or -NR₆R₇;
R₅, which may be same or different at each occurrence, is independently selected from hydrogen, substituted or unsubstituted alkyl, substituted or unsubstituted haloalkyl and substituted or unsubstituted aryl;
R₆ and R₇, which may be same or different at each occurrence, are independently selected from hydrogen, substituted or unsubstituted alkyl, -(CRₐR_{b})ᵣ-C(O)OR₅, substituted or unsubstituted cycloalkyl, substituted or unsubstituted cycloalkylalkyl, substituted or unsubstituted aryl, substituted or unsubstituted arylalkyl, substituted or unsubstituted heteroaryl, substituted or unsubstituted heteroarylalkyl, substituted or unsubstituted heterocyclyl and substituted or unsubstituted heterocyclylalkyl; or R₆ and R₇, together with the nitrogen atom to which they are attached, may form a substituted or unsubstituted, saturated or unsaturated 3 to 10 membered cyclic ring, wherein the unsaturated cyclic ring may have one or two double bonds;
R₈ is substituted or unsubstituted alkyl or substituted or unsubstituted aryl;
"n" is an integer ranging from 0 to 3, both inclusive; "p" is an integer ranging from 0 to 3, both inclusive; "q" is an integer ranging from 0 to 3, both inclusive; and "r" is an integer ranging from 1 to 3, both inclusive;
or a pharmaceutically acceptable salt thereof.

In some aspects, the compound of Formula (V) has the following structure: 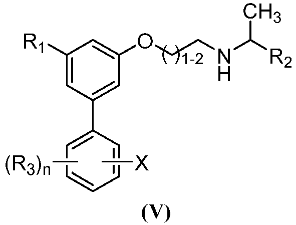 wherein
R₁ is selected from halogen, substituted or unsubstituted alkyl, substituted or unsubstituted haloalkyl, substituted or unsubstituted cycloalkyl, substituted or unsubstituted alkoxy, substituted or unsubstituted haloalkoxy, -(CRₐR_{b})₁-₃OH, -C(O)NH-alkyl, -S(O)₂- alkyl, - S(O)₂NH-alkyl, -C(O)OH, -C(O)O-alkyl, -(CRₐR_{b})₁₋₃C(O)OH and -(CRₐR_{b})₁₋₃C(O)O- alkyl;
R₂ is substituted or unsubstituted phenyl or substituted or unsubstituted naphthyl, wherein the substituents may be one or more and are independently selected from halogen, substituted or unsubstituted alkyl, substituted or unsubstituted haloalkyl, -OR₄, -NR₅R₆ and substituted or unsubstituted cycloalkyl;
X is selected from -C(O)OH, -C(O)O-alkyl, -C(O)NR₅R₆, -(CRₐR_{b})₁₋₃C(O)OH, -C(O)O-alkyl, -O-(CRₐR_{b})1₋₃C(O)OH, -O-(CRₐR_{b})₁₋₃C(O)O-alkyl, -CR_{C}=CR_{C}- -C(O)OH, halogen, substituted or unsubstituted alkyl, substituted or unsubstituted haloalkyl,- OR₄ and -S(O)₂-alkyl;
Rₐ and R_{b} are independently selected from hydrogen, halogen, hydroxy, substituted or unsubstituted alkyl, substituted or unsubstituted haloalkyl and substituted or unsubstituted cycloalkyl; or Rₐ and R_{b}, together with the carbon atom to which they are attached, may form a substituted or unsubstituted 3 to 6 membered saturated carbocyclic ring;
R_{c} is independently selected from hydrogen, halogen and substituted or unsubstituted alkyl;
R₃, which may be same or different at each occurrence, is independently selected from halogen, cyano, substituted or unsubstituted alkyl, substituted or unsubstituted haloalkyl and - OR₄;
R₄ is selected from hydrogen, substituted or unsubstituted alkyl, substituted or unsubstituted haloalkyl and substituted or unsubstituted cycloalkyl;
R₅ and R₆ are independently selected from hydrogen, substituted or unsubstituted alkyl and substituted or unsubstituted cycloalkyl; and "n" is an integer ranging from 0 to 2, both inclusive; or a pharmaceutically acceptable salt thereof.

In some aspects, the compound of Formula (VI) has the following structure: 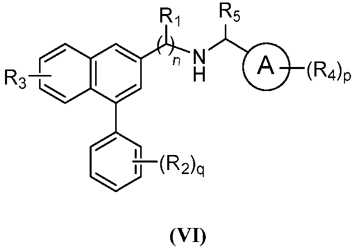 wherein
ring A is phenyl or naphthyl;
R₁ is hydrogen or substituted or unsubstituted (C₁-C₆)alkyl;
R₂, which may be same or different at each occurrence, is independently selected from halogen, cyano, substituted or unsubstituted (C₁-C₆)alkyl, substituted or unsubstituted (C₁-C₆)haloalkyl, substituted or unsubstituted (C₁-C₆)hydroxyalkyl, -X-C(O)-Z, -OR₉, - NR₇R₈, -NR₇C(O)R₆, -S(O)₀₋₂R₆, -S(O)₂NR₇R₈, -NR₇S(O)₂R₆, substituted or unsubstituted (C₃-C₁₂) cycloalkyl, substituted or unsubstituted phenyl, substituted or unsubstituted 5- to 6-membered heteroaryl, substituted or unsubstituted 5- to 6-membered heterocyclyl and ring D;
ring D is 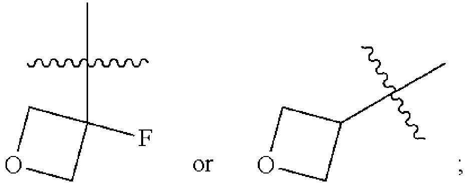
X is selected from a bond, -(CRₐR_{b})ₘ-, -NR₂-, -O(CRₐR_{b})ₘ-, -(CRₐR_{b})ₘO-, - C(O)NR₁₂-, -(CRₐR_{b})ₘO-(CRₐR_{b})ₘ₋ and -C(O)NR₁₂(CRₐR_{b})ₘ-;
Rₐ and R_{b} which may be same or different at each occurrence, are independently selected from hydrogen, halogen, hydroxy, substituted or unsubstituted (C₁-C₆)alkyl, substituted or unsubstituted (C₁-C₆)haloalkyl and substituted or unsubstituted (C₃-C₆)cycloalkyl; or Rₐ and R_{b}, together with the carbon atom to which they are attached, form a substituted or unsubstituted 3 to 6 membered saturated carbocyclic ring;
Z is -OR₁₀ or -NR₇R₈;
R₃ is selected from hydrogen, halogen, substituted or unsubstituted (C₁-C₆)alkyl, substituted or unsubstituted (C₁-C₆)haloalkyl, -OR₉, and substituted or unsubstituted (C₃-C₁₂)cycloalkyl;
R₄, which may be same or different at each occurrence, is independently selected from halogen, substituted or unsubstituted (C₁-C₆)alkyl, substituted or unsubstituted (C₁-C₆)haloalkyl, substituted or unsubstituted (C₁-C₆)alkoxyalkyl, -SF₅ and -OR₉;
R₅ is substituted or unsubstituted (C₁-C₆)alkyl;
R₆ is selected from substituted or unsubstituted (C₁-C₆)alkyl, substituted or unsubstituted (C₃-C₁₂)cycloalkyl and substituted or unsubstituted (C₆-C₁₄)aryl;
R₇ and R₈, which may be same or different at each occurrence, are independently selected from hydrogen, substituted or unsubstituted (C₁-C₆)alkyl, -(CRₐR_{b})₁₋₂R₁₁, -(CR_{c}R_{d})ₘ-OH and substituted or unsubstituted (C₃-C₁₂)cycloalkyl;
R_{c} and R_{d} which may be same or different at each occurrence, are independently hydrogen or substituted or unsubstituted (C₁-C₆)alkyl;
R₉ is independently selected from hydrogen, substituted or unsubstituted (C₁-C₆)alkyl, substituted or unsubstituted (C₁-C₆)haloalkyl, substituted or unsubstituted (C₁-C₆)alkoxyalkyl and substituted or unsubstituted (C₃-C₁₂)cycloalkyl;
R₁₀ is selected from hydrogen, substituted or unsubstituted (C₁-C₆)alkyl and -(CRₐR_{b})₁₋₂phenyl;
R₁₁ is substituted or unsubstituted phenyl, wherein the substituents are selected from halogen, (C₁-C₆)alkyl and -OR₉;
R₁₂ is hydrogen or substituted or unsubstituted (C₁-C₆)alkyl;
"m" is an integer ranging from 1 to 3, both inclusive; "n" is an integer ranging from 1 to 3, both inclusive; "p" is an integer ranging from 0 to 3, both inclusive; and "q" is an integer ranging from 1 to 3, both inclusive;
or a pharmaceutically acceptable salt thereof.

It should be understood that Formula (I), Formula (II), Formula (III), Formula (IV), Formula (V), and Formula (VI) structurally encompasses all tautomers, stereoisomers, enantiomers and diastereomers, including isotopes wherever applicable and pharmaceutically acceptable salts that may be contemplated from the chemical structure of the genera described herein.

Additionally, the following compounds are disclosed in this disclosure:
i) the compound of 3-((S)-2-((((R)-1-(naphthalen-1-yl)ethyl)amino)methyl) morpholino)-5-(trifluoromethyl)benzoic acid hydrochloride (see Compound 1) 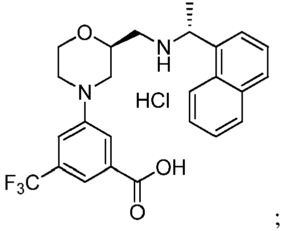
ii) the compound of 2-methyl-5-((S)-2-(2-(((R)-1-(naphthalen-1-yl)ethyl)amino) ethyl)-2H-benzo[b][1,4]oxazin-4(3H)-yl)benzoic acid hydrochloride (see Compound 2) 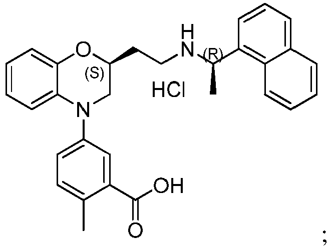
iii) the compound of 2-methyl-5-((2R,4S)-2-((((R)-1-(naphthalen-1-yl)ethyl)amino) methyl)chroman-4-yl)benzoic acid hydrochloride (Compound 3) 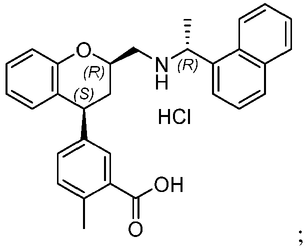
iv) the compound of 3-((1R,3S)-3-((((R)-1-(4-fluoro-3-methoxyphenyl)ethyl)amino) methyl)-1,2,3,4-tetrahydronaphthalen-1-yl)-2,6-dimethylbenzoic acid hydrochloride (see Compound 4) 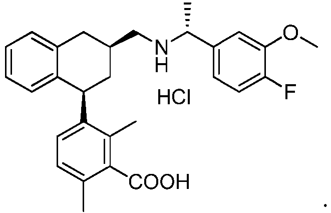
v) the compound of (R)-3-(4-fluoro-3'-(2-((1-(3-methoxy phenyl)ethyl)amino) ethoxy)-5'-(trifluoromethyl)-[1,1'-biphenyl]-3-yl)propanoic acid (see Compound 5) 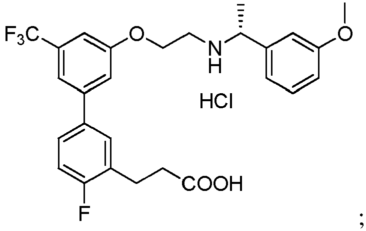 and
vi) the compound of (R)-N-((R)-1-(3-methoxyphenyl)ethyl)-1-(4-(4-(trifluoromethyl) phenyl)naphthalen-2-yl)propan-1-amine hydrochloride (see Compound 6)

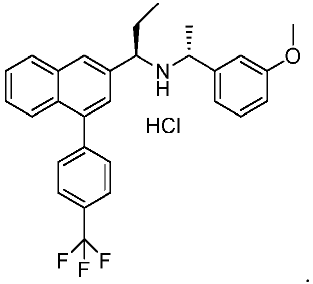

Example pharmaceutical compositions comprising the above one or more compounds are disclosed throughout this disclosure, including, for example, in the Summary section and below of this disclosure.

### Methods

The present disclosure includes a method for treating or managing secondary hyperparathyroidism associated with chronic kidney disease (CKD) in a subject in need thereof, said method comprises administering to the subject a therapeutically effective amount of one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt or hydrochloride salt thereof (such as 2-methyl-5-((2R,4S)-2-((((R)-1-(naphthalen-1-yl)ethyl)amino)methyl) chroman-4-yl)benzoic acid hydrochloride (Compound 3)). In some embodiments, the subject can be on dialysis or not on dialysis. In some embodiments, the therapeutically effective amount of the one or more compounds of Formula (I), Formula (II), Formula (III), Formula (IV), Formula (V), Formula (VI), pharmaceutically acceptable salt(s) thereof, or a combination thereof is a total daily dose amount of about 5 mg to about 50 mg, or about 10 mg to 40 mg, or about 20 mg to about 40 mg. In some embodiments, the therapeutically effective amount of the one or more compounds of Formula (I), Formula (II), Formula (III), Formula (IV), Formula (V), Formula (VI), pharmaceutically acceptable salt(s) thereof, or a combination thereof is a total daily dose amount of about 5 mg, about 10 mg, about 15 mg, about 20 mg, about 25 mg, about 30 mg, about 35 mg, about 40 mg, about 45 mg, or about 50 mg. In some embodiments, the administration of the therapeutically effective amount of the one or more compounds of Formula (I), Formula (II), Formula (III), Formula (IV), Formula (V), Formula (VI), pharmaceutically acceptable salt(s) thereof, or a combination thereof is about 25 mg once or twice daily, or about 20 mg once or twice daily. In some embodiments, the administration is oral. In another embodiment, administration does not require dose adjustment or titration of the one or more compounds of Formula (I), Formula (II), Formula (III), Formula (IV), Formula (V), Formula (VI), pharmaceutically acceptable salt(s) thereof, or a combination thereof to the subject.

The present disclosure also includes a pharmaceutical composition comprising one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt, or hydrochloride salt thereof (such as Compound 3)). In another embodiment, a kit comprising a pharmaceutical composition comprising one or more compounds of Formula (I), Formula (II), Formula (III),
Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt, or hydrochloride salt thereof (such as Compound 3)) and an instructional material for use thereof.

The present disclosure also includes a method of lowering or suppressing one or more iPTH levels in a subject suffering from secondary hyperparathyroidism associated with CKD, said method comprises administering to the subject a therapeutically effective amount of one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt or hydrochloride salt thereof (such as Compound 3)). As one skilled in the art would understand, an iPTH level is measured at a certain timepoint, and iPTH levels refers to two or more iPTH levels measured at a different timepoints. In some embodiments, the subject can be on dialysis or not on dialysis. In some embodiments, the therapeutically effective amount of the one or more compounds of Formula (I), Formula (II), Formula (III), Formula (IV), Formula (V), Formula (VI), pharmaceutically acceptable salt(s) thereof, or a combination thereof is a total daily dose amount of about 5 mg to about 50 mg, or about 10 mg to 40 mg, or about 20 mg to about 40 mg. In some embodiments, the therapeutically effective amount of the one or more compounds of Formula (I), Formula (II), Formula (III), Formula (IV), Formula (V), Formula (VI), pharmaceutically acceptable salt(s) thereof, or a combination thereof is a total daily dose amount of about 5 mg, about 10 mg, about 15 mg, about 20 mg, about 25 mg, about 30 mg, about 35 mg, about 40 mg, about 45 mg, or about 50 mg. In some embodiments, the administration of the therapeutically effective amount of the one or more compounds of Formula (I), Formula (II), Formula (III), Formula (IV), Formula (V), Formula (VI), pharmaceutically acceptable salt(s) thereof, or a combination thereof is about 25 mg once or twice daily, or about 20 mg once or twice daily. In some embodiments, the administration is oral. In some embodiments, administration does not require dose adjustment or titration of the one or more compounds of Formula (I), Formula (II), Formula (III), Formula (IV), Formula (V), Formula (VI), pharmaceutically acceptable salt(s) thereof, or a combination thereof to the subject.

The present disclosure further includes use of one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt or hydrochloride salt thereof (such as Compound 3)) in an oral formulation in the manufacture of a medicament for the treatment or management of secondary hyperparathyroidism associated with CKD in a subject in need thereof, wherein the oral formulation comprises a therapeutically effective amount of the one or more compounds of Formula (I), Formula (II), Formula (III), Formula (IV), Formula (V), Formula (VI), pharmaceutically acceptable salt(s) thereof, or a combination thereof. In some embodiments, the subject can be on dialysis or not on dialysis. In some embodiments, the therapeutically effective amount of the one or more compounds of Formula (I), Formula (II), Formula (III), Formula (IV), Formula (V), Formula (VI), pharmaceutically acceptable salt(s) thereof, or a combination thereof is a total daily dose amount of about 5 mg to about 50 mg, or about 10 mg to 40 mg, or about 20 mg to about 40 mg. In some embodiments, the therapeutically effective amount of the one or more compounds of Formula (I), Formula (II), Formula (III), Formula (IV), Formula (V), Formula (VI), pharmaceutically acceptable salt(s) thereof, or a combination thereof is a total daily dose amount of about 5 mg, about 10 mg, about 15 mg, about 20 mg, about 25 mg, about 30 mg, about 35 mg, about 40 mg, about 45 mg, or about 50 mg. In some embodiments, the administration of the therapeutically effective amount of the one or more compounds of Formula (I), Formula (II), Formula (III), Formula (IV), Formula (V), Formula (VI), pharmaceutically acceptable salt(s) thereof, or a combination thereof is about 25 mg once or twice daily, or about 20 mg once or twice daily. In some embodiments, the administration is oral. In some embodiments, administration does not require dose adjustment or titration of the one or more compounds of Formula (I), Formula (II), Formula (III), Formula (IV), Formula (V), Formula (VI), pharmaceutically acceptable salt(s) thereof, or a combination thereof to the subject.

The present disclosure further includes use of one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt or hydrochloride salt thereof (such as Compound 3)) in an oral formulation in the manufacture of a medicament for the lowering or suppression of one or more iPTH levels in a subject suffering from secondary hyperparathyroidism associated with CKD, wherein the oral formulation comprises a therapeutically effective amount of the one or more compounds of Formula (I), Formula (II), Formula (III), Formula (IV), Formula (V), Formula (VI), pharmaceutically acceptable salt(s) thereof, or a combination thereof. In some embodiments, the subject can be on dialysis or not on dialysis. In some embodiments, the therapeutically effective amount of the one or more compounds of Formula (I), Formula (II), Formula (III), Formula (IV), Formula (V), Formula (VI), pharmaceutically acceptable salt(s) thereof, or a combination thereof is a total daily dose amount of about 5 mg to about 50 mg, or about 10 mg to 40 mg, or about 20 mg to about 40 mg. In some embodiments, the therapeutically effective amount of the one or more compounds of Formula (I), Formula (II), Formula (III), Formula (IV), Formula (V), Formula (VI), pharmaceutically acceptable salt(s) thereof, or a combination thereof is a total daily dose amount of about 5 mg, about 10 mg, about 15 mg, about 20 mg, about 25 mg, about 30 mg, about 35 mg, about 40 mg, about 45 mg, or about 50 mg. In some embodiments, the administration of the therapeutically effective amount of the one or more compounds of Formula (I), Formula (II), Formula (III), Formula (IV), Formula (V), Formula (VI), pharmaceutically acceptable salt(s) thereof, or a combination thereof is about 25 mg once or twice daily, or about 20 mg once or twice daily. In some embodiments, the administration is oral. In some embodiments, administration does not require dose adjustment or titration of the one or more compounds of Formula (I), Formula (II), Formula (III), Formula (IV), Formula (V), Formula (VI), pharmaceutically acceptable salt(s) thereof, or a combination thereof to the subject.

The present disclosure further includes a method of treating or managing secondary hyperparathyroidism associated with CKD in a subject in need thereof, said method comprising:
(a) identifying a subject having secondary hyperparathyroidism associated with CKD in need of treatment or management, characterized by an elevated baseline iPTH concentration level in the subject; and
(b) administering to the subject a therapeutically effective amount of one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt or hydrochloride salt thereof (such as Compound 3)).

Patients who have a baseline iPTH concentration level of about 10 pg/ml to about 65 pg/ml are considered patients with a normal baseline iPTH concentration level. However, patients with a baseline iPTH concentration level of more than about 65 pg/ml are considered patients with an elevated baseline iPTH concentration level. By way of example, patients on dialysis having a baseline iPTH concentration level of at least about 300 pg/ml to about 1250 pg/ml are considered patients with an elevated baseline iPTH concentration level. A patient's baseline iPTH concentration level is measured prior to first dose administration of the one or more compounds of Formula (I), Formula (II), Formula (III), Formula (IV), Formula (V), Formula (VI), pharmaceutically acceptable salt(s) thereof, or a combination thereof. Two blood samples are collected from the patient before the first dose administration, the iPTH concentration level for each sample is determined, and the higher of these 2 iPTH concentration level values is determined as the patient's baseline iPTH concentration level.

In some embodiments, the subject can be on dialysis or not on dialysis. In another embodiment, the therapeutically effective amount of the one or more compounds of Formula (I), Formula (II), Formula (III), Formula (IV), Formula (V), Formula (VI), pharmaceutically acceptable salt(s) thereof, or a combination thereof is a total daily dose amount of about 5 mg to about 50 mg, or about 10 mg to 40 mg, or about 20 mg to about 40 mg. In some embodiments, the therapeutically effective amount of the one or more compounds of Formula (I), Formula (II), Formula (III), Formula (IV), Formula (V), Formula (VI), pharmaceutically acceptable salt(s) thereof, or a combination thereof is a total daily dose amount of about 5 mg, about 10 mg, about 15 mg, about 20 mg, about 25 mg, about 30 mg, about 35 mg, about 40 mg, about 45 mg, or about 50 mg. In some embodiments, the administration of the therapeutically effective amount of the one or more compounds of Formula (I), Formula (II), Formula (III), Formula (IV), Formula (V), Formula (VI), pharmaceutically acceptable salt(s) thereof, or a combination thereof is about 25 mg once or twice daily, or about 20 mg once or twice daily. In some embodiments, the administration is oral. In some embodiments, administration does not require dose adjustment or titration of the one or more compounds of Formula (I), Formula (II), Formula (III), Formula (IV), Formula (V), Formula (VI), pharmaceutically acceptable salt(s) thereof, or a combination thereof to the subject.

In another embodiment, a method of treating or managing secondary hyperparathyroidism associated with CKD in a subject in need thereof, said method comprising:
(a) identifying a subject having secondary hyperparathyroidism associated with CKD in need of treatment or management, characterized by a baseline iPTH concentration level in the subject, wherein the baseline iPTH concentration level is in the range of about 110 pg/ml to about 1500 pg/ml, or about 300 pg/ml to about 1250 pg/ml; and
(b) administering to the subject a therapeutically effective amount of one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt or hydrochloride salt thereof (such as Compound 3)).

In some embodiments, the subject can be on dialysis or not on dialysis. In some embodiments, the therapeutically effective amount of the one or more compounds of Formula (I), Formula (II), Formula (III), Formula (IV), Formula (V), Formula (VI), pharmaceutically acceptable salt(s) thereof, or a combination thereof is a total daily dose amount of about 5 mg to about 50 mg, or about 10 mg to 40 mg, or about 20 mg to about 40 mg. In some embodiments, the therapeutically effective amount of the one or more compounds of Formula (I), Formula (II), Formula (III), Formula (IV), Formula (V), Formula (VI), pharmaceutically acceptable salt(s) thereof, or a combination thereof is a total daily dose amount of about 5 mg, about 10 mg, about 15 mg, about 20 mg, about 25 mg, about 30 mg, about 35 mg, about 40 mg, about 45 mg, or about 50 mg. In some embodiments, the administration of the therapeutically effective amount of the one or more compounds of Formula (I), Formula (II), Formula (III), Formula (IV), Formula (V), Formula (VI), pharmaceutically acceptable salt(s) thereof, or a combination thereof is about 25 mg once or twice daily, or about 20 mg once or twice daily. In some embodiments, the administration is oral. In another embodiment, administration does not require dose adjustment or titration of the one or more compounds of Formula (I), Formula (II), Formula (III), Formula (IV), Formula (V), Formula (VI), pharmaceutically acceptable salt(s) thereof, or a combination thereof to the subject.

In another embodiment, a method for treating or managing secondary hyperparathyroidism associated with CKD in a subject in need thereof, said method comprises administering to the subject a therapeutically effective amount of a calcimimetic. In some embodiments, the therapeutically effective amount of the calcimimetic is a total daily dose amount of about 5 mg to about 50 mg, or about 10 mg to 40 mg, or about 20 mg to about 40 mg. In some embodiments, the therapeutically effective amount of the calcimimetic is a total daily dose amount of about 5 mg, about 10 mg, about 15 mg, about 20 mg, about 25 mg, about 30 mg, about 35 mg, about 40 mg, about 45 mg, or about 50 mg. In some embodiments, the administration of the therapeutically effective amount of the calcimimetic is about 25 mg once or twice daily, or about 20 mg once or twice daily. In some embodiments, the administration is oral. In some embodiments, administration does not require dose adjustment or titration of the calcimimetic to the subject. In some embodiments, secondary hyperparathyroidism is treated or managed without inducing hyperphosphatemia or hypocalcemia in the subject. In some embodiments, the calcimimetic comprises one or more compounds of Formula (I), Formula (II), Formula (III), Formula (IV), Formula (V), Formula (VI), pharmaceutically acceptable salt(s) thereof, or a combination thereof, or 2-methyl-5-((2R,4S)-2-((((R)-1-(naphthalen-1-yl)ethyl)amino)methyl) chroman-4-yl)benzoic acid, an internal salt or hydrochloride salt thereof (such as Compound 3).

Subjects with secondary hyperparathyroidism associated with CKD, include subjects diagnosed or having Stage 3b CKD, Stage 4 CKD or Stage 5 CKD. Preferably, the subject is a patient having Stage 5 CKD.

Subjects with secondary hyperparathyroidism associated with CKD, include subjects with a baseline iPTH concentration level ranging from about 110 pg/ml to about 1500 pg/ml, or about 300 pg/ml to about 1600 pg/ml, or about 110 pg/ml to about 1350 pg/ml. Preferably, a baseline iPTH concentration level ranging from about 300 pg/ml to about 1250 pg/ml. A patient on dialysis can have a baseline iPTH concentration level of about 300 pg/ml to about 1250 pg/ml. A patient not on dialysis can have a baseline iPTH concentration level of about 110 pg/ml to about 1350 pg/ml.

In another embodiment, in any one of the methods or uses disclosed herein, the subject's iPTH level can be lowered by at least about 30% compared to the baseline iPTH concentration level, or at least about 15% compared to the baseline iPTH concentration level, or about 15% to about 30% compared to the baseline iPTH concentration level.

In another embodiment, in any one of the methods or uses disclosed herein one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, internal salt, or hydrochloride salt thereof (Compound 3)) is administered as a pharmaceutical composition. For example, the pharmaceutical composition can be in the form of a tablet or capsule. In some embodiments, the pharmaceutical composition comprises about 5 mg, about 10 mg, about 15 mg, about 20 mg, about 25 mg, about 30 mg, about 35 mg, about 40 mg, or about 50 mg of the one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt or hydrochloride salt thereof (such as Compound 3)). In some embodiments, the pharmaceutical composition is administered to the subject once daily, twice daily, or more than twice daily. In some embodiments, the pharmaceutical composition is administered to the subject in a total daily dose amount of the one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt or hydrochloride salt thereof (such as Compound 3)) of about 10 mg to about 40 mg; most preferably, about 20 mg to about 40 mg. Preferably, the pharmaceutical composition is administered to the subject in a total daily dose amount of the one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt or hydrochloride salt thereof (such as Compound 3)) of 25 mg once daily or 20 mg twice daily.

In another embodiment, pharmaceutical compositions suitable for oral administration may be presented as discrete units such as tablets, cachets, or capsules (each containing a predetermined amount of the active ingredient). The tablet, cachet, or capsule can comprise one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt or hydrochloride salt thereof (such as Compound 3)) and at least one ingredient selected from the group consisting of a diluent, a disintegrant, a binder, a lubricant, a surfactant, glidant, and a combination thereof. A tablet or cachet may be made by compression or molding. Compressed tablets, cachets or granules in a capsule may be prepared by compressing, in a suitable machine, the active ingredient in a free-flowing form such as a powder or granules, optionally mixed with diluent, a disintegrant, a binder, a lubricant, a surfactant and/or glidant. Molded tablets, cachets or granules in a capsule may be made by molding, in a suitable machine, a mixture of the powdered compound moistened with an inert liquid diluent. The tablet, cachet or granules in a capsule may be optionally coated or scored. The tablet, cachet or capsule may be formulated to provide an immediate release of the active ingredient(s) (such as Compound 3) therein. Dyestuffs, pigments, and/or dragee coatings can be added to the tablet or cachet for identification or to characterize different Compound 3 doses. In another embodiment, the tablet, cachet or each granule in a capsule is round (or circle), disc, sphere, oval, oblong, capsule-shaped, square, rectangle, triangle, pentagon, hexagon, octagon, bullet, diamond, or arrowhead. The tablet, cachet or granules in the capsule can one or more layers. For example, a tablet, cachet or each granule in the capsule can have a core layer and an outer layer that is outside of the core layer.

In another embodiment, pharmaceutical compositions that can be administered orally as hard shell capsules. The hard shell capsules of a suitable size may be made, e.g., from hard gelatin and/or hydroxypropyl methycellulose (HPMC). The size of the hard shell capsule can be size 0 or size 00. In another embodiment, the capsule comprises one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt or hydrochloride salt thereof (such as Compound 3)), a starch (such as starch 1500 LM or the like), microcrystalline cellulose and crospovidone. In another embodiment, the capsule comprises about 35% to about 38% w/w of the one or more compounds of Formula (I), Formula (II), Formula (III), Formula (IV), Formula (V), Formula (VI), pharmaceutically acceptable salt(s) thereof, or a combination thereof (such as 2-methyl-5-((2R,4S)-2-((((R)-1-(naphthalen-1-yl)ethyl)amino)methyl) chroman-4-yl)benzoic acid, an internal salt or hydrochloride salt thereof (such as Compound 3)).

In another embodiment, pharmaceutical compositions that can be administered orally as push-fit capsules made of gelatin. In another embodiment, the hard shell or push-fit capsules can comprise at least one ingredient selected from the group consisting of a filler (such as lactose), a binder (such as starches), and/or a lubricant (such as talc or magnesium stearate), a stabilizer, and a combination thereof. All formulations for oral administration should be dosages suitable for such administration.

In another embodiment, the pharmaceutical composition disclosed in this application can be used to treat or manage a disease in a subject in need thereof, wherein the disease is hyperparathyroidism (including hyperparathyroidism, secondary hyperparathyroidism or tertiary hyperparathyroidism), chronic renal failure (with or without dialysis), chronic kidney disease (with or without dialysis) and their complications, or a combination thereof. Uses and methods of treatment or management regarding the above are also included herein.

In another embodiment, the pharmaceutical composition disclosed in this application can be used to treat or manage a disease in a subject in need thereof, wherein the disease is selected from the group consisting of parathyroid adenoma; parathyroid hyperplasia; parathyroid carcinoma; vascular or valvular calcification; abnormal calcium homeostasis; hypercalcemia; abnormal phosphorous homeostasis; hypophosphatemia; bone-related diseases or complications arising due to hyperparathyroidism, chronic kidney disease or parathyroid carcinoma; post-renal transplantation bone loss; osteitis fibrosa cystica; adynamic bone disease; renal bone diseases; cardiovascular complications arising due to hyperparathyroidism or chronic kidney disease; malignancies in which (Ca2+)e ions are abnormally high; cardiac, renal or intestinal dysfunctions; podocyte-related diseases; abnormal intestinal motility; diarrhea; and augmenting gastrin or gastric acid secretion to directly or indirectly benefit in atrophic gastritis or to improve absorption of pharmacological compounds, drugs or supplements from gastro-intestinal tract by augmenting gastric acidity. Uses and methods of treatment or management regarding the above are also included herein.

The invention has been described broadly and generically herein. Each of the narrower species and subgeneric groupings falling within the generic disclosure also form part of the invention. This includes the generic description of the invention with a proviso or negative limitation removing any patient matter from the genus, regardless of whether or not the excised material is specifically recited herein.

Other embodiments are within the following claims. In addition, where features or embodiments of the invention are described in terms of Markush groups, those skilled in the art will recognize that the invention is also thereby described in terms of any individual member or subgroup of members of the Markush group.

### EXAMPLES

The compounds described herein may be prepared by synthetic organic chemistry methods. Further, in the schemes described herein, where specific bases, acids, reagents, solvents, coupling agents, etc., are mentioned, it is understood that other bases, acids, reagents, solvents, coupling agents etc., unless otherwise specified, may also be used and are therefore included within the scope of the invention. Variations in reaction conditions, for example, temperature and/or duration of the reaction, which may be used as known in the art, are also within the scope of the invention. All the isomers of the compounds described in these schemes, unless otherwise specified, are also encompassed within the scope of this invention.

### Example 1 - Preparation of Representative Compositions

The pharmaceutical compositions in tablet form containing a total weight of 100 mg/tablet and 500 mg/tablet were produced at 40 kg and 200 kg scale, respectively, by the following composition ratios given in Table 1 below. A 100 mg tablet contained an equivalent amount of 5 mg free base, whereas a 500 mg tablet contained an equivalent amount of 25 mg free base. Both pharmaceutical compositions were prepared with following components mentioned as in Table 1 below.

**Table 1**

| **S. No** | **Components** | **Batch size** | | **Quantities per 5 mg tablet (mg)** | **Quantities per 25 mg tablet (mg)** |
|---|---|---|---|---|---|
| | | **40 kg** | **200kg** | | |
| 1. | Compound 1, Compound 2, Compound 3, Compound 4, Compound 5, or Compound 6 | 2.160 | 10.8 | 5.4 | 27 |
| 2. | Starch 1500 Partially Pregelatinized Maize Starch | 16.360 | 81.8 | 40.9 | 204.5 |
| 3. | Microcrystalline Cellulose (AVICEL^{®} PH 102) | 12.280 | 61.4 | 30.7 | 153.5 |
| 4. | Crospovidone (Kollidon^{®} CL-SF) | 4.000 | 20 | 10 | 50 |
| 5. | Povidone K-30 (Kollidon^{®} K-30) | 2.400 | 12 | 6 | 30 |
| 6. | Colloidal Silicon Dioxide, (Aerosil ^{®} 200 Pharma) | 0.800 | 4 | 2 | 10 |
| 7. | Sodium Lauryl Sulfate (SLS) | 0.800 | 4 | 2 | 10 |
| 8. | Talc (197 Pharma) | 0.400 | 2 | 1 | 5 |
| 9. | Magnesium Stearate (Hyqual^{®}, Vegetable Source) | 0.800 | 4 | 2 | 10 |
| | Total | 80 | 200 | 100mg | 500mg |

The v-blender speed was verified and then added half of the starch 1500 to Colloidal Silicon Dioxide and mixed in v-blender. The previously weighed Compound 1, Compound 2, Compound 3, Compound 4, Compound 5, or Compound 6 in a glove bag was then added into the v-blender. After that the remaining half of the starch 1500 was added into the same glove bag and rinsed then added into the v-blender. Blending was continued for about 18 min at 20 RPM (± 2 RPM). This premix blend was passed through a Fitz-mill set-up knifes forward at 3000 RPM with a 0093 (mm) screen size followed by about 1.0 kg of Microcrystalline Cellulose. Further the remaining Microcrystalline Cellulose 11.18 kg, Sodium Lauryl Sulfate (SLS), Crospovidone, Kollidon^{®} CL-SF, Povidone (Kollidon^{®} K-30) and Talc (197 Pharma) were screened through a 20 (mm) mesh then added into the v-blender followed by the milled pre-mix blend. All these materials were blended for 18 min at 20 RPM (± 2 RPM). After that, approximately 700 g of blended material was added to Magnesium Stearate and hand mixed then passed through a 30 (mm) mesh hand screen. This Magnesium Stearate was added into v-blender and blended for an additional 5 min at 20 RPM (± 2 RPM). Following the blending and milling, the tablet compression was carried out using 1/4 inch Standard Concave B Tooling for the production of 5 mg tablets and B Tooling (0.6496" x 0.2992") Capsule Shaped by following the parameters mentioned in Table 2 below.

**Table 2**

| **Tableting Specifications** | | |
|---|---|---|
| **Parameter** | **5 mg Tablets** | **25 mg Tablets** |
| Tooling Description | 1/4 inch Standard Concave B Tooling | B Tooling 0.6496" x 0.2992" Capsule Shaped |
| Individual Target Tablet Weight | 100 mg | 500 mg |
| Individual Tablet Weight Control Range | 93.0 mg to 107.0 mg (± 7%) | 465.0 mg to 535.0 mg (± 7%) |
| Individual Tablet Weight Action Range | 95.0 mg to 105.0 mg (± 5%) | 475.0 mg to 525.0 mg (± 5%) |

Following the compression of tablets, the obtained tablets were coated using standard Opadry^{®} II (polyvinyl alcohol, titanium dioxide, talc, polyethylene glycol 3350, iron oxide yellow, and iron oxide red), to the amount of about 4% in a total theoretical weight of the tablet using the tablet coating procedures. The compressed tablets were coated with a coating suspension comprising 10% wt. solids.

Table 3 below shows a representative blend for 90 mg capsules comprising Compound 3.

### Example 2 - Pharmacokinetic Analysis of Representative Compositions

**Table 3**

| **S. No** | **Components** | **Quantities per 90 mg blend** | **For 2 gm** | **w/w% in formulation** |
|---|---|---|---|---|
| | | | **In gm** | |
| 1. | Compound 3 | 96.75 | 0.774 | 38.7 |
| 2. | Starch 1500 LM | 75.75 | 0.606 | 30.3 |
| 3. | Microcrystalline Cellulose (such as AVICEL PH 102) | 50 | 0.4 | 20 |
| 4. | Crospovidone (such as Kollidon CL-SF) | 15 | 0.12 | 6 |
| 5. | Povidone (such as Kollidon K-30) | 5 | 0.04 | 2 |
| 6. | Colloidal Silicon Dioxide (such as Aerosil 200 Pharma) | 2.5 | 0.02 | 1 |
| 7. | Sodium Lauryl Sulfate (SLS) | 2.5 | 0.02 | 1 |
| 8. | Magnesium Stearate | 2.5 | 0.02 | 1 |
| | Capsule Fill Weight | 250 | | 100 |
| | Total | -- | 2.0 | -- |
| | Capsule Size | -- | -- | 0 |

Male Sprague Dawley Rats (weight 225 ± 25 gm; aged 8 to 10 weeks) were administered orally with the formulation. Each of the tested compounds was dosed at 5 mg/kg at a dose volume of 1 mL/kg. Blood samples were collected at time points 0.25, 0.5, 1, 2, 4, 6, 8 and 24 hours and analyzed the plasma concentrations of administered compound and calculated PK parameters. The plasma concentrations of the tested compounds were measured by liquid chromatography with tandem mass spectrometry (LCMS-MS) using plasma precipitation extraction method. The plasma concentration-time data was subjected to Non-Compartmental Analysis using Phoenix WinNonlin^{®} (Version 5.3) software to assess the pharmacokinetic parameters. Peak plasma concentrations (Cmax) and time for the peak plasma concentrations (Tmax) were the observed values. The area under the concentration time curve (AUC) was calculated by linear trapezoidal rule.

Pharmacokinetic parameters such as area under the curve (AUC) and Concentration at its maximum (Cmax) were summarized in Table 4 below.

**Table 4**

| **PK Parameter** | **AUC₀₋₁ (h* µM)** | **Cₘₐₓ (µM)** | **Tₘₐₓ (h)** |
|---|---|---|---|
| Compound 1 | 7.3 ± 0.5 | 3.6 ± 1 | 0.25-1 |
| Compound 2 | 16 ± 3 | 4.9 ± 3 | 0.25 |
| Compound 3 | 15 ± 4 | 3.4 ± 1 | 0.5 - 2 |
| Compound 4 | 5.5 ± 1 | 1.5 ± 1 | 0.5 - 2 |

The results indicated that the pharmaceutical compositions of this disclosure, when orally administered to a subject, distribute the compound throughout the body of the subject.

### Example 3 - Therapeutic Efficacy, Pharmacokinetics (PK), Pharmacodynamics (PD) and Safety of Representative Compound

A randomized, double-blind, placebo-controlled phase 2 clinical study was conducted to assess the efficacy, pharmacokinetics (PK), pharmacodynamics (PD) and safety of 2-methyl-5-((2R,4S)-2-((((R)-1-(naphthalen-1-yl)ethyl)amino)methyl) chroman-4-yl)benzoic acid, hydrochloride (Compound 3), as a monotherapy in patients with CKD with secondary hyperparathyroidism (SHPT). The patients enrolled in the study were either on dialysis or not on dialysis. Patients could continue taking doses (whether stable dosing or otherwise) of calcium supplements, phosphate binders, vitamin D, vitamin D analogues or a combination thereof for the treatment of SHPT in combination with Compound 3. Patients can take at least one calcium supplement, phosphate binder, vitamin D, vitamin D analogue or a combination thereof concurrently or concomitantly with Compound 3. Dosing for such calcium supplements, phosphate binders, vitamin D, vitamin D analogues or a combination thereof for the treatment of SHPT can be individualized based on patient's iPTH levels, and/or can correlate with doses presently used for the treatment of SHPT.

The investigational tablet was in the form of a round tablet with 5 mg of Compound 3 or a capsule-shaped tablet with 25 mg of Compound 3, both plain-coated tablets for oral administration. A matching placebo for 5 mg and 25 mg tablets were provided for oral administration.

The study was conducted in two parts: Pharmacokinetic-Pharmacodynamic (PK-PD) Study and Main Study, as discussed further herein.

### A. PK-PD Study

This was an open-label, non-randomized, single arm study enrolling 10 patients of which 6 patients were on dialysis and 4 were not on dialysis. All patients received 25 mg Compound 3, once daily for 8 days, and thereafter underwent PK-PD assessments. All 6 patients (100%) on dialysis group completed the study while 2 patients out of 4 (50%) on not on dialysis group completed the study. One objective of this study were to determine PK parameters maximum concentration, time to maximum concentration, area under the curve (AUC) (AUC from time 0 up to the last quantifiable concentration; AUC from time 0 to infinity; AUC from time 0 to 24 hours), half-life, apparent clearance, and apparent volume of distribution from blood samples collected at scheduled time-points. Yet another objective of this study was to determine PD parameters mean absolute change and mean percentage change from baseline in corrected total serum calcium and phosphorus.

### B. Main Study

This was a double-blind, randomized, placebo-controlled, multicenter study with an additional non-randomized open-label arm conducted in patients with secondary hyper-parathyroidism (SHPT) associated with CKD which was further stratified in 2 groups: on dialysis and not on dialysis. The iPTH levels of patients in the "on dialysis" group were in a range of 428.2 pg/ml to 1240.6 pg/ml (mean 857.76 pg/ml) while those in the "not on dialysis" group were in a range of 125.0 pg/ml to 654.7 pg/ml (mean 297.83 pg/ml).

Total 179 patients were enrolled in this study of which 108 patients were on dialysis and 71 were not on dialysis. Patients of on dialysis group were further randomized to 3 subgroups administering 10 mg Compound 3, 25 mg Compound 3, or placebo once daily in a double blind fashion enrolling 35 patients, 36 patients and 18 patients, respectively, and an additional non-randomized open label arm enrolling 19 patients receiving 20 mg Compound 3, twice daily. *See* Table 5 below. A total of 98 patients completed the study. 10 patients discontinued the study. None of the patients discontinued the study due to adverse drug reactions.

**Table 5 - Main Study: On Dialysis Cohort Treatments**

| **Double-Blind Treatment** | | | **Open-Label Treatment** |
|---|---|---|---|
| **Arm 1** | **Arm 2** | **Arm 3** | **Arm 4** |
| 10 mg Compound 3 (2 tablets of 5 mg Compound 3) | 25 mg Compound 3 (1 tablet) | Placebo 5 mg (2 tablets) | 20 mg Compound 3(4 tablets of 5 mg Compound 3) |
| Placebo 25 mg (1 tablet) | Placebo 5 mg (2 tablets) | Placebo 25 mg (1 tablet) | |
| 3 tablets QD* | 3 tablets QD | 3 tablets QD | 4 tablets BID* |
| | | | Total: 8 tablets |

| | | | |
|---|---|---|---|
| *BID = twice daily; QD = once daily | | | |

Patients on not on dialysis group were randomized to two treatment subgroups administering 10 mg Compound 3, or placebo once daily in a double blind fashion enrolling 37 and 18 patients, respectively, and an additional non-randomized open label arm enrolling 16 patients receiving 5 mg Compound 3, twice daily. *See* Table 6 below. 4 patients discontinued the study. None of the patients discontinued the study due to adverse drug reactions.

**Table 6 - Main Study: Not On Dialysis Cohort Treatments**

| **Double-Blind Treatment** | | **Open-Label Treatment** |
|---|---|---|
| **Arm 1** | **Arm 2** | **Arm 3** |
| 10 mg Compound 3 (2 tablets of 5 mg Compound 3) | Placebo 5 mg (2 tablets) | 5 mg Compound 3 (1 tablet) |
| 2 tablets QD* | 2 tablets QD | 1 tablets BID* |
| | | Total: 2 tablets |

| | | |
|---|---|---|
| *BID = twice daily; QD = once daily | | |

Table 7 below shows the duration of treatment concerning the PK-PD Study and Main Study:

**Table 7 - Duration of Treatment for PK/PD and Efficacy Studies**

| **Study Period** | **PK-PD Study** | **Efficacy Study** |
|---|---|---|
| Screening¹ | Maximum 30 days | Maximum 30 days |
| Treatment | 8 days (Day 1 to Day 8)² | 90 days |
| Follow Up | 7 days post the last dose | 7 days post the last dose |
| | EOT*: Day 8 | EOT: Day 90 |
| | EOS*: Day 15 | EOS: Day 97 |

| | | |
|---|---|---|
| *EOS = End of Study; EOT = End of Treatment ¹ Screening period included washout for Cinacalcet. Patients who were on Cinacalcet and were justified to be included in the study based on investigator's discretion were given adequate washout viz, should have stopped Cincalcet (last dose) at least 21 days prior to Day 1 of the study. ² PK-PD profiling was done for 10 days (Day 1 to Day 10). | | |

The efficacy, PK, PD and safety of Compound 3, as a monotherapy in patients with CKD with secondary hyperparathyroidism (SHPT), were assessed. One objective of the study was to evaluate the mean percentage change in iPTH from baseline at the end of 90 days. Yet another objective of the study was to determine the proportion of patients with >30% reduction in iPTH from baseline at the end of 90 days. Yet another objective of the study was to ensure safety of patients.

For efficacy assessments, the primary efficacy assessment included measurement of iPTH and the exploratory efficacy assessment included measurement of parathyroid gland size at scheduled time points. Blood samples for measurement of iPTH were collected during Day -30 to Day -1 (Screening Period); Day 1; Day 8 +/- 3 days; Day 15 +/- 3 days; Day 30 +/-3 days; Day 60 +/- 3 days; and Day 90 +/- 3 days (EOT/Early Discontinuations). Two blood samples were collected during Screening Period and higher of these 2 values was considered as a baseline value. During treatment period of the Main Study, blood samples were collected on Day 30, Day 60, and Day 90. If the blood sample collected on Day 90 could not be analysed for any reason, the second blood sample had to be collected within 3 days of end of treatment (EOT). Chemiluminescence microparticles immunoassay (CMIA) (such as Abbott's ARCHITECT^{®} Intact PTH assay) was used to quantify iPTH in serum and plasma.

As for exploratory efficacy assessment, parathyroid gland size was measured by ultrasonography (USG) in eligible patients during screening and at EOT (Day 90) whenever feasible.

For PK-PD assessments, blood samples for PK assessments were collected at scheduled time points (Screening Period; Day 1 (Pre-dose and post-dose); Day 2; Day 8 EOT (Pre-dose and post-dose); Day 9; Day 10; and Day 15 +/- 3 days (Follow-up visit)) in the PK-PD study. The PD profiling was done in parallel to the PK profiling in the PK-PD study. Blood samples (approximately 1 x 4 mL) for PK assessments were collected by venipuncture or cannulation at the selected time points into tubes containing dipotassium ethylenediaminetetraacetic acid (K2EDTA) anti-coagulant. The collected blood sample were kept on ice until centrifugation and later centrifuged at 4oC at 5000 rpm for 10 minutes and the separated plasma collected in 2 tubes/aliquots. The plasma samples were stored at -20oC or lower at the clinical centre until they were shipped for analysis. Blood samples (approximately 1 x 8 mL) for PD assessments were collected in PK-PD study and Main Study at selected time points The Main Study's selected time points were Screening Period; Day 1; Day 8 +/- 3 days; Day 15 +/- 3 days; Day 30 +/-3 days; Day 60 +/- 3 days; and Day 90 +/- 3 days (EOT/Early Discontinuations). Blood samples (approximately 1 x 5 mL) for exploratory PD assessments for PK-PD study and Main Study were collected at selected time points.

For safety assessments, safety was assessed through the summary of adverse events (AE), physical examinations and electrocardiogram (ECG) measurements, vital signs (e.g., blood pressure, pulse rate, respiratory rate and body temperature), body weight and laboratory data (e.g., haemtaology, biochemistry and urinalysis).

All AEs (both serious and non-serious) were derived from spontaneous, unsolicited reports of patients, by observation and by routine open questions. The AE reporting extended from date of informed consent until completion of the final visit. Any ongoing AE at EOS visit was followed until the outcome was evident and resolved, clinically stabilized or a plausible explanation was found. Pre-existing diseases including deranged laboratory values (before participating in the study) were not considered AEs, unless the disease worsened during the study period. Signs and symptoms clearly associated with the disease under study (including symptoms of disease progression) were reported as AEs if they were newly emergent, or were determined as severe or worsening, or if deterioration of disease-related signs and symptoms to be caused directly by the study drug.

Physical examination, vital signs (measured in a sitting position after resting for at least 3 minutes), ECG (recorded by qualified personnel after the patient had been resting in the supine position for at least 3 minutes) and clinical laboratory examinations were done as part of safety assessments. Abnormal laboratory values/ECGs/vital signs/physical examination were reported as AEs only if the abnormality was clinically relevant or significant or believed that the abnormality must be reported as an AE. Any dose (and associated symptoms) given to the patient that exceeded the dose prescribed to the patient, at a minimum, was recorded as a non-serious AE in the patient file and CRF/electronic CRF. Any case of overdose leading to an AE or SAE had to be reported to medical monitor according to reporting requirements. In case of an accidental overdose, the patient had to be monitored by for any adverse clinical events, as deemed necessary.

The Kidney Disease Outcomes Quality Initiative (KDOQI) guidelines are the highly practiced and referred-to guidelines for evaluation and management of CKD. The targets mentioned in these guidelines are considered in this study as efficacy endpoints. Moreover, the primary and secondary endpoints chosen for the study are the ones used during development of calcimimetic product and well accepted by various regulatory agencies. Similarly, other efficacy and safety parameters proposed are in line with the conventional endpoints used in such type of studies (for example, Cinacalcet Phase 2 studies were conducted with similar endpoints).

Change from baseline to all measured and relevant time points was calculated. The baseline value for this study was the higher of the 2 values obtained during screening period for serum iPTH concentration, corrected total serum calcium and phosphorous. The baseline value for all other parameters was the last valid and planned visit value prior to first dose of IP in the study. For continuous data, summary statistics included the number, arithmetic mean, standard deviation (SD), median, minimum and maximum. For categorical data, frequency and percentage were presented.

Summary of statistical analyses is provided in Table 8. Prior and concomitant medications were coded using the World Health Organization-Drug Dictionary classification and a listing was prepared. All concomitant medications received after the start of dosing were summarized. Study compliance and the extent of exposure to the study medication were summarized and listed. All patients who discontinued from treatment were listed and the reasons for discontinuation were tabulated.

**Table 8 - Summary of Statistical Analyses**

| **Analysis** | **Methods** |
|---|---|
| Efficacy Endpoints | Descriptive statistics was used to summarise efficacy endpoints. |
| | Mean percentage change in iPTH from baseline at the end of 90 days was compared using the ANCOVA model with treatment as factor and baseline result as the covariate. The difference in the estimated mean percentage change in iPTH from baseline at the end of 90 days between each dose of Compound 3 and Placebo along with the corresponding 95% CI was calculated based on the ANCOVA model |
| | All categorical secondary efficacy endpoints were analysed using Chi-square test separately for on-dialysis and not on dialysis patients. A 95% CI was provided for individual treatment proportion and also for difference in proportions between each dose of Compound 3 and Placebo. A Fisher's Exact test was used if the expected cell counts were <5. |
| | Exploratory efficacy endpoint was analyzed using ANCOVA model. |
| PK Endpoints | PK calculations were performed using appropriate software, e.g., Phoenix^{™} WinNonlin^{®} (Pharsight Corporation) and/or SAS^{®} (SAS Institute Inc.). |
| | Plasma concentrations and derived PK parameters of Compound 3 were presented descriptively by day and time point (plasma concentrations only) for the PK analysis population. |
| | Concentration values below the lower limit of quantification were taken as 0 for descriptive statistics which included the number of observations, arithmetic mean, SD, minimum and maximum. |
| | PK parameters were summarized by the number. |
| PD Endpoints | The PD and exploratory PD parameters were summarized by the number of observations, arithmetic mean, SD, CV, median, minimum and maximum for Days 1 and 8. |
| | Mean absolute change and mean percentage change in corrected total serum calcium and phosphorus from baseline was compared between each dose of Compound 3 and Placebo using ANCOVA model with treatment as factor and baseline result as the covariate by each visit. This analysis was performed separately for patients on-dialysis and not on dialysis based on PD population. |
| | Mean absolute change and mean percentage change in corrected calcium, phosphorus, calcitonin and FGF23 levels at 2 to 4 hours post-dose on Day 90 was compared between each dose of Compound 3 and Placebo using ANCOVA model with treatment as factor and baseline result as the covariate. This analysis was performed separately for patients on-dialysis and not on dialysis based on PD population. |
| | All PD parameters and PD derived parameters for corrected total serum calcium etc. were summarized by descriptive statistics in summary tables for observed concentrations, absolute change and percentage change from baseline values based on PD population at Days 30, 60 and 90. |
| Safety Endpoints | All AEs were coded by SOC and PT according to the Medical Dictionary for Regulatory Activities Version 21.1. Events were classified as TEAEs if they started on or after the date of the first dose of study drug or aggravated in severity following the administration of study drug. The number of AEs and the number and percentage of patients experiencing AEs were summarized by severity and relationship to the study medication. The SAEs and AEs leading to premature study discontinuation were summarized. |
| | Shift tables for changes from baseline to each visit in the 12-lead ECG and in the physical examination were presented by the incidence of a clinically significant finding |
| | Descriptive statistics was performed for those laboratory parameters on a continuous scale for the raw scores and change from baseline at each visit. Shift tables for the hematology and biochemistry laboratory parameters comparing values (low, normal and high) using the standard reference ranges was presented for the baseline laboratory measurement vs. the endpoint measurement. Patients with an incidence of clinically significant abnormalities that occurred during treatment as well as those that occurred prior to the start of the study treatment were presented by treatment. |
| | Changes in vital signs (pulse rate, respiratory rate, axillary body temperature, blood pressure) and body weight from baseline to each study visit were summarized. |

| | |
|---|---|
| AE = adverse event; ANCOVA = analysis of covariance; CI = confidence; SAE - serious adverse event; TEAE = Treatment Emergent Adverse Event | |

### Results

In the PK-PD study, peak effect of Compound 3 on iPTH suppression was observed within 4 hours of dosing on Day 1 (>80.0% from baseline in patients on dialysis and not on dialysis) and Day 8 (>70.0% and >85.0% from baseline in patients on dialysis and not on dialysis, respectively).

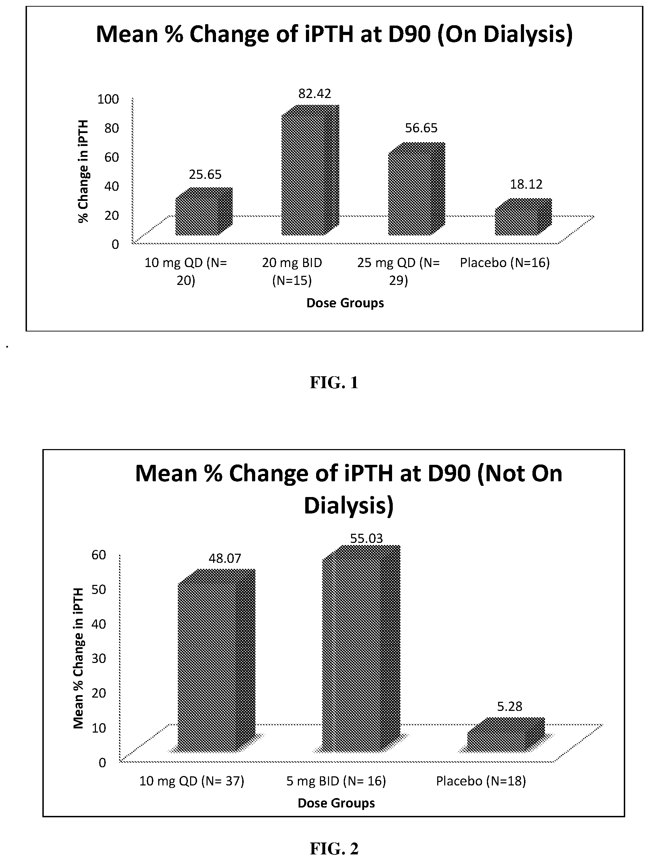

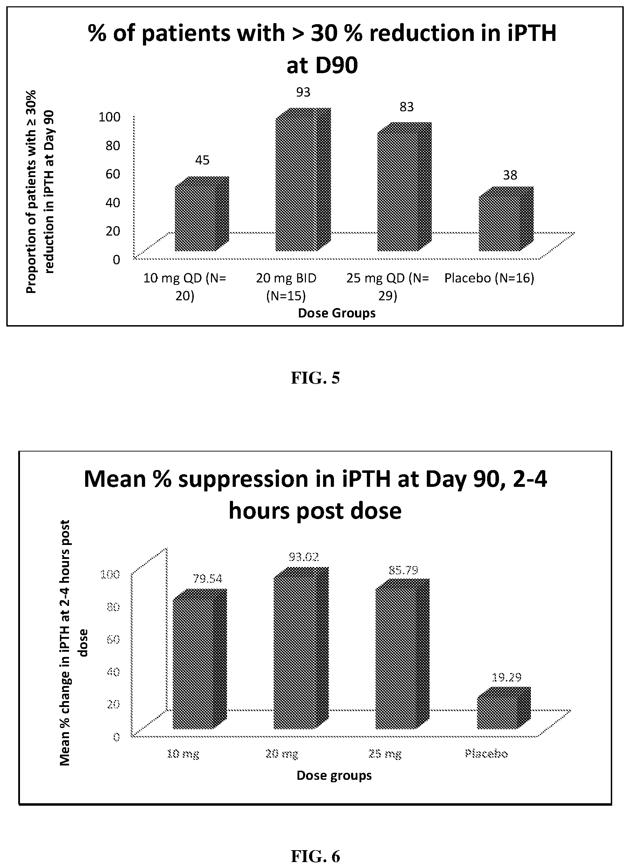

As shown in FIG. 1, in the Main Study for patients on dialysis, the mean percentage suppression in iPTH from baseline at the end of 90 days for patients who completed the entire study as per protocol was higher in 25 mg Compound 3 arm (-56.65%) than in 10 mg Compound 3 (-25.65%) and Placebo (-18.12%) arms. The difference in mean percentage change in iPTH from baseline at the end of 90 days between 25 mg Compound 3, versus Placebo arm was statistically significant (p = 0.0539). As shown in FIG. 6, in an exploratory analysis of peak iPTH suppression on Day 90, measured 2 to 4 hours post-dose, the mean percentage suppression in iPTH from baseline at the end of 90 days was -79.54% in 10 mg Compound 3 arm; -85.79% in 25 mg Compound 3 arm; and -19.29% in the Placebo arm. The difference in mean percentage change in iPTH from baseline at the end of 90 days between 10 mg Compound 3 versus Placebo arm (p = 0.0293) and between 25 mg Compound 3 versus Placebo arm (p = 0.0125) was statistically significant.

As further shown in FIG. 1, regarding the iPTH suppression for 20 mg Compound 3 BID in open label arm for dialysis patients, the mean percentage suppression in iPTH from baseline at end of 90 days was -82.42%. The difference in mean percentage change in iPTH from baseline at the end of 90 days between 20 mg Compound 3 BID versus Placebo arm was statistically significant (p = 0.0032) with ≥30% reduction in iPTH from baseline at end of 90 days seen in 93% of patients which was also significant compared to placebo (p = 0.0012). As shown in FIG. 6, in exploratory analysis, measured 2 to 4 hours post-dose was -93.02%, also showed that the difference in mean percentage change in iPTH from baseline at the end of 90 days between 20 mg Compound 3 BID versus Placebo arm was statistically significant (p = 0.0035).

As shown in FIG. 2, for not on dialysis patients, in exploratory analysis, the mean percentage suppression in iPTH from baseline at the end of 90 days (2 to 4 hours post-dose) was -48.07% in 10 mg Compound 3 arm and -5.28% in the Placebo arm. The difference in mean percentage change in iPTH from baseline at the end of 90 days between 10 mg Compound 3 versus Placebo arm was statistically significant (p = 0.0068). In open label arm administering 5 mg Compound 3, the mean percentage suppression in iPTH from baseline at the end of 90 days (2 to 4 hours post-dose) was -55.03% in 5 mg Compound 3 BID arm. The difference in mean percentage change in iPTH from baseline at the end of 90 days between 5 mg Compound 3 BID versus Placebo arm was statistically significant (p = 0.0006).

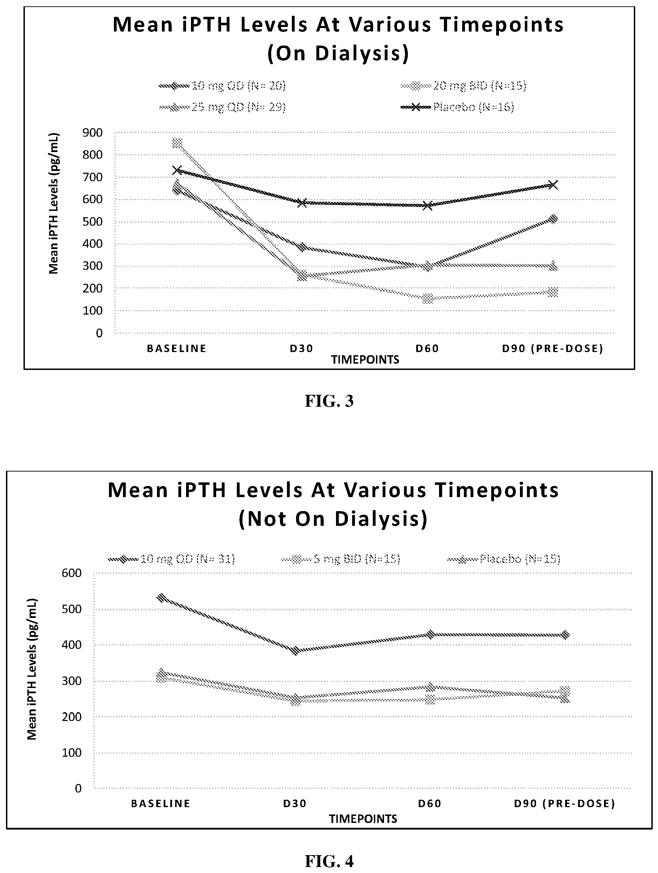

FIG. 3 shows mean iPTH levels for patients on dialysis at different time points (e.g., baseline (t=0); 30 days; 60 days; 90 days) and at different daily doses (i.e., placebo; 10 mg QD; 20 mg BID; 25 mg QD) of Compound 3.

FIG. 4 shows mean iPTH levels for patients not on dialysis at different time points (e.g., baseline (t=0); 30 days; 60 days; 90 days) and at different daily doses (i.e., placebo; 5 mg BID; 10 mg QD) of Compound 3.

FIG. 5 shows % of patients in each dose arm (i.e., placebo; 10 mg QD; 20 mg BID; 25 mg QD) who achieved equal to or more than 30% reduction at D90 from baseline iPTH concentration levels.

Thus, iPTH suppression from baseline to Day 90 by daily doses of 5 mg BID, 10 mg QD, 20 mg BID and 25 mg QD Compound 3 is significantly better compared to placebo.

### Example 4 - Surprising Advantages of the Claimed Invention

Cinacalcet, which is a currently approved oral calcimimetic (Sensipar^{®}), has wide variation in doses (30mg; 60 mg; 90 mg; 120 mg; 180 mg) and requires a dose titration strategy in which the dose is progressively increased to the therapeutic dose to treat or manage secondary hyperparathyroidism associated with CKD in a patient on dialysis. However, many patients cannot be titrated to the most therapeutically effective dose since such patients are unable to tolerate Cinacalcet during the dose titration process, or the dose titration process in general. Patients' tolerance levels to Cinacalcet are varied, which is due, in part, by inter-individual pharmacokinetic variability and different sensitivities and tolerances to gastrointestinal (GI) side effects. Also, as stated earlier, patients are required to expend time, money and resources to participate in a dose titration process, which they simply cannot or are unwilling to commit. As a result, Cinacalcet has a low patient adherence with prescribing instructions, and patients typically receive insufficient dosages of same.

2-methyl-5-((2R,4S)-2-((((R)-1-(naphthalen-1-yl)ethyl)amino)methyl) chroman-4-yl)benzoic acid hydrochloride (Compound 3) is a novel calcimimetic that does not require complex dose adjustment or titration like other approved calcimimetics for treating or managing secondary hyperparathyroidism associated with CKD in a patient on dialysis or is not on dialysis. Compound 3 can be given to patients as doses of about 5 mg, about 10 mg, about 20 mg or about 25 mg once or twice daily, without any dose titration requirement. Also, as shown in Table 9 below, patients who were administered pharmaceutical compositions comprising Compound 3 showed reduction in GI-related adverse events, such as nausea, vomiting and diarrhea, all of which were reduced to mild to moderate in severity. Table 9 also shows that the GI related adverse events are not related to dose, which serve as an advantage to switch to any desired dose without risk of experiencing GI side-effects.

**Table 9 - Adverse Events Related to GI Disturbances**

| **Adverse Event Incidence Related to GI Disturbances in Patients on Dialysis** | | | | | |
|---|---|---|---|---|---|
| Event | Compound 3 10 mg QD (N=34) (%) | Compound 3 20 mg BID (N=19) (%) | Compound 3 25 mg QD (N=35) (%) | Total in Compound 3 Group (N=88) (%) | Placebo QD (N=18) (%) |
| Nausea | 0 | 5.3 | 0 | 5.3 | 5.6 |
| Vomiting | 0 | 0 | 0 | 0 | 5.6 |
| Diarrhea | 0 | 0 | 0 | 0 | 0 |

| **Adverse Event Incidence Related to GI Disturbances in Patients Not on Dialysis** | | | | | |
|---|---|---|---|---|---|
| Event | Compound 3 5 mg BID (N=16) (%) | Compound 3 10 mg QD (N=37) (%) | Total in Compound 3 Group (N=71) (%) | Placebo QD (N=18) (%) | |
| Nausea | 0 | 0 | 0 | 5.6 | |
| Vomiting | 0 | 0 | 0 | 0 | |
| Diarrhea | 0 | 2.7 | 2.7 | 0 | |

In addition to the invention's surprising advantages discussed above, in terms of hypocalcemia, PTH lowering or suppression leads to reduced calcium levels, and predictability of this response is extremely important in preventing clinically significant hypocalcemia. Moreover, cinaclacet shows a non-PTH dependent mechanism that further contributes to a hypocalcemic effect in patients, which may create complications for such patients. In contrast, and as discussed further in the Examples section below, surprisingly, Compound 3 is found to suppress iPTH levels and with no symptomatic hypocalcemic effect in CKD patients in a Phase 2 clinical study using the dose range of 5 mg to 50 mg of Compound 3.

Additionally, the invention disclosed herein has another surprising advantage to Cinacalcet. In Cinacalcet studies involving CKD patients who are not on dialysis, hyperphosphatemia occurs data at week 32, it shows that phosphorus levels for Cinacalcet were 4.5 +/- 1.0 mg/dL (+21.4%) and for placebo it was 4.0 +/- 0.7 mg/dL (+6.8%) (Chonchol et al., "A randomised, double-blind, placebo-controlled study to assess the efficacy and safety of cinacalcet HCl in participants with CKD not receiving dialysis," Am. J. of Kidney Diseases, 53(2):197-207 (2009)). In contrast, surprisingly, CKD patients (who were either on dialysis or not on dialysis) who were administered pharmaceutical compositions comprising Compound 3 showed no increase in phosphorous levels caused by Compound 3 administration during a period of 90 days.

Compound 3 also showed a favorable safety and tolerability profile. No trends indicative of adverse treatment effect were noted in clinical laboratory evaluation, vital signs or ECG.

Adverse events related to GI disturbances in patients on dialysis and not on dialysis are summarized in Table 9. Table 9 shows that, when Compound 3 is administered to patients on dialysis and not on dialysis, such patients showed marked decrease in GI disturbances viz. nausea, vomiting and diarrhea, all of which were of mild to moderate in severity.

In terms of hypocalcemia, Phase 2 clinical study of Compound 3 showed that when suppressing iPTH levels in CKD patients, Compound 3 had no symptomatic hypocalcemic effect when administered in a dose range from about 5 mg to about 50 mg. Although hypocalcemia was seen in three patients (1 patient each in the 10 mg and 25 mg Compound 3 QD arms and 1 patient in the 20 mg Compound 3 BID arm), patient withdrawal from the study or withheld from administration of study drug (Compound 3) on symptomatic grounds were nil, in both patients on dialysis and not on dialysis.

In terms of hyperphosphatemia, Phase 2 clinical study of Compound 3 for CKD patients not on dialysis showed that the difference in mean percentage change in phosphate from baseline at the end of 90 days between 10 mg Compound 3 versus Placebo arm and 5 mg Compound 3 and Placebo arm was not significant, p = 0.9222 and p=0.8559, respectively. Accordingly, there was no increase in phosphorous levels due to administration of Compound 3 over a period of 90 days in CKD patients not on dialysis.

In CKD patients on dialysis, the difference in mean percentage change in phosphate from baseline at the end of 90 days between 10 mg Compound 3 versus Placebo, 20 mg Compound 3 versus placebo and 25 mg Compound 3 versus placebo arms was not significant, p = 0.6346, p=0.9736 and p = 0.9979, respectively. Again, there was no increase in phosphorous levels due to administration of Compound 3 over a period of 90 days in CKD patients on dialysis.

Thus, potent and clinically desirable suppression of iPTH can be achieved without inducing hyperphosphatemia in CKD patients not on dialysis and on dialysis after administering Compound 3 in a dose range from about 5 mg to about 50 mg over a period of time.

Overall, the results show that Compound 3 reduces iPTH in patients with SHPT associated with CKD on dialysis and not on dialysis. The peak effect of Compound 3 on mean percentage suppression in iPTH becomes evident within 4 hours after acute and chronic dosing as seen in PK-PD study and Main Study. The efficacy of all the doses of Compound 3 in terms of mean percentage suppression in iPTH from baseline at peak (2 to 4 hours post-dose) on Day 90 was clinically and statistically significant compared with Placebo in patients on dialysis and not on dialysis. A dose response was observed in terms of mean percentage suppression in iPTH from baseline at the end of Day 90 between 10 mg QD, 25 mg QD and 20 mg BID Compound 3 arms with latter 2 doses showing higher suppression over Placebo in patients on dialysis. No significant difference in mean percentage suppression in iPTH with Compound 3 10 mg QD and 5 mg BID dose versus Placebo was noted at the end of Day 90 in patients not on dialysis. However, there was a significant suppression in iPTH at peak (2 to 4 hours post-dose) on Day 90 in patients not on dialysis suggesting a trend for efficacy if higher doses or more frequent dosing regimens are utilized.

Compound 3 also demonstrates efficacy in terms of significant reduction in iPTH from baseline i.e., at least 30% in higher proportion of patients. Here, the proportion of patients showing ≥30% reduction in iPTH from baseline at the end of 90 days was significantly higher in 25 mg Compound 3 QD and 20 mg Compound 3 BID arms compared with Placebo in patients on dialysis. The responder rates of around 80% and 90% seen within 90 days of treatment with Compound 3 25 mg QD and 20 mg BID, respectively, are higher than the responder rates reported in longer duration trials on fully titrated doses of Cinacalcet (Sensipar^{®} [Cinacalcet] Prescribing Information) and Etelcalcetide (Parsabiv^{®} [etelcalcetide] Prescribing Information).

Such higher efficacy as described above was not associated with clinically adverse consequences on calcium and phosphate levels. In our study, only 6 patients (5.7%) (including patients administered higher [25 mg QD or 20 mg BID] and lower dose [10 mg QD] Compound 3) had TEAEs of hypocalcaemia. Of these, 3 patients had hypocalcaemia considered by the investigator to be related to the study drug. None of these hypocalcaemia events were symptomatic. Also, there were no adverse events of hyperphosphatemia in the study.

For patients not on dialysis, the difference in proportion of patients who achieved ≥30% reduction in iPTH from baseline at the end of 90 days administered Compound 3 in QD or BID dosing regimen versus Placebo was not significant. The total dose administered in the QD and BID dosing regimens was 10 mg per day, which showed no significant effect compared with Placebo irrespective of the dialysis status. In a double-blind, randomized, placebo-controlled study of Cinacalcet in patients with CKD not on dialysis, frequent serum calcium levels of <8.4 mg/dL and increases in serum phosphorous levels were observed (*Id*.). Due to such unfavorable benefit: risk ratio, Cinacalcet was not approved for use in patients not on dialysis. However, none of the patients not on dialysis treated with Compound 3 experienced adverse events of hypocalcaemia or hyperphosphatemia, making it the first oral calcimimetic to demonstrate favorable safety profile in this population.

The adverse events were balanced between the treatment arms for patients on dialysis and not on dialysis. Compared to historic studies with Cinacalcet, the incidence of hypocalcaemia and gastrointestinal side-effects is very low with Compound 3. None of the SAEs reported during the study were related to the study drug. No evidence of cardiovascular risk was observed after treatment with Compound 3. No difference in the incidence of adverse events was observed in patients administered low or high dose of Compound 3, indicating wider therapeutic window available for dosing.

Thus, Compound 3 is a novel calcimimetic agent with an excellent potency, efficacy and safety profile devoid of cytochrome P450 2D6 or human Ether-à-go-go-related gene (hERG) liabilities. The molecule has demonstrated an excellent PK profile in non-clinical studies, leading to robust efficacy translation *in-vivo.* No hypocalcaemia was observed at the non-clinical efficacious doses. Compound 3 was found to be safe and well tolerated in healthy subjects in the first-in-human Phase 1 single- and multiple-dose study. An excellent PK-PD and concentration-response correlation was seen with Compound 3 in Phase 1 study over the entire dose range studied. The results of this Phase 2 study demonstrated favorable benefit: risk ratio of Compound 3 in SHPT patients irrespective of dialysis status. The higher suppression of iPTH without symptomatic hypocalcaemia, hyperphosphatemia and absence of intolerable gastrointestinal adverse reactions seen with Compound 3 is in line with clinically desirable profile of an ideal calcimimetic agent.

Unless otherwise indicated, all numbers expressing quantities of ingredients, properties such as molecular weight, reaction conditions, and so forth used in the specification are to be understood as being modified in all instances by the term "about." Accordingly, unless indicated to the contrary, the numerical parameters set forth in the specification are approximations that may vary depending upon the desired properties sought to be obtained by the invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups for claims ultimately claiming priority hereto as permitted by applicable law.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims ultimately claiming priority hereto as permitted by applicable law. Moreover, any combination of the above-described elements in all possible variations thereof is encompassed by the invention unless otherwise indicated herein or otherwise clearly contradicted by context.

In all of the foregoing embodiments disclosed herein, it is to be understood that all embodiments may be further limited by using "consisting of" or "consisting essentially of" language, rather than "comprising". When used, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

## Claims

1. A solid pharmaceutical composition comprising a pharmaceutically effective amount of one or more compounds, wherein the one or more compounds is selected from the group consisting of compounds having the structure of Formula (I), Formula (II), Formula (III), Formula (IV), Formula (V), or Formula (VI): 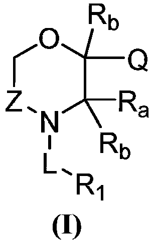 wherein
Q is 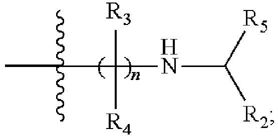
Rₐ is hydrogen;
R_{b} is hydrogen;
L is a bond or -(CR_{c}R_{d})ₘ;
R_{c} and R_{d} are independently selected from hydrogen or substituted or unsubstituted alkyl;
R₁ is 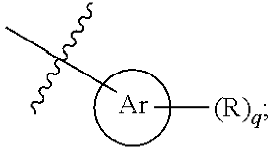
ring Ar is phenyl or naphthyl;
R, which may be same or different at each occurrence, is independently selected from halogen, nitro, cyano, substituted or unsubstituted alkyl, substituted or unsubstituted alkenyl, substituted or unsubstituted alkynyl, substituted or unsubstituted haloalkyl, - OR₆, -C(O)R₆, -(CRₑR_{f})₀₋₃-C(O)OR₆, -(CRₑR_{f})₁₋₂cycloalkylene-C(O)OR₆, - cycloalkylene (CRₑR_{f})₀₋₂-C(O)OR₆, -O(CRₑR_{f})₀₋₃-C(O)OR₆, -O-cycloalkylene-C(O)OR₆, -C(O)NR₇-(CRₑR_{f})₁₋₂-C(O)OR₆, -C(O)NR₇R₈, -S(O)₀₋₂R₆, and - S(O)₂NR₇R₈;
Rₑ and R_{f} are independently hydrogen or substituted or unsubstituted alkyl;
R₂ is substituted or unsubstituted aryl;
R₃ and R₄ are independently selected from hydrogen, halogen, and substituted or unsubstituted alkyl;
R₅ is substituted or unsubstituted alkyl or haloalkyl;
R₆, which may be same or different at each occurrence, is independently selected from hydrogen, substituted or unsubstituted alkyl and substituted or unsubstituted haloalkyl;
R₇ and R₈, which may be same or different at each occurrence, are independently selected from hydrogen, substituted or unsubstituted alkyl, substituted or unsubstituted cycloalkyl, and substituted or unsubstituted aryl;
Z is -CR_{g}Rₕ-;
R_{g} and Rₕ are hydrogen;
"m" is an integer ranging from 1 to 3, both inclusive; "n" is an integer ranging from 1 to 3, both inclusive; and "q" is an integer ranging from 0 to 4, both inclusive; 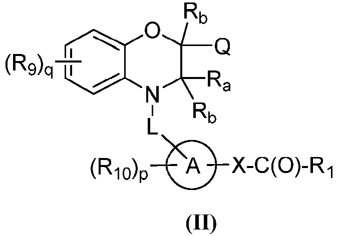
wherein
Q is 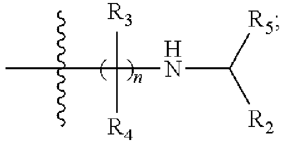
Rₐ is hydrogen, halogen or alkyl;
R_{b} is selected from hydrogen, alkyl, and haloalkyl;
or Rₐ and R_{b} together attached on the same carbon form C(O);
L is a bond or -CR_{c}R_{d}-;
ring A is phenyl;
R_{c} and R_{d} are independently selected from hydrogen, halogen, and alkyl;
X is selected from a bond, -(CRₑR_{f})ₘ, -O-, -O(CRₑR_{f})ₘ-, -(CRₑR_{f})ₘO-, - C(O)(CRₑR_{f})ₘ , -C(O)NR₇-, -C(O)NR₇(CRₑR_{f})ₘ-, -cycloalkylene-, and -O-cycloalkylene-;
Rₑ and R_{f}, which may be same or different at each occurrence, are independently selected from hydrogen, halogen, alkyl, haloalkyl and cycloalkyl; or Rₑ and R_{f}, together with the carbon atom to which they are attached, may form a 3 to 7 membered saturated carbocyclic ring;
R₁ is -OR₆ or -NR₇R₈;
R₂ is substituted or unsubstituted aryl;
R₃ and R₄ are independently selected from hydrogen, halogen, alkyl, haloalkyl, and cycloalkyl;
R₅ is alkyl or haloalkyl;
R₆ is hydrogen or alkyl;
R₇ and R₈ are independently selected from hydrogen, alkyl, cycloalkyl cycloalkylalkyl and aryl;
R₉, which may be same or different at each occurrence, is independently selected from halogen, cyano, alkyl, haloalkyl, and cycloalkyl;
R₁₀, which may be same or different at each occurrence, is independently selected from halogen, cyano, alkyl, alkenyl, alkynyl, haloalkyl, hydroxyalkyl, -OR₆, -C(O)R₆, - NR₇C(O)R₆, -S(O)₀₋₂R₆, -S(O)₂NR₇R₈, and -NR₇S(O)₂R₆;
"m" is an integer ranging from 1 to 3, both inclusive; "n" is an integer ranging from 1 to 3, both inclusive; "p" is an integer ranging from 0 to 2, both inclusive; and "q" is an integer ranging from 0 to 1, both inclusive; 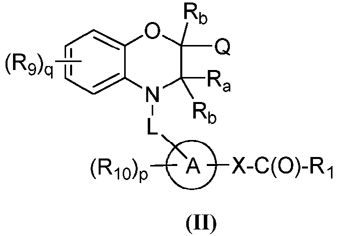
wherein Q is 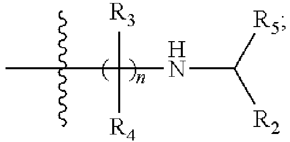
Rₐ is hydrogen; R_{b} is hydrogen or alkyl;
L is a bond, or -CR_{c}R_{d};
ring A is phenyl;
R_{c} and R_{d} are independently selected from hydrogen, halogen, and alkyl;
X is selected from a bond, -(CRₑR_{f})ₘ, -O-, -O(CRₑR_{f})ₘ-, -(CRₑR_{f})ₘO-, - C(O)(CRₑR_{f})ₘ-, -C(O)NR₇-, and -C(O)NR₇(CRₑR_{f})ₘ-;
Rₑ and R_{f}, which may be same or different at each occurrence, are independently selected from hydrogen, halogen, alkyl, haloalkyl and cycloalkyl; or Rₑ and R_{f}, together with the carbon atom to which they are attached, form a 3 to 7 membered saturated carbocyclic ring;
R₁ is -OR₆ or -NR₇R₈;
R₂ is phenyl or naphthyl, wherein the phenyl is substituted with halogen, alkyl, haloalkyl, alkoxy or haloalkoxy;
R₃ and R₄ are hydrogen;
R₅ is alkyl;
R₆ is hydrogen or alkyl;
R₇ and R₈ are hydrogen or alkyl;
R₉ is independently selected from halogen, cyano, alkyl, haloalkyl, and cycloalkyl;
R₁₀, which may be same or different at each occurrence, is independently selected from halogen, cyano, alkyl, haloalkyl, hydroxyalkyl, -OR₆, -C(O)R₆, -NR₇R₈, - NR₇C(O)R₆, and -(O)₂NR₇R₈;
"m" is an integer ranging from 1 to 3, both inclusive; "n" is an integer ranging from 1 to 3, both inclusive; "p" is an integer ranging from 0 to 2, both inclusive; "q" is an integer ranging from 0 to 1, both inclusive; 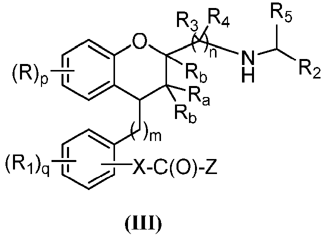
wherein
Rₐ is selected from hydrogen, halogen, substituted or unsubstituted alkyl, cyano, substituted or unsubstituted cycloalkyl and substituted or unsubstituted haloalkyl;
R_{b}, which may be same or different at each occurrence, is independently selected from hydrogen, halogen, substituted or unsubstituted alkyl, substituted or unsubstituted cycloalkyl and substituted or unsubstituted haloalkyl;
R_{c} which may be same or different at each occurrence, is independently selected from halogen, hydroxy, substituted or unsubstituted alkyl, substituted or unsubstituted haloalkyl, substituted or unsubstituted alkenyl, substituted or unsubstituted alkynyl, substituted or unsubstituted cycloalkyl, OR₆, nitro, cyano, -C(O)OR₆, -(CH₂)ᵣ-C(O)OR₆, -O-C(O)OR₆, -O(CH₂)ᵣ-C(O)OR₆, -NR₇R₈, -(CH₂)ᵣNR₇R₈-, - C(O)R₉, -C(O)NR₇R₈, -(CH₂)ᵣ-C(O)NR₇R₈, -NR₇C(O)R₉, -S(O)₀₋₂R₆, - S(O)₂NR₇R₈, and -NR₇S(O)₂R₉;
X is selected from a bond, -(CR_{c}R_{d})ᵣ₋, -O-, -NR₇-, -NR₇(CR_{c}R_{d})ᵣ-, - O(CR_{c}R_{d})ᵣ, -C(O)NR₇-, -C(O)NR₇(CR_{c}R_{d})ᵣ, -(CRcR_{d})ᵣNR₇(CR_{c}R_{d})ᵣ, - (CR_{c}R_{d})ᵣcycloalkylene-, cycloalkylene, -cycloalkylene(CR_{c}R_{d})ᵣ₋ and -O-cycloalkylene where cycloalkylene may be substituted or unsubstituted;
R_{c} and R_{d}, which may be same or different at each occurrence, are independently selected from hydrogen, halogen, hydroxy, cyano, nitro, substituted or unsubstituted alkyl, substituted or unsubstituted haloalkyl and substituted or unsubstituted cycloalkyl; or R_{c} and R_{d}, together with the carbon atom to which they are attached, may form a substituted or unsubstituted 3 to 7 membered saturated carbocyclic ring;
Z is -OR₆ or -NR₁₀R₁₁;
R₁, which may be same or different at each occurrence, is independently selected from halogen, nitro, cyano, substituted or unsubstituted alkyl, substituted or unsubstituted alkenyl, substituted or unsubstituted alkynyl, substituted or unsubstituted haloalkyl, substituted or unsubstituted cycloalkyl, -OR₆, -C(O)R₉, -NR₇R₈, - (CH₂)ᵣNR₇R₈-, -(CH₂)ᵣ-C(O)OR₆, -O-C(O)OR₆, -O(CH₂)ᵣ-C(O)OR₆, - C(O)NR₇R₈, -(CH₂)ᵣ-C(O)NR₇R₈, -NR₇C(O)R₉, -S(O)₀₋₂R₇, -S(O)₂NR₇R₈ and -NR₇S(O)₂R₉;
R₂ is selected from substituted or unsubstituted aryl, substituted or unsubstituted heteroaryl and substituted or unsubstituted heterocyclyl;
R₃ and R₄ may be same or different and are independently selected from hydrogen, halogen, substituted or unsubstituted alkyl, substituted or unsubstituted haloalkyl, substituted or unsubstituted alkenyl, substituted or unsubstituted alkynyl, substituted or unsubstituted alkoxy, substituted or unsubstituted haloalkoxy and substituted or unsubstituted cycloalkyl;
R₅ is substituted or unsubstituted alkyl;
R₆, which may be same or different at each occurrence, is independently selected from hydrogen, substituted or unsubstituted alkyl, substituted or unsubstituted haloalkyl, substituted or unsubstituted alkenyl, substituted or unsubstituted alkynyl, and substituted or unsubstituted aryl;
R₇ and R₈, which may be same or different at each occurrence, are independently selected from hydrogen, substituted or unsubstituted alkyl, substituted or unsubstituted alkenyl, substituted or unsubstituted alkynyl, substituted or unsubstituted cycloalkyl, substituted or unsubstituted cycloalkylalkyl, substituted or unsubstituted aryl, substituted or unsubstituted arylalkyl, substituted or unsubstituted heteroaryl, substituted or unsubstituted heteroarylalkyl, substituted or unsubstituted heterocyclyl, and substituted or unsubstituted heterocyclylalkyl; or R₇ and R₈, together with the nitrogen atom to which they are attached, may form a substituted or unsubstituted, saturated or unsaturated 3 to 12 membered cyclic ring, wherein the unsaturated cyclic ring may have one or two double bonds;
at each occurrence, R₉ is substituted or unsubstituted alkyl or substituted or unsubstituted aryl;
R₁₀ and R₁₁ may be same or different and are independently selected from hydrogen, substituted or unsubstituted alkyl, substituted or unsubstituted alkenyl, substituted or unsubstituted alkynyl, -(CR_{c}R_{d})ᵣ-C(O)OR₆, substituted or unsubstituted cycloalkyl, substituted or unsubstituted cycloalkylalkyl, substituted or unsubstituted aryl, substituted or unsubstituted arylalkyl, substituted or unsubstituted heteroaryl, substituted or unsubstituted heteroarylalkyl, substituted or unsubstituted heterocyclyl, and substituted or unsubstituted heterocyclylalkyl; or R₁₀ and R₁₁, together with the nitrogen atom to which they are attached, may form a substituted or unsubstituted, saturated or unsaturated 3 to 12 membered cyclic ring, wherein the unsaturated cyclic ring may have one or two double bonds;
"n" is an integer ranging from 1 to 3, both inclusive; "m" is an integer ranging from 0 to 3, both inclusive; "p" is an integer ranging from 0 to 4, both inclusive; "q" is an integer ranging from 0 to 3, both inclusive; and "r" is an integer ranging from 1 to 3, both inclusive; 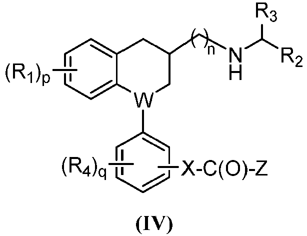
wherein
W is CH or N;
R₁, which may be same or different at each occurrence, is independently selected from halogen, nitro, cyano, substituted or unsubstituted alkyl, substituted or unsubstituted haloalkyl, substituted or unsubstituted cycloalkyl, -C(O)OR₅, -(CRₐR_{b})ᵣ-C(O)OR₅, -O-C(O)OR₅, -O(CRₐR_{b})ᵣ₋C(O)OR₅, -NR₆R₇, -C(O)R₈, -C(O)NR₆R₇, - NR₆C(O)R₈, -S(O)₀₋₂R₅, -S(O)₂NR₆R₇ and -NR₆S(O)₂R₈;
R₂ is substituted or unsubstituted aryl;
R₃ is substituted or unsubstituted alkyl;
R₄, which may be same or different at each occurrence, is independently selected from halogen, cyano, substituted or unsubstituted alkyl, substituted or unsubstituted haloalkyl, -OR₅, -NR₆R₇, -C(O)R₈, -C(O)NR₆R₇, -NR₆C(O)R₈, -S(O)₀₋₂R₅, -S(O)₂NR₆R₇ and -NR₅S(O)₂R₈;
X is selected from a bond, -(CRₐR_{b})ᵣ-, -O-, -NR₇-, -O(CRₐR_{b})ᵣ-, - C(O)NR₇-, -C(O)NR₇(CRₐR_{b})ᵣ₋, -(CRₐR_{b})ᵣcycloalkylene-, cycloalkylene, cycloalkylene-(CRₐR_{b})ᵣ- and -O-cycloalkylene;
Rₐ and R_{b}, which may be same or different at each occurrence, are independently selected from hydrogen, halogen, hydroxy, cyano, substituted or unsubstituted alkyl, substituted or unsubstituted haloalkyl and substituted or unsubstituted cycloalkyl; or Rₐ and R_{b}, together with the carbon atom to which they are attached, may form a substituted or unsubstituted 3 to 7 membered saturated carbocyclic ring;
Z is -OR₅ or -NR₆R₇;
R₅, which may be same or different at each occurrence, is independently selected from hydrogen, substituted or unsubstituted alkyl, substituted or unsubstituted haloalkyl and substituted or unsubstituted aryl;
R₆ and R₇, which may be same or different at each occurrence, are independently selected from hydrogen, substituted or unsubstituted alkyl, -(CRₐR_{b})ᵣ-C(O)OR₅, substituted or unsubstituted cycloalkyl, substituted or unsubstituted cycloalkylalkyl, substituted or unsubstituted aryl, substituted or unsubstituted arylalkyl, substituted or unsubstituted heteroaryl, substituted or unsubstituted heteroarylalkyl, substituted or unsubstituted heterocyclyl and substituted or unsubstituted heterocyclylalkyl; or R₆ and R₇, together with the nitrogen atom to which they are attached, may form a substituted or unsubstituted, saturated or unsaturated 3 to 10 membered cyclic ring, wherein the unsaturated cyclic ring may have one or two double bonds;
R₈ is substituted or unsubstituted alkyl or substituted or unsubstituted aryl;
"n" is an integer ranging from 0 to 3, both inclusive; "p" is an integer ranging from 0 to 3, both inclusive; "q" is an integer ranging from 0 to 3, both inclusive; and "r" is an integer ranging from 1 to 3, both inclusive; 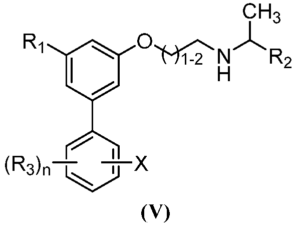
wherein
R₁ is selected from halogen, substituted or unsubstituted alkyl, substituted or unsubstituted haloalkyl, substituted or unsubstituted cycloalkyl, substituted or unsubstituted alkoxy, substituted or unsubstituted haloalkoxy, -(CRₐR_{b})₁₋₃OH, - C(O)NH-alkyl, -S(O)₂- alkyl, -S(O)₂NH-alkyl, -C(O)OH, -C(O)O-alkyl, -(CRₐR_{b})₁₋₃C(O)OH and -(CRₐR_{b})₁₋₃C(O)O- alkyl;
R₂ is substituted or unsubstituted phenyl or substituted or unsubstituted naphthyl, wherein the substituents may be one or more and are independently selected from halogen, substituted or unsubstituted alkyl, substituted or unsubstituted haloalkyl, - OR₄, -NR₅R₆ and substituted or unsubstituted cycloalkyl;
X is selected from -C(O)OH, -C(O)O-alkyl, -C(O)NR₅R₆, -(CRₐR_{b})₁₋₃C(O)OH, - C(O)O-alkyl, -O-(CRₐR_{b})1-₃C(O)OH, -O-(CRₐR_{b})₁₋₃C(O)O-alkyl, -CR_{C}=CR_{C}- - C(O)OH, halogen, substituted or unsubstituted alkyl, substituted or unsubstituted haloalkyl,- OR₄ and -S(O)₂-alkyl;
Rₐ and R_{b} are independently selected from hydrogen, halogen, hydroxy, substituted or unsubstituted alkyl, substituted or unsubstituted haloalkyl and substituted or unsubstituted cycloalkyl; or Rₐ and R_{b} , together with the carbon atom to which they are attached, may form a substituted or unsubstituted 3 to 6 membered saturated carbocyclic ring;
R_{c} is independently selected from hydrogen, halogen and substituted or unsubstituted alkyl;
R₃, which may be same or different at each occurrence, is independently selected from halogen, cyano, substituted or unsubstituted alkyl, substituted or unsubstituted haloalkyl and -OR₄;
R₄ is selected from hydrogen, substituted or unsubstituted alkyl, substituted or unsubstituted haloalkyl and substituted or unsubstituted cycloalkyl;
R₅ and R₆ are independently selected from hydrogen, substituted or unsubstituted alkyl and substituted or unsubstituted cycloalkyl; and "n" is an integer ranging from 0 to 2, both inclusive; 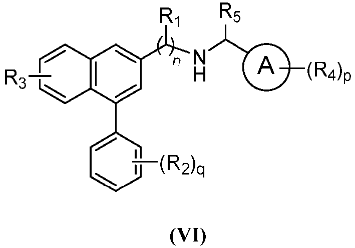
wherein
ring A is phenyl or naphthyl;
R₁ is hydrogen or substituted or unsubstituted (C₁-C₆)alkyl;
R₂, which may be same or different at each occurrence, is independently selected from halogen, cyano, substituted or unsubstituted (C₁-C₆)alkyl, substituted or unsubstituted (C₁-C₆)haloalkyl, substituted or unsubstituted (C₁-C₆)hydroxyalkyl, -X-C(O)-Z, -OR₉, -NR₇R₈, -NR₇C(O)R₆, -S(O)₀₋₂R₆, -S(O)₂NR₇R₈, -NR₇S(O)₂R₆, substituted or unsubstituted (C₃-C₁₂) cycloalkyl, substituted or unsubstituted phenyl, substituted or unsubstituted 5- to 6-membered heteroaryl, substituted or unsubstituted 5- to 6-membered heterocyclyl and ring D;
ring D is 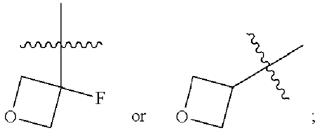
X is selected from a bond, -(CRₐR_{b})ₘ-, -NR₂-, -O(CRₐR_{b})ₘ-, -(CRₐR_{b})ₘO-, -C(O)NR₁₂-, -(CRₐR_{b})ₘO-(CRₐR_{b})ₘ- and -C(O)NR₁₂(CRₐR_{b})ₘ-;
Rₐ and R_{b} which may be same or different at each occurrence, are independently selected from hydrogen, halogen, hydroxy, substituted or unsubstituted (C₁-C₆)alkyl, substituted or unsubstituted (C₁-C₆)haloalkyl and substituted or unsubstituted (C₃-C₆)cycloalkyl; or Rₐ and R_{b}, together with the carbon atom to which they are attached, form a substituted or unsubstituted 3 to 6 membered saturated carbocyclic ring;
Z is -OR₁₀ or -NR₇R₈;
R₃ is selected from hydrogen, halogen, substituted or unsubstituted (C₁-C₆)alkyl, substituted or unsubstituted (C₁-C₆)haloalkyl, -OR₉, and substituted or unsubstituted (C₃-C₁₂)cycloalkyl;
R₄, which may be same or different at each occurrence, is independently selected from halogen, substituted or unsubstituted (C₁-C₆)alkyl, substituted or unsubstituted (C₁-C₆)haloalkyl, substituted or unsubstituted (C₁-C₆)alkoxyalkyl, -SF₅ and -OR₉;
R₅ is substituted or unsubstituted (C₁-C₆)alkyl;
R₆ is selected from substituted or unsubstituted (C₁-C₆)alkyl, substituted or unsubstituted (C₃-C₁₂)cycloalkyl and substituted or unsubstituted (C₆-C₁₄)aryl;
R₇ and R₈, which may be same or different at each occurrence, are independently selected from hydrogen, substituted or unsubstituted (C₁-C₆)alkyl, -(CRₐR_{b})₁₋₂R₁₁, - (CR_{c}R_{d})ₘ-OH and substituted or unsubstituted (C₃-C₁₂)cycloalkyl;
R_{c} and R_{d} which may be same or different at each occurrence, are independently hydrogen or substituted or unsubstituted (C₁-C₆)alkyl;
R₉ is independently selected from hydrogen, substituted or unsubstituted (C₁-C₆)alkyl, substituted or unsubstituted (C₁-C₆)haloalkyl, substituted or unsubstituted (C₁-C₆)alkoxyalkyl and substituted or unsubstituted (C₃-C₁₂)cycloalkyl;
R₁₀ is selected from hydrogen, substituted or unsubstituted (C₁-C₆)alkyl and - (CRₐR_{b})₁₋₂phenyl;
R₁₁ is substituted or unsubstituted phenyl, wherein the substituents are selected from halogen, (C₁-C₆)alkyl and -OR₉;
R₁₂ is hydrogen or substituted or unsubstituted (C₁-C₆)alkyl;
"m" is an integer ranging from 1 to 3, both inclusive; "n" is an integer ranging from 1 to 3, both inclusive; "p" is an integer ranging from 0 to 3, both inclusive; and "q" is an integer ranging from 1 to 3, both inclusive;
or pharmaceutically acceptable salts thereof, or a combination thereof, and at least two pharmaceutically acceptable excipients, wherein the at least two pharmaceutically acceptable excipients is microcrystalline cellulose, crospovidone, starch, magnesium stearate, sodium lauryl sulfate, colloidal silicon dioxide, or a combination thereof; and
wherein the solid pharmaceutical composition is suitable for oral administration and is in the form of a tablet or capsule.

2. The solid pharmaceutical composition of claim 1, wherein the one or more compounds is:
3-((S)-2-((((R)-1-(naphthalen-1-yl)ethyl)amino)methyl) morpholino)-5-(trifluoromethyl)benzoic acid, an internal salt or hydrochloric acid salt thereof ("Compound 1");
2-methyl-5-((S)-2-(2-(((R)-1-(naphthalen-1-yl)ethyl)amino) ethyl)-2H-benzo[b][1,4]oxazin-4(3H)-yl)benzoic acid, an internal salt or hydrochloric acid salt thereof ("Compound 2");
2-methyl-5-((2R,4S)-2-((((R)-1-(naphthalen-1-yl)ethyl)amino) methyl)chroman-4-yl)benzoic acid hydrochloride ("Compound 3");
3-((1R,3 S)-3-((((R)-1-(4-fluoro-3-methoxyphenyl)ethyl)amino) methyl)-1,2,3,4-tetrahydronaphthalen-1-yl)-2,6-dimethylbenzoic acid, an internal salt or hydrochloric acid salt thereof ("Compound 4");
(R)-3-(4-fluoro-3'-(2-((1-(3-methoxy phenyl)ethyl)amino) ethoxy)-5'-(trifluoromethyl)-[1,1'-biphenyl]-3-yl)propanoic acid, an internal salt or hydrochloric acid salt thereof ("Compound 5");
(R)-N-((R)-1-(3-methoxyphenyl)ethyl)-1-(4-(4-(trifluoromethyl) phenyl)naphthalen-2-yl)propan-1-amine hydrochloride ("Compound 6"); or
a combination thereof.

3. The solid pharmaceutical composition of claim 1 or 2, wherein the one or more compounds is Compound 3.

4. The solid pharmaceutical composition of any one of claims 1-3, wherein the composition is in the form of a tablet, wherein the tablet is coated with a film.

5. The solid pharmaceutical composition of claim 4, wherein the film is a resistant starch, a high-amylose maize starch, Poly(methacrylic acid-co-methyl methacrylate) 1:2, Poly(ethyl acrylate-co-methyl methacrylate-co-trimethylammonioethyl methacrylate chloride) 1:2:0.1, Poly(ethyl acrylate-co-methyl methacrylate-co-trimethylammonioethyl methacrylate chloride) 1:2:0.2, starch acetate, ethyl cellulose, croscarmellose sodium, sodium starch glycolate, cellulose ether, vinyl polymer, glycol, acrylic polymer, maltodextrin, polydextrose, cellulose acetate phthalate, cellulose acetate trimellitate, hydroxypropyl methylcellulose acetate succinate-hypromellose acetate succinate, hydroxypropyl methylcellulose phthalate, polyvinyl acetate phthalate, poly(methacrylic acid-co-methyl methacrylate), shellac, or a combination thereof.

6. A solid pharmaceutical composition comprising one or more compounds and one or more ingredients, wherein the one or more compounds is Compound 1, Compound 2, Compound 3, Compound 4, Compound 5, Compound 6, a pharmaceutical acceptable salt thereof, or a combination thereof, and wherein the one or more ingredients is:
(a) microcrystalline cellulose in the amount of 5% to 50% by weight of the total weight of the solid pharmaceutical composition;
(b) crospovidone in the amount of 5% to 20% by weight of the total weight of the solid pharmaceutical composition, or povidone in the amount of 3% to 20% by weight of the total weight of the solid pharmaceutical composition, or a combination thereof;
(c) pregelatinized starch in the amount of 20% to 50% by weight of the total weight of the solid pharmaceutical composition;
(d) magnesium stearate in the amount of 0.5% to 5% by weight of the total weight of the solid pharmaceutical composition, or talc in the amount of 0.5% to 2% by weight of the total weight of the solid pharmaceutical composition, or a combination thereof;
(e) sodium lauryl sulfate (SLS) in the amount of 0.5% to 3% by weight of the total weight of the solid pharmaceutical composition;
(f) colloidal silicon dioxide in amount of 0.5% to 3% by weight of the total weight of the solid pharmaceutical composition; or
(g) a combination thereof.

7. A solid pharmaceutical composition comprising 2-methyl-5-((2R,4S)-2-((((R)-1-(naphthalen-1-yl)ethyl)amino) methyl)chroman-4-yl)benzoic acid hydrochloride and one or more ingredients, wherein the one or more ingredients is:
(a) microcrystalline cellulose in the amount of 5% to 50% by weight of the total weight of the solid pharmaceutical composition;
(b) crospovidone in the amount of 5% to 20% by weight of the total weight of the solid pharmaceutical composition, or povidone in the amount of 3% to 20% by weight of the total weight of the solid pharmaceutical composition, or a combination thereof;
(c) pregelatinized starch in the amount of 20% to 50% by weight of the total weight of the solid pharmaceutical composition;
(d) magnesium stearate in the amount of 0.5% to 5% by weight of the total weight of the solid pharmaceutical composition, or talc in the amount of 0.5% to 2% by weight of the total weight of the solid pharmaceutical composition, or a combination thereof;
(e) sodium lauryl sulfate (SLS) in the amount of 0.5% to 3% by weight of the total weight of the solid pharmaceutical composition;
(f) colloidal silicon dioxide in amount of 0.5% to 3% by weight of the total weight of the solid pharmaceutical composition; or
(g) a combination thereof.

8. The solid pharmaceutical composition of any one of claims 1 to 6, wherein the one or more compounds is in the amount of 0.1 mg to 200 mg..

9. The solid pharmaceutical composition of any one of claims 1 to 6, wherein the one or more compounds is in the amount of 1 mg to 50 mg, or 5 mg to 25 mg.

10. The solid pharmaceutical composition of claim 7, wherein the 2-methyl-5-((2R,4S)-2-((((R)-1-(naphthalen-1-yl)ethyl)amino) methyl)chroman-4-yl)benzoic acid hydrochloride is in the amount of 0.1 mg to 200 mg, 1 mg to 50 mg, or 5 mg to 25 mg..

11. The solid pharmaceutical composition of any one of claims 1-3, wherein the pharmaceutical composition is a capsule, wherein the capsule comprises 35% w/w to 39% w/w of the one or more compounds.

12. A solid pharmaceutical composition as described in any one of claims 1-11 for use in treating secondary hyperparathyroidism associated with chronic kidney disease (CKD) in a subject in need thereof.

13. The solid pharmaceutical composition for use as claimed in claim 12, wherein the compound in the solid pharmaceutical composition is 2-methyl-5-((2R,4S)-2-((((R)-1-(naphthalen-1-yl)ethyl)amino) methyl)chroman-4-yl)benzoic acid hydrochloride.

14. The solid pharmaceutical composition for use as claimed in claim 12 or claim 13, wherein said use comprises the administration of the one or more compounds in a total daily dose of 5 mg to 50 mg.

15. The solid pharmaceutical composition for use as claimed in any one of claim 14, wherein the administration is 25 mg once or twice daily, or 20 mg once or twice daily.

16. The solid pharmaceutical composition for use as claimed in any one of claims 12 to 15, wherein said use comprises oral administration of said solid pharmaceutical composition.

17. The solid pharmaceutical composition for use as claimed in any one of claims 12 to 16, wherein said use does not include dose-titration.

18. The solid pharmaceutical composition for use as claimed in any one of claims 12 to 17, wherein the subject is not on dialysis.

19. The solid pharmaceutical composition for use as claimed in any one of claims 12 to 17, wherein the subject is on dialysis.

20. The solid pharmaceutical composition for use as claimed in any one of claims 12 to 19, wherein the secondary hyperparathyroidism is treated or managed without inducing hyperphosphatemia or hypocalcemia in the subject.

21. The solid pharmaceutical composition for use as claimed in any one of claims 12 to 20, wherein the subject has been previously diagnosed or has Stage 3b CKD, Stage 4 CKD or Stage 5 CKD.

22. A solid pharmaceutical composition as described in any one of claims 1-11 for use in lowering or suppressing one or more intact parathyroid hormone (iPTH) levels in a subject suffering from secondary hyperparathyroidism associated with CKD.

23. The solid pharmaceutical composition for use as claimed in claim 22, wherein the compound in the solid pharmaceutical composition is 2-methyl-5-((2R,4S)-2-((((R)-1-(naphthalen-1-yl)ethyl)amino) methyl)chroman-4-yl)benzoic acid hydrochloride.

24. The solid pharmaceutical composition for use as claimed in claim 22 or 23, wherein the subject is not on dialysis.

25. The solid pharmaceutical composition for use as claimed in claim 22 or 23, wherein the subject is on dialysis.

26. The solid pharmaceutical composition for use as claimed in any one of claims 22 to 25, wherein said use comprises the administration of the one or more compounds in a total daily dose amount of 5 mg to 50 mg.

27. The solid pharmaceutical composition for use as claimed in claim 26, wherein the total daily dose amount is 10 mg to 40 mg.

28. The solid pharmaceutical composition for use as claimed in claim 26, wherein the total daily dose amount is administered 25 mg once or twice daily, or 20 mg once or twice daily.

29. The solid pharmaceutical composition for use as claimed in any one of claims 22 to 27, wherein said use comprises oral administration of said solid pharmaceutical composition.

30. The solid pharmaceutical composition for use as claimed in any one of claims 22 to 29, wherein the administration does not require dose adjustment to the subject.

31. The solid pharmaceutical composition for use as claimed in any one of claims 22 to 30, wherein the subject's iPTH level is reduced by at least 30% compared to the baseline iPTH concentration level before treatment.

32. The solid pharmaceutical composition for use as claimed in any one of claims 22 to 30, wherein the subject's iPTH level is reduced by at least 15% compared to the baseline iPTH concentration level before treatment.

33. A solid pharmaceutical composition of 2-methyl-5-((2R,4S)-2-((((R)-1-(naphthalen-1-yl)ethyl)amino) methyl)chroman-4-yl)benzoic acid, hydrochloride salt for use in the treatment or management of secondary hyperparathyroidism associated with CKD.

34. A solid pharmaceutical composition of 2-methyl-5-((2R,4S)-2-((((R)-1-(naphthalen-1-yl)ethyl)amino) methyl)chroman-4-yl)benzoic acid, hydrochloride salt for use in the treatment or management of secondary hyperparathyroidism associated with CKD by lowering or suppressing one for more iPTH levels in a subject.

35. The solid pharmaceutical composition of 2-methyl-5-((2R,4S)-2-((((R)-1-(naphthalen-1-yl)ethyl)amino) methyl)chroman-4-yl)benzoic acid, hydrochloride salt for use as claimed in claim 34, wherein the subject's iPTH level is reduced by at least 30% compared to the baseline iPTH concentration level before treatment.

36. The solid pharmaceutical composition of 2-methyl-5-((2R,4S)-2-((((R)-1-(naphthalen-1-yl)ethyl)amino) methyl)chroman-4-yl)benzoic acid, hydrochloride salt for use as claimed in claim 34, wherein the subject's iPTH level is reduced by at least 15% compared to the baseline iPTH concentration level before treatment.

## Patentansprüche

1. Feste pharmazeutische Zusammensetzung, umfassend eine pharmazeutisch wirksame Menge von einer oder mehreren Verbindungen, wobei die eine oder mehreren Verbindungen ausgewählt sind aus der Gruppe bestehend aus Verbindungen mit der Struktur der Formel (I), Formel (II), Formel (III), Formel (IV), Formel (V) oder Formel (VI): 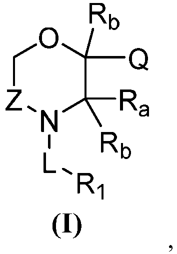 wobei
Q wie folgt ist, 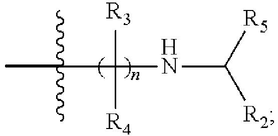
Rₐ Wasserstoff ist;
R_{b} Wasserstoff ist;
L eine Bindung oder -(CR_{c}R_{d})ₘ ist;
R_{c} und R_{d} unabhängig ausgewählt sind aus Wasserstoff oder substituiertem oder unsubstituiertem Alkyl;
R₁ wie folgt ist, 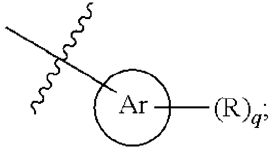
Ring Ar Phenyl oder Naphthyl ist;
R, das bei jedem Auftreten gleich oder verschieden sein kann, unabhängig ausgewählt ist aus Halogen, Nitro, Cyano, substituiertem oder unsubstituiertem Alkyl, substituiertem oder unsubstituiertem Alkenyl, substituiertem oder unsubstituiertem Alkinyl, substituiertem oder unsubstituiertem Haloalkyl, -OR₆, -C(O)R₆, -(CRₑR_{f})₀₋₃-C(O)OR₆, -(CRₑR_{f})₁₋₂Cycloalkylen-C(O)OR₆, -Cycloalkylen(CRₑR_{f})₀₋₂-C(O)OR₆, -O(CRₑR_{f})₀₋₃-C(O)OR₆, -O-Cycloalkylen-C(O)OR₆, -C(O)NR₇-(CRₑR_{f})₁₋₂-C(O)OR₆, -C(O)NR₇R₈, -S(O)₀₋₂R₆ und - S(O)₂NR₇R₈;
Rₑ und R_{f} unabhängig Wasserstoff oder substituiertes oder unsubstituiertes Alkyl sind;
R₂ substituiertes oder unsubstituiertes Aryl ist;
R₃ und R₄ unabhängig ausgewählt sind aus Wasserstoff, Halogen und substituiertem oder unsubstituiertem Alkyl;
R₅ substituiertes oder unsubstituiertes Alkyl oder Haloalkyl ist;
R₆, das bei jedem Auftreten gleich oder verschieden sein kann, unabhängig ausgewählt ist aus Wasserstoff, substituiertem oder unsubstituiertem Alkyl und substituiertem oder unsubstituiertem Haloalkyl;
R₇ und R₈, die bei jedem Auftreten gleich oder verschieden sein können, unabhängig ausgewählt sind aus Wasserstoff, substituiertem oder unsubstituiertem Alkyl, substituiertem oder unsubstituiertem Cycloalkyl und substituiertem oder unsubstituiertem Aryl;
Z -CR_{g}Rₕ- ist;
R_{g} und Rₕ Wasserstoff sind;
"m" eine ganze Zahl ist, die im Bereich von 1 bis 3 liegt, beide einschließlich; "n" eine ganze Zahl ist, die im Bereich von 1 bis 3 liegt, beide einschließlich; und "q" eine ganze Zahl ist, die im Bereich von 0 bis 4 liegt, beide einschließlich; 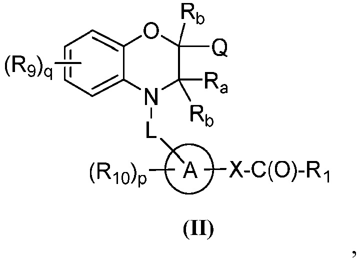
wobei
Q wie folgt ist, 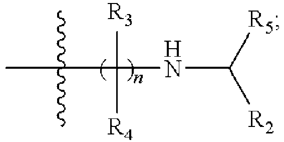
Rₐ Wasserstoff, Halogen oder Alkyl ist;
R_{b} ausgewählt ist aus Wasserstoff, Alkyl und Haloalkyl;
oder Rₐ und R_{b} zusammen an der gleichen Kohlenstoffform C(O) angehängt sind;
L eine Bindung oder -CR_{c}R_{d}- ist;
Ring A Phenyl ist;
R_{c} und R_{d} unabhängig ausgewählt sind aus Wasserstoff, Halogen und Alkyl;
X ausgewählt ist aus einer Bindung, -(CRₑR_{f})ₘ, -O-, -O(CRₑR_{f})ₘ-, -(CRₑR_{f})ₘO-
, -C(O)(CRₑR_{f})ₘ-, -C(O)NR₇-, -C(O)NR₇(CRₑR_{f})ₘ-, -Cycloalkylen- und -O-Cycloalkylen-;
Rₑ und R_{f}, die bei jedem Auftreten gleich oder verschieden sein können, unabhängig ausgewählt sind aus Wasserstoff, Halogen, Alkyl, Haloalkyl und Cycloalkyl; oder Rₑ und R_{f} zusammen mit dem Kohlenstoffatom, an das sie angehängt sind, einen 3- bis 7-gliedrigen gesättigten carbocyclischen Ring bilden können;
R₁ -OR₆ oder -NR₇R₈ ist;
R₂ substituiertes oder unsubstituiertes Aryl ist;
R₃ und R₄ unabhängig ausgewählt sind aus Wasserstoff, Halogen, Alkyl, Haloalkyl und Cycloalkyl;
R₅ Alkyl oder Haloalkyl ist;
R₆ Wasserstoff oder Alkyl ist;
R₇ und R₈ unabhängig ausgewählt sind aus Wasserstoff, Alkyl, Cycloalkyl, Cycloalkylalkyl und Aryl;
R₉, das bei jedem Auftreten gleich oder verschieden sein kann, unabhängig ausgewählt ist aus Halogen, Cyano, Alkyl, Haloalkyl und Cycloalkyl;
R₁₀, das bei jedem Auftreten gleich oder verschieden sein kann, unabhängig ausgewählt ist aus Halogen, Cyano, Alkyl, Alkenyl, Alkinyl, Haloalkyl, Hydroxyalkyl, -OR₆, -C(O)R₆, - NR₇C(O)R₆, -S(O)₀₋₂R₆, -S(O)₂NR₇R₈ und -NR₇S(O)₂R₆;
"m" eine ganze Zahl ist, die im Bereich von 1 bis 3 liegt, beide einschließlich; "n" eine ganze Zahl ist, die im Bereich 1 bis 3 liegt, beide einschließlich; "p" eine ganze Zahl ist, die im Bereich 0 bis 2 liegt, beide einschließlich; und "q" eine ganze Zahl ist, die im Bereich von 0 bis 1 liegt, beide einschließlich; 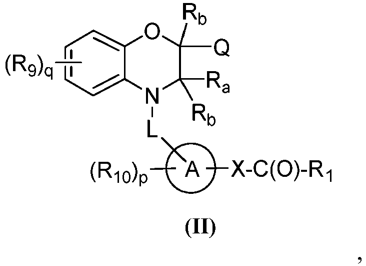
wobei Q wie folgt ist, 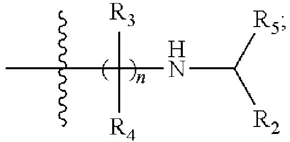
Rₐ Wasserstoff ist; R_{b} Wasserstoff oder Alkyl ist;
L eine Bindung oder -CR_{c}R_{d} ist;
Ring A Phenyl ist;
R_{c} und R_{d} unabhängig ausgewählt sind aus Wasserstoff, Halogen und Alkyl;
X ausgewählt ist aus einer Bindung, -(CRₑR_{f})ₘ, -O-, -O(CRₑR_{f})ₘ-, -(CRₑR_{f})ₘO-, -C(O)(CRₑR_{f})ₘ-, -C(O)NR₇- und -C(O)NR₇(CRₑR_{f})ₘ-;
Rₑ und R_{f}, die bei jedem Auftreten gleich oder verschieden sein können, unabhängig ausgewählt sind aus Wasserstoff, Halogen, Alkyl, Haloalkyl und Cycloalkyl; oder Rₑ und R_{f} zusammen mit dem Kohlenstoffatom, an das sie angehängt sind, einen 3- bis 7-gliedrigen gesättigten carbocyclischen Ring bilden;
R₁ -OR₆ oder -NR₇R₈ ist;
R₂ Phenyl oder Naphthyl ist, wobei das Phenyl mit Halogen, Alkyl, Haloalkyl, Alkoxy oder Haloalkoxy substituiert ist;
R₃ und R₄ Wasserstoff sind;
R₅ Alkyl ist;
R₆ Wasserstoff oder Alkyl ist;
R₇ und R₈ Wasserstoff oder Alkyl sind;
R₉ unabhängig aus Halogen, Cyano, Alkyl, Haloalkyl und Cycloalkyl ausgewählt ist;
R₁₀, das bei jedem Auftreten gleich oder verschieden sein kann, unabhängig ausgewählt ist aus Halogen, Cyano, Alkyl, Haloalkyl, Hydroxyalkyl, -OR₆, -C(O)R₆, -NR₇R₈, - NR₇C(O)R₆ und -(O)₂NR₇R₈;
"m" eine ganze Zahl ist, die im Bereich von 1 bis 3 liegt, beide einschließlich; "n" eine ganze Zahl ist, die im Bereich 1 bis 3 liegt, beide einschließlich; "p" eine ganze Zahl ist, die im Bereich 0 bis 2 liegt, beide einschließlich; "q" eine ganze Zahl ist, die im Bereich von 0 bis 1 liegt, beide einschließlich; 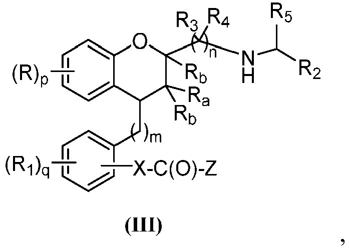
wobei
Rₐ ausgewählt ist aus Wasserstoff, Halogen, substituiertem oder unsubstituiertem Alkyl, Cyano, substituiertem oder unsubstituiertem Cycloalkyl und substituiertem oder unsubstituiertem Haloalkyl;
R_{b}, das bei jedem Auftreten gleich oder verschieden sein kann, unabhängig ausgewählt ist aus Wasserstoff, Halogen, substituiertem oder unsubstituiertem Alkyl, substituiertem oder unsubstituiertem Cycloalkyl und substituiertem oder unsubstituiertem Haloalkyl;
R_{c}, das bei jedem Auftreten gleich oder verschieden sein kann, unabhängig ausgewählt ist aus Halogen, Hydroxy, substituiertem oder unsubstituiertem Alkyl, substituiertem oder unsubstituiertem Haloalkyl, substituiertem oder unsubstituiertem Alkenyl, substituiertem oder unsubstituiertem Alkinyl, substituiertem oder unsubstituiertem Cycloalkyl, OR₆, Nitro, Cyano, - C(O)OR₆, -(CH₂)ᵣ-C(O)OR₆, -O-C(O)OR₆, -O(CH₂)ᵣ-C(O)OR₆, -NR₇R₈, - (CH₂)ᵣNR₇R₈-, -C(O)R₉, -C(O)NR₇R₈, -(CH₂)ᵣ-C(O)NR₇R₈, -NR₇C(O)R₉, -S(O)₀₋₂R₆, -S(O)₂NR₇R₈ und -NR₇S(O)₂R₉;
X ausgewählt ist aus einer Bindung, -(CR_{c}R_{d})ᵣ-, -O-, -NR₇-, -NR₇(CR_{c}R_{d})ᵣ-, -O(CR_{c}R_{d})ᵣ, -C(O)NR₇-, -C(O)NR₇(CR_{c}R_{d})ᵣ, -(CRₑR_{d})ᵣNR₇(CRₑR_{d})ᵣ, - (CRₑR_{d})ᵣCycloalkylen-, Cycloalkylen, -Cycloalkylen(CR_{c}R_{d})ᵣ- und -O-Cycloalkylen, wobei Cycloalkylen substituiert oder unsubstituiert sein kann;
R_{c} und R_{d}, die bei jedem Auftreten gleich oder verschieden sein können, unabhängig ausgewählt sind aus Wasserstoff, Halogen, Hydroxy, Cyano, Nitro, substituiertem oder unsubstituiertem Alkyl, substituiertem oder unsubstituiertem Haloalkyl und substituiertem oder unsubstituiertem Cycloalkyl; oder R_{c} und R_{d} zusammen mit dem Kohlenstoffatom, an das sie angehängt sind, einen substituierten oder unsubstituierten 3- bis 7-gliedrigen gesättigten carbocyclischen Ring bilden können;
Z -OR₆ oder -NR₁₀R₁₁ ist;
R₁, das bei jedem Auftreten gleich oder verschieden sein kann, unabhängig ausgewählt ist aus Halogen, Nitro, Cyano, substituiertem oder unsubstituiertem Alkyl, substituiertem oder unsubstituiertem Alkenyl, substituiertem oder unsubstituiertem Alkinyl, substituiertem oder unsubstituiertem Haloalkyl, substituiertem oder unsubstituiertem Cycloalkyl, -OR₆, -C(O)R₉, -NR₇R₈, -(CH₂)ᵣNR₇R₈-, -(CH₂)ᵣ-C(O)OR₆, -O-C(O)OR₆, -O(CH₂)ᵣ-C(O)OR₆, - C(O)NR₇R₈, -(CH₂)ᵣ-C(O)NR₇R₈, -NR₇C(O)R₉, -S(O)₀₋₂R₇, -S(O)₂NR₇R₈ und - NR₇S(O)₂R₉;
R₂ ausgewählt ist aus substituiertem oder unsubstituiertem Aryl, substituiertem oder unsubstituiertem Heteroaryl und substituiertem oder unsubstituiertem Heterocyclyl;
R₃ und R₄ gleich oder verschieden sein können und unabhängig ausgewählt sind aus Wasserstoff, Halogen, substituiertem oder unsubstituiertem Alkyl, substituiertem oder unsubstituiertem Haloalkyl, substituiertem oder unsubstituiertem Alkenyl, substituiertem oder unsubstituiertem Alkinyl, substituiertem oder unsubstituiertem Alkoxy, substituiertem oder unsubstituiertem Haloalkoxy und substituiertem oder unsubstituiertem Cycloalkyl;
R₅ substituiertes oder unsubstituiertes Alkyl ist;
R₆, das bei jedem Auftreten gleich oder verschieden sein kann, unabhängig ausgewählt ist aus Wasserstoff, substituiertem oder unsubstituiertem Alkyl, substituiertem oder unsubstituiertem Haloalkyl, substituiertem oder unsubstituiertem Alkenyl, substituiertem oder unsubstituiertem Alkinyl und substituiertem oder unsubstituiertem Aryl;
R₇ und R₈, die bei jedem Auftreten gleich oder verschieden sein können, unabhängig ausgewählt sind aus Wasserstoff, substituiertem oder unsubstituiertem Alkyl, substituiertem oder unsubstituiertem Alkenyl, substituiertem oder unsubstituiertem Alkinyl, substituiertem oder unsubstituiertem Cycloalkyl, substituiertem oder unsubstituiertem Cycloalkylalkyl, substituiertem oder unsubstituiertem Aryl, substituiertem oder unsubstituiertem Arylalkyl, substituiertem oder unsubstituiertem Heteroaryl, substituiertem oder unsubstituiertem Heteroarylalkyl, substituiertem oder unsubstituiertem Heterocyclyl und substituiertem oder unsubstituiertem Heterocyclylalkyl; oder R₇ und R₈ zusammen mit dem Stickstoffatom, an das sie angehängt sind, einen substituierten oder unsubstituierten, gesättigten oder ungesättigten 3- bis 12-gliedrigen cyclischen Ring bilden können, wobei der ungesättigte cyclische Ring eine oder zwei Doppelbindungen aufweisen kann;
bei jedem Vorkommen R₉ substituiertes oder unsubstituiertes Alkyl oder substituiertes oder unsubstituiertes Aryl ist;
R₁₀ und R₁₁ gleich oder verschieden sein können und unabhängig ausgewählt sind aus Wasserstoff, substituiertem oder unsubstituiertem Alkyl, substituiertem oder unsubstituiertem Alkenyl, substituiertem oder unsubstituiertem Alkinyl, -(CR_{c}R_{d})ᵣ-C(O)OR₆, substituiertem oder unsubstituiertem Cycloalkyl, substituiertem oder unsubstituiertem Cycloalkylalkyl, substituiertem oder unsubstituiertem Aryl, substituiertem oder unsubstituiertem Arylalkyl, substituiertem oder unsubstituiertem Heteroaryl, substituiertem oder unsubstituiertem Heteroarylalkyl, substituiertem oder unsubstituiertem Heterocyclyl und substituiertem oder unsubstituiertem Heterocyclylalkyl; oder R₁₀ und R₁₁ zusammen mit dem Stickstoffatom, an das sie angehängt sind, einen substituierten oder unsubstituierten, gesättigten oder ungesättigten 3- bis 12-gliedrigen cyclischen Ring bilden können, wobei der ungesättigte cyclische Ring eine oder zwei Doppelbindungen aufweisen kann;
"n" eine ganze Zahl ist, die im Bereich von 1 bis 3 liegt, beide einschließlich; "m" eine ganze Zahl ist, die im Bereich von 0 bis 3 liegt, beide einschließlich; "p" eine ganze Zahl ist, die im Bereich von 0 bis 4 liegt, beide einschließlich; "q" eine ganze Zahl ist, die im Bereich von 0 bis 3 liegt, beide einschließlich; und "r" eine ganze Zahl ist, die im Bereich von 1 bis 3 liegt, beide einschließlich; 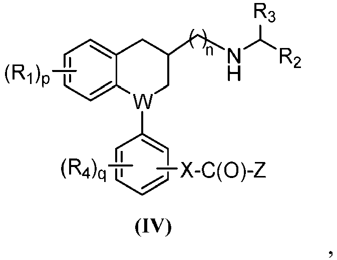
wobei
W CH oder N ist;
R₁, das bei jedem Auftreten gleich oder verschieden sein kann, unabhängig ausgewählt ist aus Halogen, Nitro, Cyano, substituiertem oder unsubstituiertem Alkyl, substituiertem oder unsubstituiertem Haloalkyl, substituiertem oder unsubstituiertem Cycloalkyl, -C(O)OR₅, - (CRₐR_{b})ᵣ-C(O)OR₅, -O-C(O)OR₅, -O(CRₐR_{b})ᵣ-C(O)OR₅, -NR₆R₇, -C(O)R₈, - C(O)NR₆R₇, -NR₆C(O)R₈, -S(O)₀₋₂R₅, -S(O)₂NR₆R₇ und -NR₆S(O)₂R₈;
R₂ substituiertes oder unsubstituiertes Aryl ist;
R₃ substituiertes oder unsubstituiertes Alkyl ist;
R₄, das bei jedem Auftreten gleich oder verschieden sein kann, unabhängig ausgewählt ist aus Halogen, Cyano, substituiertem oder unsubstituiertem Alkyl, substituiertem oder unsubstituiertem Haloalkyl, -OR₅, -NR₆R₇, -C(O)R₈, -C(O)NR₆R₇, -NR₆C(O)R₈, - S(O)₀₋₂R₅, -S(O)₂NR₆R₇ und -NR₅S(O)₂R₈;
X ausgewählt ist aus einer Bindung, -(CRₐR_{b})ᵣ-, -O-, -NR₇-, -O(CRₐR_{b})ᵣ-, - C(O)NR₇-, -C(O)NR₇(CRₐR_{b})ᵣ-, -(CRₐR_{b})ᵣCycloalkylen-, Cycloalkylen, Cycloalkylen-(CRₐR_{b})ᵣ- und -O-Cycloalkylen;
Rₐ und R_{b}, die bei jedem Auftreten gleich oder verschieden sein können, unabhängig ausgewählt sind aus Wasserstoff, Halogen, Hydroxy, Cyano, substituiertem oder unsubstituiertem Alkyl, substituiertem oder unsubstituiertem Haloalkyl und substituiertem oder unsubstituiertem Cycloalkyl; oder Rₐ und R_{b} zusammen mit dem Kohlenstoffatom, an das sie angehängt sind, einen substituierten oder unsubstituierten 3- bis 7-gliedrigen gesättigten carbocyclischen Ring bilden können;
Z -OR₅ oder -NR₆R₇ ist;
R₅, das bei jedem Auftreten gleich oder verschieden sein kann, unabhängig ausgewählt ist aus Wasserstoff, substituiertem oder unsubstituiertem Alkyl, substituiertem oder unsubstituiertem Haloalkyl und substituiertem oder unsubstituiertem Aryl;
R₆ und R₇, die bei jedem Auftreten gleich oder verschieden sein können, unabhängig ausgewählt sind aus Wasserstoff, substituiertem oder unsubstituiertem Alkyl, -(CRₐR_{b})ᵣ-C(O)OR₅, substituiertem oder unsubstituiertem Cycloalkyl, substituiertem oder unsubstituiertem Cycloalkylalkyl, substituiertem oder unsubstituiertem Aryl, substituiertem oder unsubstituiertem Arylalkyl, substituiertem oder unsubstituiertem Heteroaryl, substituiertem oder unsubstituiertem Heteroarylalkyl, substituiertem oder unsubstituiertem Heterocyclyl und substituiertem oder unsubstituiertem Heterocyclylalkyl; oder R₆ und R₇ zusammen mit dem Stickstoffatom, an das sie angehängt sind, einen substituierten oder unsubstituierten, gesättigten oder ungesättigten 3- bis 10-gliedrigen cyclischen Ring bilden können, wobei der ungesättigte cyclische Ring eine oder zwei Doppelbindungen aufweisen kann;
R₈ substituiertes oder unsubstituiertes Alkyl oder substituiertes oder unsubstituiertes Aryl ist;
"n" eine ganze Zahl ist, die im Bereich von 0 bis 3 liegt, beide einschließlich; "p" eine ganze Zahl ist, die im Bereich von 0 bis 3 liegt, beide einschließlich; "q" eine ganze Zahl ist, die im Bereich von 0 bis 3 liegt, beide einschließlich; und "r" eine ganze Zahl ist, die im Bereich von 1 bis 3 liegt, beide einschließlich; 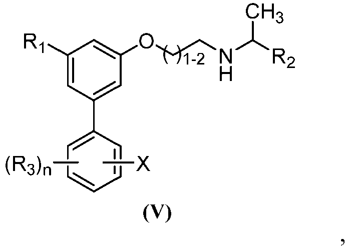
wobei
R₁ ausgewählt ist aus Halogen, substituiertem oder unsubstituiertem Alkyl, substituiertem oder unsubstituiertem Haloalkyl, substituiertem oder unsubstituiertem Cycloalkyl, substituiertem oder unsubstituiertem Alkoxy, substituiertem oder unsubstituiertem Haloalkoxy, -(CRₐR_{b})₁₋₃OH, -C(O)NH-Alkyl, -S(O)₂-Alkyl, -S(O)₂NH-Alkyl, -C(O)OH, -C(O)O-Alkyl, -(CRₐR_{b})₁₋₃C(O)OH und -(CRₐR_{b})₁₋₃C(O)O-Alkyl;
R₂ substituiertes oder unsubstituiertes Phenyl oder substituiertes oder unsubstituiertes Naphthyl ist, wobei die Substituenten ein oder mehrere sein können und unabhängig ausgewählt sind aus Halogen, substituiertem oder unsubstituiertem Alkyl, substituiertem oder unsubstituiertem Haloalkyl, -OR₄, -NR₅R₆ und substituiertem oder unsubstituiertem Cycloalkyl;
X ausgewählt ist aus -C(O)OH, -C(O)O-Alkyl, -C(O)NR₅R₆, -(CRₐR_{b})₁₋₃C(O)OH, - C(O)O-Alkyl, -O-(CRₐR_{b})₁₋₃C(O)OH, -O-(CRₐR_{b})₁₋₃C(O)O-Alkyl, -CR_{C}=CR_{C}-, -C(O)OH, Halogen, substituiertem oder unsubstituiertem Alkyl, substituiertem oder unsubstituiertem Haloalkyl, -OR₄ und -S(O)₂-Alkyl;
Rₐ und R_{b} unabhängig ausgewählt sind aus Wasserstoff, Halogen, Hydroxy, substituiertem oder unsubstituiertem Alkyl, substituiertem oder unsubstituiertem Haloalkyl und substituiertem oder unsubstituiertem Cycloalkyl; oder Rₐ und R_{b} zusammen mit dem Kohlenstoffatom, an das sie angehängt sind, einen substituierten oder unsubstituierten 3- bis 6-gliedrigen gesättigten carbocyclischen Ring bilden können;
R_{c} unabhängig ausgewählt ist aus Wasserstoff, Halogen und substituiertem oder unsubstituiertem Alkyl;
R₃, das bei jedem Auftreten gleich oder verschieden sein kann, unabhängig ausgewählt ist aus Halogen, Cyano, substituiertem oder unsubstituiertem Alkyl, substituiertem oder unsubstituiertem Haloalkyl und -OR₄;
R₄ ausgewählt ist aus Wasserstoff, substituiertem oder unsubstituiertem Alkyl, substituiertem oder unsubstituiertem Haloalkyl und substituiertem oder unsubstituiertem Cycloalkyl;
R₅ und R₆ unabhängig ausgewählt sind aus Wasserstoff, substituiertem oder unsubstituiertem Alkyl und substituiertem oder unsubstituiertem Cycloalkyl; und "n" eine ganze Zahl ist, die im Bereich von 0 bis 2 liegt, beide einschließlich; 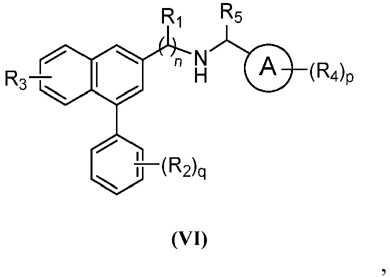
wobei
Ring A Phenyl oder Naphthyl ist;
R₁ Wasserstoff oder substituiertes oder unsubstituiertes (C₁-C₆)Alkyl ist;
R₂, das bei jedem Auftreten gleich oder verschieden sein kann, unabhängig ausgewählt ist aus Halogen, Cyano, substituiertem oder unsubstituiertem (C₁-C₆)Alkyl, substituiertem oder unsubstituiertem (C₁-C₆)Haloalkyl, substituiertem oder unsubstituiertem (C₁-C₆)Hydroxyalkyl, - X-C(O)-Z, -OR₉, -NR₇R₈, -NR₇C(O)R₆, -S(O)₀₋₂R₆, -S(O)₂NR₇R₈, -NR₇S(O)₂R₆, substituiertem oder unsubstituiertem (C₃-C₁₂)Cycloalkyl, substituiertem oder unsubstituiertem Phenyl, substituiertem oder unsubstituiertem 5- bis 6-gliedrigem Heteroaryl, substituiertem oder unsubstituiertem 5- bis 6-gliedrigem Heterocyclyl und Ring D;
Ring D wie folgt ist, 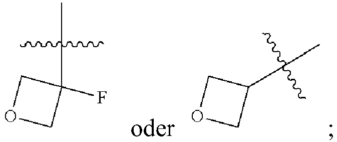
X ausgewählt ist aus einer Bindung, -(CRₐR_{b})ₘ-, -NR₂-, -O(CRₐR_{b})ₘ-,-(CRₐR_{b})ₘO-, -C(O)NR₁₂-, -(CRₐR_{b})ₘO-(CRₐR_{b})ₘ- und -C(O)NR₁₂(CRₐR_{b})ₘ-;
Rₐ und R_{b}, die bei jedem Auftreten gleich oder verschieden sein können, unabhängig ausgewählt sind aus Wasserstoff, Halogen, Hydroxy, substituiertem oder unsubstituiertem (C₁-C₆)Alkyl, substituiertem oder unsubstituiertem (C₁-C₆)Haloalkyl und substituiertem oder unsubstituiertem (C₃-C₆)Cycloalkyl; oder Rₐ und R_{b} zusammen mit dem Kohlenstoffatom, an das sie angehängt sind, einen substituierten oder unsubstituierten 3- bis 6-gliedrigen gesättigten carbocyclischen Ring bilden;
Z -OR₁₀ oder -NR₇R₈ ist;
R₃ ausgewählt ist aus Wasserstoff, Halogen, substituiertem oder unsubstituiertem (C₁-C₆)Alkyl, substituiertem oder unsubstituiertem (C₁-C₆)Haloalkyl, -OR₉ und substituiertem oder unsubstituiertem (C₃-C₁₂)Cycloalkyl;
R₄, das bei jedem Auftreten gleich oder verschieden sein kann, unabhängig ausgewählt ist aus Halogen, substituiertem oder unsubstituiertem (C₁-C₆)Alkyl, substituiertem oder unsubstituiertem (C₁-C₆)Haloalkyl, substituiertem oder unsubstituiertem (C₁-C₆)Alkoxyalkyl,-SF₅ und -OR₉;
R₅ substituiertes oder unsubstituiertes (C₁-C₆)Alkyl ist;
R₆ ausgewählt ist aus substituiertem oder unsubstituiertem (C₁-C₆)Alkyl, substituiertem oder unsubstituiertem (C₃-C₁₂)Cycloalkyl und substituiertem oder unsubstituiertem (C₆-C₁₄)Aryl;
R₇ und R₈, die bei jedem Auftreten gleich oder verschieden sein können, unabhängig ausgewählt sind aus Wasserstoff, substituiertem oder unsubstituiertem (C₁-C₆)Alkyl, -(CRₐR_{b})₁₋₂R₁₁, -(CR_{c}R_{d})ₘ-OH und substituiertem oder unsubstituiertem (C₃-C₁₂)Cycloalkyl;
R_{c} und R_{d}, die bei jedem Auftreten gleich oder verschieden sein können, unabhängig Wasserstoff oder substituiertes oder unsubstituiertes (C₁-C₆)Alkyl sind;
R₉ unabhängig ausgewählt ist aus Wasserstoff, substituiertem oder unsubstituiertem (C₁-C₆)Alkyl, substituiertem oder unsubstituiertem (C₁-C₆)Haloalkyl, substituiertem oder unsubstituiertem (C₁-C₆)Alkoxyalkyl und substituiertem oder unsubstituiertem (C₃-C₁₂)Cycloalkyl;
R₁₀ ausgewählt ist aus Wasserstoff, substituiertem oder unsubstituiertem (C₁-C₆)-Alkyl und -(CRₐR_{b})₁₋₂Phenyl;
R₁₁ substituiertes oder unsubstituiertes Phenyl ist, wobei die Substituenten ausgewählt sind aus Halogen, (C₁-C₆)Alkyl und -OR₉;
R₁₂ Wasserstoff oder substituiertes oder unsubstituiertes (C₁-C₆)Alkyl ist;
"m" eine ganze Zahl ist, die im Bereich von 1 bis 3 liegt, beide einschließlich; "n" eine ganze Zahl ist, die im Bereich von 1 bis 3 liegt, beide einschließlich; "p" eine ganze Zahl ist, die im Bereich von 0 bis 3 liegt, beide einschließlich; und "q" eine ganze Zahl ist, die im Bereich von 1 bis 3 liegt, beide einschließlich;
oder pharmazeutisch verträgliche Salze davon oder eine Kombination davon und zumindest zwei pharmazeutisch unbedenkliche Hilfsstoffe, wobei die zumindest zwei pharmazeutisch verträglichen Hilfsstoffe mikrokristalline Cellulose, Crospovidon, Stärke, Magnesiumstearat, Natriumlaurylsulfat, kolloidales Siliziumdioxid oder eine Kombination davon sind; und
wobei die feste pharmazeutische Zusammensetzung für eine orale Verabreichung geeignet ist und in der Form einer Tablette oder Kapsel vorliegt.

2. Feste pharmazeutische Zusammensetzung nach Anspruch 1, wobei die eine oder mehreren Verbindungen Folgendes sind:
3-((S)-2-((((R)-1-(Naphthalen-1-yl)ethyl)amino)methyl)morpholino)-5-(trifluormethyl)benzoesäure, ein inneres Salz oder Chlorwasserstoffsäuresalz davon ("Verbindung 1");
2-Methyl-5-((S)-2-(2-(((R)-1-(naphthalen-1-yl)ethyl)amino)ethyl)-2H-benzo[b][1,4]oxazin-4(3H)-yl)benzoesäure, ein inneres Salz oder Chlorwasserstoffsäuresalz davon ("Verbindung 2");
2-Methyl-5-((2R,4S)-2-((((R)-1-(naphthalen-1-yl)ethyl)amino)methyl)chroman-4-yl)benzoesäurehydrochlorid ("Verbindung 3");
3-((1R,3S)-3-((((R)-1-(4-Fluor-3-methoxyphenyl)ethyl)amino)methyl)-1,2,3,4-tetrahydronaphthalen-1-yl)-2,6-dimethylbenzoesäure, ein inneres Salz oder Chlorwasserstoffsäuresalz davon ("Verbindung 4");
(R)-3-(4-Fluor-3'-(2-((1-(3-methoxyphenyl)ethyl)amino)ethoxy)-5'-(trifluormethyl)-[1,1'-biphenyl]-3-yl)propansäure, ein inneres Salz oder Chlorwasserstoffsäuresalz davon ("Verbindung 5");
(R)-N-((R)-1-(3-Methoxyphenyl)ethyl)-1-(4-(4-(trifluormethyl)phenyl)naphthalen-2-yl)propan-1-aminhydrochlorid ("Verbindung 6"); oder
eine Kombination davon.

3. Feste pharmazeutische Zusammensetzung nach Anspruch 1 oder 2, wobei es sich bei der einen oder den mehreren Verbindungen um Verbindung 3 handelt.

4. Feste pharmazeutische Zusammensetzung nach einem der Ansprüche 1-3, wobei die Zusammensetzung in der Form einer Tablette vorliegt, wobei die Tablette mit einem Film beschichtet ist.

5. Feste pharmazeutische Zusammensetzung nach Anspruch 4, wobei der Film eine resistente Stärke, eine Maisstärke mit hohem Amylosegehalt, Poly(methacrylsäure-co-methylmethacrylat) 1:2, Poly(ethylacrylat-co-methylmethacrylat-co-trimethylammonioethylmethacrylatchlorid) 1:2:0,1, Poly(ethylacrylat-co-methylmethacrylat-co-trimethylammonioethylmethacrylatchlorid) 1:2:0,2, Stärkeacetat, Ethylcellulose, Croscarmellosenatrium, Natriumstärkeglycolat, Celluloseether, Vinylpolymer, Glycol, Acrylpolymer, Maltodextrin, Polydextrose, Celluloseacetatphthalat, Celluloseacetattrimellitat, Hydroxypropylmethylcelluloseacetatsuccinat-Hypromelloseacetatsuccinat, Hydroxypropylmethylcellulosephthalat, Polyvinylacetatphthalat, Poly(methacrylsäure-co-methylmethacrylat), Schellack oder eine Kombination davon ist.

6. Feste pharmazeutische Zusammensetzung, umfassend eine oder mehrere Verbindungen und einen oder mehrere Inhaltsstoffe, wobei die eine oder mehreren Verbindungen Verbindung 1, Verbindung 2, Verbindung 3, Verbindung 4, Verbindung 5, Verbindung 6, ein pharmazeutisch verträgliches Salz davon oder eine Kombination davon sind und wobei der eine oder die mehreren Inhaltsstoffe Folgendes sind:
(a) mikrokristalline Cellulose in der Menge von 5 Gew.-% bis 50 Gew.-% des Gesamtgewichts der festen pharmazeutischen Zusammensetzung;
(b) Crospovidon in der Menge von 5 Gew.-% bis 20 Gew.-% des Gesamtgewichts der festen pharmazeutischen Zusammensetzung oder Povidon in der Menge von 3 Gew.-% bis 20 Gew.-% des Gesamtgewichts der festen pharmazeutischen Zusammensetzung oder eine Kombination davon;
(c) vorverkleisterte Stärke in der Menge von 20 Gew.-% bis 50 Gew.-% des Gesamtgewichts der festen pharmazeutischen Zusammensetzung;
(d) Magnesiumstearat in der Menge von 0,5 Gew.-% bis 5 Gew.-% des Gesamtgewichts der festen pharmazeutischen Zusammensetzung oder Talk in der Menge von 0,5 Gew.-% bis 2 Gew.-% des Gesamtgewichts der festen pharmazeutischen Zusammensetzung oder eine Kombination davon;
(e) Natriumlaurylsulfat (SLS) in der Menge von 0,5 Gew.-% bis 3 Gew.-% des Gesamtgewichts der festen pharmazeutischen Zusammensetzung;
(f) kolloidales Siliciumdioxid in einer Menge von 0,5 Gew.-% bis 3 Gew.-% des Gesamtgewichts der festen pharmazeutischen Zusammensetzung; oder
(g) eine Kombination davon.

7. Feste pharmazeutische Zusammensetzung, umfassend 2-Methyl-5-((2R,4S)-2-((((R)-1-(naphthalen-1-yl)ethyl)amino)methyl)chroman-4-yl)benzoesäurehydrochlorid und einen oder mehrere Inhaltsstoffe, wobei der eine oder die mehreren Inhaltsstoffe Folgendes sind:
(a) mikrokristalline Cellulose in der Menge von 5 Gew.-% bis 50 Gew.-% des Gesamtgewichts der festen pharmazeutischen Zusammensetzung;
(b) Crospovidon in der Menge von 5 Gew.-% bis 20 Gew.-% des Gesamtgewichts der festen pharmazeutischen Zusammensetzung oder Povidon in der Menge von 3 Gew.-% bis 20 Gew.-% des Gesamtgewichts der festen pharmazeutischen Zusammensetzung oder eine Kombination davon;
(c) vorverkleisterte Stärke in der Menge von 20 Gew.-% bis 50 Gew.-% des Gesamtgewichts der festen pharmazeutischen Zusammensetzung;
(d) Magnesiumstearat in der Menge von 0,5 Gew.-% bis 5 Gew.-% des Gesamtgewichts der festen pharmazeutischen Zusammensetzung oder Talk in der Menge von 0,5 Gew.-% bis 2 Gew.-% des Gesamtgewichts der festen pharmazeutischen Zusammensetzung oder eine Kombination davon;
(e) Natriumlaurylsulfat (SLS) in der Menge von 0,5 Gew.-% bis 3 Gew.-% des Gesamtgewichts der festen pharmazeutischen Zusammensetzung;
(f) kolloidales Siliciumdioxid in einer Menge von 0,5 Gew.-% bis 3 Gew.-% des Gesamtgewichts der festen pharmazeutischen Zusammensetzung; oder
(g) eine Kombination davon.

8. Feste pharmazeutische Zusammensetzung nach einem der Ansprüche 1 bis 6, wobei die eine oder mehreren Verbindungen in der Menge von 0,1 mg bis 200 mg vorliegen.

9. Feste pharmazeutische Zusammensetzung nach einem der Ansprüche 1 bis 6, wobei die eine oder mehreren Verbindungen in der Menge von 1 mg bis 50 mg oder 5 mg bis 25 mg vorliegen.

10. Feste pharmazeutische Zusammensetzung nach Anspruch 7, wobei das 2-Methyl-5-((2R,4S)-2-((((R)-1-(naphthalen-1-yl)ethyl)amino)methyl)chroman-4-yl)benzoesäurehydrochlorid in der Menge von 0,1 mg bis 200 mg, 1 mg bis 50 mg oder 5 mg bis 25 mg vorliegt.

11. Feste pharmazeutische Zusammensetzung nach einem der Ansprüche 1-3, wobei die pharmazeutische Zusammensetzung eine Kapsel ist, wobei die Kapsel 35 Gew.-% bis 39 Gew.-% der einen oder mehreren Verbindungen umfasst.

12. Feste pharmazeutische Zusammensetzung wie in einem der Ansprüche 1-11 beschrieben zur Verwendung beim Behandeln von sekundärem Hyperparathyreoidismus im Zusammenhang mit chronischer Nierenerkrankung (CKD) bei einem Subjekt, das dessen bedarf.

13. Feste pharmazeutische Zusammensetzung zur Verwendung nach Anspruch 12, wobei die Verbindung in der festen pharmazeutischen Zusammensetzung 2-Methyl-5-((2R,4S)-2-((((R)-1-(naphthalen-1-yl)ethyl)amino)methyl)chroman-4-yl)benzoesäurehydrochlorid ist.

14. Feste pharmazeutische Zusammensetzung zur Verwendung nach Anspruch 12 oder Anspruch 13, wobei die Verwendung die Verabreichung der einen oder mehreren Verbindungen in einer täglichen Gesamtdosis von 5 mg bis 50 mg umfasst.

15. Feste pharmazeutische Zusammensetzung zur Verwendung nach einem von Anspruch 14, wobei die Verabreichung 25 mg einmal oder zweimal täglich oder 20 mg einmal oder zweimal täglich ist.

16. Feste pharmazeutische Zusammensetzung zur Verwendung nach einem der Ansprüche 12 bis 15, wobei die Verwendung eine orale Verabreichung der festen pharmazeutischen Zusammensetzung umfasst.

17. Feste pharmazeutische Zusammensetzung zur Verwendung nach einem der Ansprüche 12 bis 16, wobei die Verwendung keine Dosistitration beinhaltet.

18. Feste pharmazeutische Zusammensetzung zur Verwendung nach einem der Ansprüche 12 bis 17, wobei das Subjekt nicht auf Dialyse ist.

19. Feste pharmazeutische Zusammensetzung zur Verwendung nach einem der Ansprüche 12 bis 17, wobei das Subjekt auf Dialyse ist.

20. Feste pharmazeutische Zusammensetzung zur Verwendung nach einem der Ansprüche 12 bis 19, wobei der sekundäre Hyperparathyreoidismus behandelt oder kontrolliert wird, ohne Hyperphosphatämie oder Hypokalzämie bei dem Subjekt zu induzieren.

21. Feste pharmazeutische Zusammensetzung zur Verwendung nach einem der Ansprüche 12 bis 20, wobei das Subjekt zuvor diagnostiziert worden ist oder CKD im Stadium 3b, CKD im Stadium 4 oder CKD im Stadium 5 aufweist.

22. Feste pharmazeutische Zusammensetzung wie in einem der Ansprüche 1-11 beschrieben zur Verwendung beim Senken oder Unterdrücken von einem oder mehreren intakten Parathormon(iPTH-)Niveaus bei einem Subjekt, das an sekundärem Hyperparathyreoidismus im Zusammenhang mit CKD leidet.

23. Feste pharmazeutische Zusammensetzung zur Verwendung nach Anspruch 22, wobei die Verbindung in der festen pharmazeutischen Zusammensetzung 2-Methyl-5-((2R,4S)-2-((((R)-1-(naphthalen-1-yl)ethyl)amino)methyl)chroman-4-yl)benzoesäurehydrochlorid ist.

24. Feste pharmazeutische Zusammensetzung zur Verwendung nach Anspruch 22 oder 23, wobei das Subjekt nicht auf Dialyse ist.

25. Feste pharmazeutische Zusammensetzung zur Verwendung nach Anspruch 22 oder 23, wobei das Subjekt auf Dialyse ist.

26. Feste pharmazeutische Zusammensetzung zur Verwendung nach einem der Ansprüche 22 bis 25, wobei die Verwendung die Verabreichung der einen oder mehreren Verbindungen in einer täglichen Gesamtdosismenge von 5 mg bis 50 mg umfasst.

27. Feste pharmazeutische Zusammensetzung zur Verwendung nach Anspruch 26, wobei die tägliche Gesamtdosismenge 10 mg bis 40 mg beträgt.

28. Feste pharmazeutische Zusammensetzung zur Verwendung nach Anspruch 26, wobei die tägliche Gesamtdosismenge 25 mg einmal oder zweimal täglich oder 20 mg einmal oder zweimal täglich verabreicht wird.

29. Feste pharmazeutische Zusammensetzung zur Verwendung nach einem der Ansprüche 22 bis 27, wobei die Verwendung eine orale Verabreichung der festen pharmazeutischen Zusammensetzung umfasst.

30. Feste pharmazeutische Zusammensetzung zur Verwendung nach einem der Ansprüche 22 bis 29, wobei die Verabreichung keine Dosisanpassung an das Subjekt erforderlich macht.

31. Feste pharmazeutische Zusammensetzung zur Verwendung nach einem der Ansprüche 22 bis 30, wobei das iPTH-Niveau des Subjekts um zumindest 30 % verglichen mit dem Baseline-iPTH-Konzentrationsniveau vor einer Behandlung reduziert ist.

32. Feste pharmazeutische Zusammensetzung zur Verwendung nach einem der Ansprüche 22 bis 30, wobei das iPTH-Niveau des Subjekts um zumindest 15 % verglichen mit dem Baseline-iPTH-Konzentrationsniveau vor einer Behandlung reduziert ist.

33. Feste pharmazeutische Zusammensetzung von 2-Methyl-5-((2R,4S)-2-((((R)-1-(naphthalen-1-yl)ethyl)amino)methyl)chroman-4-yl)benzoesäurehydrochloridsalz zur Verwendung bei der Behandlung oder Kontrolle von sekundärem Hyperparathyreoidismus im Zusammenhang mit CKD.

34. Feste pharmazeutische Zusammensetzung von 2-Methyl-5-((2R,4S)-2-((((R)-1-(naphthalen-1-yl)ethyl)amino)methyl)chroman-4-yl)benzoesäurehydrochloridsalz zur Verwendung bei der Behandlung oder Kontrolle von sekundärem Hyperparathyreoidismus im Zusammenhang mit CKD durch Senken oder Unterdrücken von einem für mehrere iPTH-Niveaus bei einem Subjekt.

35. Feste pharmazeutische Zusammensetzung von 2-Methyl-5-((2R,4S)-2-((((R)-1-(naphthalen-1-yl)ethyl)amino)methyl)chroman-4-yl)benzoesäurehydrochloridsalz zur Verwendung nach Anspruch 34, wobei das iPTH-Niveau des Subjekts um zumindest 30 % verglichen mit dem Baseline-iPTH-Konzentrationsniveau vor einer Behandlung reduziert ist.

36. Feste pharmazeutische Zusammensetzung von 2-Methyl-5-((2R,4S)-2-((((R)-1-(naphthalen-1-yl)ethyl)amino)methyl)chroman-4-yl)benzoesäurehydrochloridsalz zur Verwendung nach Anspruch 34, wobei das iPTH-Niveau des Subjekts um zumindest 15 % verglichen mit dem Baseline-iPTH-Konzentrationsniveau vor einer Behandlung reduziert ist.

## Revendications

1. Composition pharmaceutique solide comprenant une quantité pharmaceutiquement efficace d'un ou de plusieurs composés, dans laquelle le ou les composés sont choisis dans le groupe constitué de composés présentant la structure de Formule (I), Formule (II), Formule (III), Formule (IV), Formule (V) ou Formule (VI) : 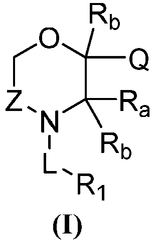 où
Q est 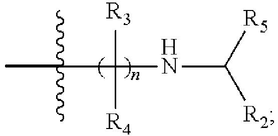
Rₐ est un hydrogène ;
R_{b} est un hydrogène ;
L est une liaison ou -(CRₑR_{d})ₘ ;
R_{c} et R_{d} sont choisis indépendamment parmi un hydrogène ou un alkyle substitué ou non substitué ;
R₁ est 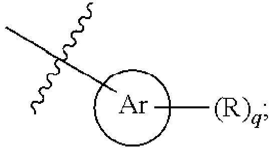
le cycle Ar est un phényle ou un naphtyle ;
R, qui peut être identique ou différent à chaque occurrence, est indépendamment choisi parmi un halogène, un nitro, un cyano, un alkyle substitué ou non substitué, un alcényle substitué ou non substitué, un alcynyle substitué ou non substitué, un halogénoalkyle substitué ou non substitué, -OR₆, -C(O)R₆, -(CRₑR_{f})₀₋₃-C(O)OR₆, -(CRₑR_{f})₁₋₂cycloalkylène-C(O)OR₆,-cycloalkylène(CRₑR_{f})₀₋₂-C(O)OR₆, -O(CRₑR_{f})₀₋₃-C(O)OR₆, -O-cycloalkylène-C(O)OR₆, -C(O)NR₇-(CRₑR_{f})₁₋₂-C(O)OR₆, -C(O)NR₇R₈, -S(O)₀₋₂R₆ et -S(O)₂NR₇R₈ ;
Rₑ et R_{f} sont indépendamment un hydrogène ou un alkyle substitué ou non substitué ;
R₂ est un aryle substitué ou non substitué ;
R₃ et R₄ sont indépendamment choisis parmi un hydrogène, un halogène et un alkyle substitué ou non substitué ;
R₅ est un alkyle ou un halogénoalkyle substitué ou non substitué ;
R₆, qui peut être identique ou différent à chaque occurrence, est indépendamment choisi parmi un hydrogène, un alkyle substitué ou non substitué et un halogénoalkyle substitué ou non substitué ;
R₇ et R₈, qui peuvent être identiques ou différents à chaque occurrence, sont indépendamment choisis parmi un hydrogène, un alkyle substitué ou non substitué, un cycloalkyle substitué ou non substitué et un aryle substitué ou non substitué ;
Z est -CR_{g}Rₕ- ;
R_{g} et Rₕ sont un hydrogène ;
« m » est un entier allant de 1 à 3, bornes incluses ; « n » est un entier allant de 1 à 3, bornes incluses ; et « q » est un entier allant de 0 à 4, bornes incluses ; 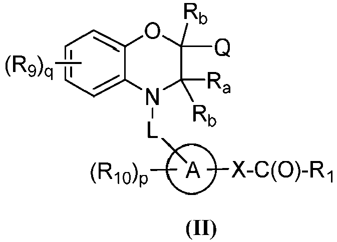
où
Q est 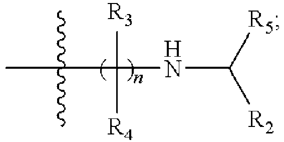
Rₐ est un hydrogène, un halogène ou un alkyle ;
R_{b} est choisi parmi un hydrogène, un alkyle et un halogénoalkyle ;
ou Rₐ et R_{b} liés ensemble sur le même carbone forment C(O) ;
L est une liaison ou -CR_{c}R_{d}- ;
le cycle A est un phényle ;
R_{c} et R_{d} sont indépendamment choisis parmi un hydrogène, un halogène et un alkyle ;
X est choisi parmi une liaison, -(CRₑR_{f})ₘ, -O-, -O(CRₑR_{f})ₘ-, -(CRₑR_{f})ₘO-,-C(O)(CRₑR_{f})ₘ-, -C(O)NR₇-, -C(O)NR₇(CRₑR_{f})ₘ-, -cycloalkylène- et -O-cycloalkylène- ;
Rₑ et R_{f}, qui peuvent être identiques ou différents à chaque occurrence, sont indépendamment choisis parmi un hydrogène, un halogène, un alkyle, un halogénoalkyle et un cycloalkyle ; ou Rₑ et R_{f}, conjointement avec l'atome de carbone auquel ils sont liés, peuvent former un cycle carbocyclique saturé à 3 à 7 chaînons ;
R₁ est -OR₆ ou -NR₇R₈ ;
R₂ est un aryle substitué ou non substitué ;
R₃ et R₄ sont indépendamment choisis parmi un hydrogène, un halogène, un alkyle, un halogénoalkyle et un cycloalkyle ;
R₅ est un alkyle ou un halogénoalkyle ;
R₆ est un hydrogène ou un alkyle ;
R₇ et R₈ sont indépendamment choisis parmi un hydrogène, un alkyle, un cycloalkyle, un cycloalkylalkyle et un aryle ;
R₉, qui peut être identique ou différent à chaque occurrence, est indépendamment choisi parmi un halogène, un cyano, un alkyle, un halogénoalkyle et un cycloalkyle ;
R₁₀, qui peut être identique ou différent à chaque occurrence, est indépendamment choisi parmi un halogène, un cyano, un alkyle, un alcényle, un alcynyle, un halogénoalkyle, un hydroxyalkyle, -OR₆, -C(O)R₆, -NR₇C(O)R₆, -S(O)₀₋₂R₆, -S(O)₂NR₇R₈ et-NR₇S(O)₂R₆ ;
« m » est un entier allant de 1 à 3, bornes incluses ; « n » est un entier allant de 1 à 3, bornes incluses ; « p » est un entier allant de 0 à 2, bornes incluses ; et « q » est un entier allant de 0 à 1, bornes incluses ; 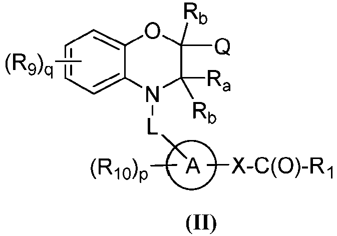
où Q est 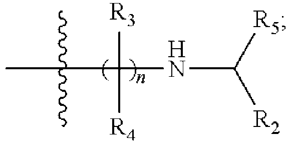
Rₐ est un hydrogène ; R_{b} est un hydrogène ou un alkyle ;
L est une liaison ou -CR_{c}R_{d}- ;
le cycle A est un phényle ;
R_{c} et R_{d} sont indépendamment choisis parmi un hydrogène, un halogène et un alkyle ;
X est choisi parmi une liaison, -(CRₑR_{f})ₘ, -O-, -O(CRₑR_{f})ₘ-, -(CRₑR_{f})ₘO-,-C(O)(CRₑR_{f})ₘ-, -C(O)NR₇- et -C(O)NR₇(CRₑR_{f})ₘ- ;
Rₑ et R_{f}, qui peuvent être identiques ou différents à chaque occurrence, sont indépendamment choisis parmi un hydrogène, un halogène, un alkyle, un halogénoalkyle et un cycloalkyle ; ou Rₑ et R_{f}, conjointement avec l'atome de carbone auquel ils sont liés, forment un cycle carbocyclique saturé à 3 à 7 chaînons ;
R₁ est -OR₆ ou -NR₇R₈ ;
R₂ est un phényle ou un naphtyle, ledit phényle étant substitué par un halogène, un alkyle, un halogénoalkyle, un alcoxy ou un halogénoalcoxy ;
R₃ et R₄ sont un hydrogène ;
R₅ est un alkyle ;
R₆ est un hydrogène ou un alkyle ;
R₇ et R₈ sont un hydrogène ou un alkyle ;
R₉ est indépendamment choisi parmi un halogène, un cyano, un alkyle, un halogénoalkyle et un cycloalkyle ;
R₁₀, qui peut être identique ou différent à chaque occurrence, est indépendamment choisi parmi un halogène, un cyano, un alkyle, un halogénoalkyle, un hydroxyalkyle, -OR₆, -C(O)R₆, -NR₇R₈, -NR₇C(O)R₆ et -(O)₂NR₇R₈ ;
« m » est un entier allant de 1 à 3, bornes incluses ; « n » est un entier allant de 1 à 3, bornes incluses ; « p » est un entier allant de 0 à 2, bornes incluses ; « q » est un entier allant de 0 à 1, bornes incluses ; 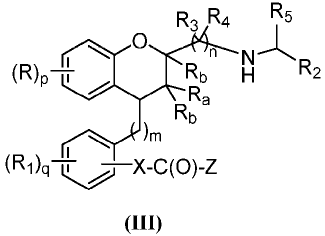
où
Rₐ est choisi parmi un hydrogène, un halogène, un alkyle substitué ou non substitué, un cyano, un cycloalkyle substitué ou non substitué et un halogénoalkyle substitué ou non substitué ;
R_{b}, qui peut être identique ou différent à chaque occurrence, est indépendamment choisi parmi un hydrogène, un halogène, un alkyle substitué ou non substitué, un cycloalkyle substitué ou non substitué et un halogénoalkyle substitué ou non substitué ;
R_{c} qui peut être identique ou différent à chaque occurrence, est indépendamment choisi parmi un halogène, un hydroxy, un alkyle substitué ou non substitué, un halogénoalkyle substitué ou non substitué, un alcényle substitué ou non substitué, un alcynyle substitué ou non substitué, un cycloalkyle substitué ou non substitué, OR₆, un nitro, un cyano, -C(O)OR₆, -(CH₂)ᵣ-C(O)OR₆, -O-C(O)OR₆, -O(CH₂)ᵣ-C(O)OR₆, -NR₇R₈, -(CH₂)ᵣNR₇R₈-, -C(O)R₉,-C(O)NR₇R₈, -(CH₂)ᵣ-C(O)NR₇R₈, -NR₇C(O)R₉, -S(O)₀₋₂R₆, -S(O)₂NR₇R₈ et-NR₇S(O)₂R₉ ;
X est choisi parmi une liaison, -(CRₑR_{d})ᵣ-, -O-, -NR₇-, -NR₇(CR_{c}R_{d})ᵣ-,-O(CR_{c}R_{d})ᵣ, -C(O)NR₇-, -C(O)NR₇(CR_{c}R_{d})ᵣ, -(CR_{c}R_{d})ᵣNR₇(CR_{c}R_{d})ᵣ,-(CR_{c}R_{d})ᵣcycloalkylène-, un cycloalkylène, -cycloalkylène(CR_{c}R_{d})ᵣ- et -O-(cycloalkylène) où un cycloalkylène peut être substitué ou non substitué ;
R_{c} et R_{d}, qui peuvent être identiques ou différents à chaque occurrence, sont indépendamment choisis parmi un hydrogène, un halogène, un hydroxy, un cyano, un nitro, un alkyle substitué ou non substitué, un halogénoalkyle substitué ou non substitué et un cycloalkyle substitué ou non substitué ; ou R_{c} et R_{d}, conjointement avec l'atome de carbone auquel ils sont liés, peuvent former un cycle carbocyclique saturé à 3 à 7 chaînons substitué ou non substitué ;
Z est -OR₆ ou -NR₁₀R₁₁ ;
R₁, qui peut être identique ou différent à chaque occurrence, est indépendamment choisi parmi un halogène, un nitro, un cyano, un alkyle substitué ou non substitué, un alcényle substitué ou non substitué, un alcynyle substitué ou non substitué, un halogénoalkyle substitué ou non substitué, un cycloalkyle substitué ou non substitué, -OR₆, -C(O)R₉, -NR₇R₈, - (CH₂)ᵣNR₇R₈-, -(CH₂)ᵣ-C(O)OR₆, -O-C(O)OR₆, -O(CH₂)ᵣ-C(O)OR₆, -C(O)NR₇R₈, -(CH₂)ᵣ-C(O)NR₇R₈, -NR₇C(O)R₉, -S(O)₀₋₂R₇, -S(O)₂NR₇R₈ et -NR₇S(O)₂R₉ ;
R₂ est choisi parmi un aryle substitué ou non substitué, un hétéroaryle substitué ou non substitué et un hétérocyclyle substitué ou non substitué ;
R₃ et R₄ peuvent être identiques ou différents et sont indépendamment choisis parmi un hydrogène, un halogène, un alkyle substitué ou non substitué, un halogénoalkyle substitué ou non substitué, un alcényle substitué ou non substitué, un alcynyle substitué ou non substitué, un alcoxy substitué ou non substitué, un halogénoalcoxy substitué ou non substitué et un cycloalkyle substitué ou non substitué ;
R₅ est un alkyle substitué ou non substitué ;
R₆, qui peut être identique ou différent à chaque occurrence, est indépendamment choisi parmi un hydrogène, un alkyle substitué ou non substitué, un halogénoalkyle substitué ou non substitué, un alcényle substitué ou non substitué, un alcynyle substitué ou non substitué et un aryle substitué ou non substitué ;
R₇ et R₈, qui peuvent être identiques ou différents à chaque occurrence, sont indépendamment choisis parmi un hydrogène, un alkyle substitué ou non substitué, un alcényle substitué ou non substitué, un alcynyle substitué ou non substitué, un cycloalkyle substitué ou non substitué, un cycloalkylalkyle substitué ou non substitué, un aryle substitué ou non substitué, un arylalkyle substitué ou non substitué, un hétéroaryle substitué ou non substitué, un hétéroarylalkyle substitué ou non substitué, un hétérocyclyle substitué ou non substitué et un hétérocyclylalkyle substitué ou non substitué ; ou R₇ et R₈, conjointement avec l'atome d'azote auquel ils sont liés, peuvent former un cycle cyclique à 3 à 12 chaînons, saturé ou insaturé, substitué ou non substitué, ledit cycle cyclique insaturé pouvant comporter une ou deux doubles liaisons ;
à chaque occurrence, R₉ est un alkyle substitué ou non substitué ou un aryle substitué ou non substitué ;
R₁₀ et R₁₁ peuvent être identiques ou différents et sont indépendamment choisis parmi un hydrogène, un alkyle substitué ou non substitué, un alcényle substitué ou non substitué, un alcynyle substitué ou non substitué, -(CR_{c}R_{d})ᵣ-C(O)OR₆, un cycloalkyle substitué ou non substitué, un cycloalkylalkyle substitué ou non substitué, un aryle substitué ou non substitué, un arylalkyle substitué ou non substitué, un hétéroaryle substitué ou non substitué, un hétéroarylalkyle substitué ou non substitué, un hétérocyclyle substitué ou non substitué et un hétérocyclylalkyle substitué ou non substitué ; ou R₁₀ et R₁₁, conjointement avec l'atome d'azote auquel ils sont liés, peuvent former un cycle cyclique à 3 à 12 chaînons, saturé ou insaturé, substitué ou non substitué, ledit cycle cyclique insaturé pouvant comporter une ou deux doubles liaisons ;
« n » est un entier allant de 1 à 3, bornes incluses ; « m » est un entier allant de 0 à 3, bornes incluses ; « p » est un entier allant de 0 à 4, bornes incluses ; « q » est un entier allant de 0 à 3, bornes incluses ; et « r » est un entier allant de 1 à 3, bornes incluses ; 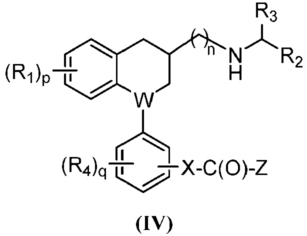
où
W est CH ou N ;
R₁, qui peut être identique ou différent à chaque occurrence, est indépendamment choisi parmi un halogène, un nitro, un cyano, un alkyle substitué ou non substitué, un halogénoalkyle substitué ou non substitué, un cycloalkyle substitué ou non substitué, -C(O)OR₅, -(CRₐR_{b})ᵣ-C(O)OR₅, -O-C(O)OR₅, -O(CRₐR_{b})ᵣ-C(O)OR₅, -NR₆R₇, -C(O)R₈, -C(O)NR₆R₇,-NR₆C(O)R₈, -S(O)₀₋₂R₅, -S(O)₂N₆R₇ et -NR₆S(O)₂R₈ ;
R₂ est un aryle substitué ou non substitué ;
R₃ est un alkyle substitué ou non substitué ;
R₄, qui peut être identique ou différent à chaque occurrence, est indépendamment choisi parmi un halogène, un cyano, un alkyle substitué ou non substitué, un halogénoalkyle substitué ou non substitué, -OR₅, -NR₆R₇, -C(O)R₈, -C(O)NR₆R₇, -NR₆C(O)R₈, -S(O)₀₋₂R₅,-S(O)₂NR₆R₇ et -NR₅S(O)₂R₈ ;
X est choisi parmi une liaison, -(CRₐR_{b})ᵣ-, -O-, -NR₇-, -O(CRₐR_{b})ᵣ-,-C(O)NR₇-, -C(O)NR₇(CRₐR_{b})ᵣ-, -(CRₐR_{b})ᵣcycloalkylène-, un cycloalkylène, cycloalkylène-(CRₐR_{b})ᵣ- et -O-cycloalkylène ;
Rₐ et R_{b}, qui peuvent être identiques ou différents à chaque occurrence, sont indépendamment choisis parmi un hydrogène, un halogène, un hydroxy, un cyano, un alkyle substitué ou non substitué, un halogénoalkyle substitué ou non substitué et un cycloalkyle substitué ou non substitué ; ou Rₐ et R_{b}, conjointement avec l'atome de carbone auquel ils sont liés, peuvent former un cycle carbocyclique saturé à 3 à 7 chaînons substitué ou non substitué ;
Z est -OR₅ ou -NR₆R₇ ;
R₅, qui peut être identique ou différent à chaque occurrence, est indépendamment choisi parmi un hydrogène, un alkyle substitué ou non substitué, un halogénoalkyle substitué ou non substitué et un aryle substitué ou non substitué ;
R₆ et R₇, qui peuvent être identiques ou différents à chaque occurrence, sont indépendamment choisis parmi un hydrogène, un alkyle substitué ou non substitué, -(CRₐR_{b})ᵣ-C(O)OR₅, un cycloalkyle substitué ou non substitué, un cycloalkylalkyle substitué ou non substitué, un aryle substitué ou non substitué, un arylalkyle substitué ou non substitué, un hétéroaryle substitué ou non substitué, un hétéroarylalkyle substitué ou non substitué, un hétérocyclyle substitué ou non substitué et un hétérocyclylalkyle substitué ou non substitué ; ou R₆ et R₇, conjointement avec l'atome d'azote auquel ils sont liés, peuvent former un cycle cyclique à 3 à 10 chaînons, saturé ou insaturé, substitué ou non substitué, ledit cycle cyclique insaturé pouvant comporter une ou deux doubles liaisons ;
R₈ est un alkyle substitué ou non substitué ou un aryle substitué ou non substitué ;
« n » est un entier allant de 0 à 3, bornes incluses ; « p » est un entier allant de 0 à 3, bornes incluses ; « q » est un entier allant de 0 à 3, bornes incluses ; et « r » est un entier allant de 1 à 3, bornes incluses ; 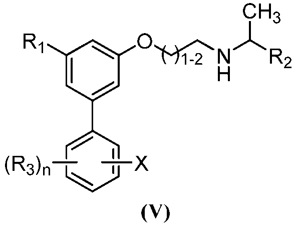
où
R₁ est choisi parmi un halogène, un alkyle substitué ou non substitué, un halogénoalkyle substitué ou non substitué, un cycloalkyle substitué ou non substitué, un alcoxy substitué ou non substitué, un halogénoalcoxy substitué ou non substitué, -(CRₐR_{b})₁₋₃OH, -C(O)NH-alkyle, -S(O)₂-alkyle, -S(O)₂NH-alkyle, -C(O)OH, -C(O)O-alkyle, -(CRₐR_{b})₁₋₃C(O)OH et -(CRₐR_{b})₁₋₃C(O)O-alkyle ;
R₂ est un phényle substitué ou non substitué ou un naphtyle substitué ou non substitué, lesdits substituants pouvant être un ou plusieurs et étant indépendamment choisis parmi un halogène, un alkyle substitué ou non substitué, un halogénoalkyle substitué ou non substitué,-OR₄, -NR₅R₆ et un cycloalkyle substitué ou non substitué ;
X est choisi parmi -C(O)OH, -C(O)O-alkyle, -C(O)NR₅R₆, -(CRₐR_{b})₁₋₃C(O)OH, -C(O)O-alkyle, -O-(CRₐR_{b})₁₋₃C(O)OH, -O-(CRₐR_{b})₁₋₃C(O)O-alkyle, -CR_{c}=CR_{c}-, -C(O)OH, un halogène, un alkyle substitué ou non substitué, un halogénoalkyle substitué ou non substitué, -OR₄ et -S(O)₂-alkyle ;
Rₐ et R_{b} sont indépendamment choisis parmi un hydrogène, un halogène, un hydroxy, un alkyle substitué ou non substitué, un halogénoalkyle substitué ou non substitué et un cycloalkyle substitué ou non substitué ; ou Rₐ et R_{b}, conjointement avec l'atome de carbone auquel ils sont liés, peuvent former un cycle carbocyclique saturé à 3 à 6 chaînons substitué ou non substitué ;
R_{c} est indépendamment choisi parmi un hydrogène, un halogène et un alkyle substitué ou non substitué ;
R₃, qui peut être identique ou différent à chaque occurrence, est indépendamment choisi parmi un halogène, un cyano, un alkyle substitué ou non substitué, un halogénoalkyle substitué ou non substitué et -OR₄ ;
R₄ est choisi parmi un hydrogène, un alkyle substitué ou non substitué, un haloagénoalkyle substitué ou non substitué et un cycloalkyle substitué ou non substitué ;
R₅ et R₆ sont indépendamment choisis parmi un hydrogène, un alkyle substitué ou non substitué et un cycloalkyle substitué ou non substitué ; et « n » est un entier compris entre 0 et 2, bornes incluses ; 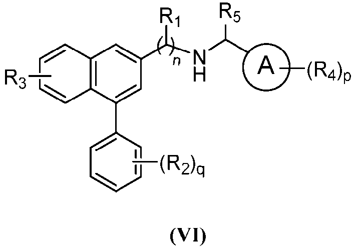
où
le cycle A est un phényle ou un naphtyle ;
R₁ est un hydrogène ou un alkyle en (C₁-C₆) substitué ou non substitué ;
R₂, qui peut être identique ou différent à chaque occurrence, est indépendamment choisi parmi un halogène, un cyano, un alkyle en (C₁-C₆) substitué ou non substitué, un halogénoalkyle en (C₁-C₆) substitué ou non substitué, un hydroxyalkyle en (C₁-C₆) substitué ou non substitué, - X-C(O)-Z, -OR₉, -NR₇R₈, -NR₇C(O)R₆, -S(O)₀₋₂R₆, -S(O)₂NR₇R₈, -NR₇S(O)₂R₆, un cycloalkyle en (C₃-C₁₂) substitué ou non substitué, un phényle substitué ou non substitué, un hétéroaryle à 5 ou 6 chaînons substitué ou non substitué, un hétérocyclyle à 5 ou 6 chaînons substitué ou non et un cycle D ;
le cycle D est 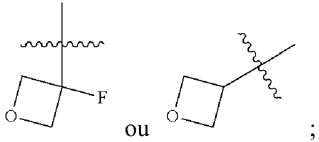
X est choisi parmi une liaison, -(CRₐR_{b})ₘ-, -NR₂-, -O(CRₐR_{b})ₘ-, -(CRₐR_{b})ₘO-, -C(O)NR12-, -(CRₐR_{b})ₘO-(CRₐR_{b})ₘ- et -C(O)NR₁₂(CRₐR_{b})ₘ- ;
Rₐ et R_{b} qui peuvent être identiques ou différents à chaque occurrence, sont indépendamment choisis parmi un hydrogène, un halogène, un hydroxy, un alkyle en (C₁-C₆) substitué ou non substitué, un halogénoalkyle en (C₁-C₆) substitué ou non substitué et un cycloalkyle en (C₃-C₆) substitué ou non substitué ; ou Rₐ et R_{b}, conjointement avec l'atome de carbone auquel ils sont liés, forment un cycle carbocyclique saturé à 3 à 6 chaînons substitué ou non substitué ;
Z est -OR₁₀ ou -NR₇R₈ ;
R₃ est choisi parmi un hydrogène, un halogène, un alkyle en (C₁-C₆) substitué ou non substitué, un halogénoalkyle en (C₁-C₆) substitué ou non substitué, -OR₉ et un cycloalkyle en (C₃-C₁₂) substitué ou non substitué ;
R₄, qui peut être identique ou différent à chaque occurrence, est indépendamment choisi parmi un halogène, un alkyle en (C₁-C₆) substitué ou non substitué, un halogénoalkyle en (C₁-C₆) substitué ou non substitué, un alcoxyalkyle en (C₁-C₆) substitué ou non substitué, -SF₅ et -OR₉ ;
R₅ est un alkyle en (C₁-C₆) substitué ou non substitué ;
R₆ est choisi parmi un alkyle en (C₁-C₆) substitué ou non substitué, un cycloalkyle en (C₃-C₁₂) substitué ou non substitué et un aryle en (C₆-C₁₄) substitué ou non substitué ;
R₇ et R₈, qui peuvent être identiques ou différents à chaque occurrence, sont indépendamment choisis parmi un hydrogène, un alkyle en (C₁-C₆) substitué ou non substitué, - (CRₐR_{b})₁₋₂R₁₁, -(CR_{c}R_{d})ₘ-OH et un cycloalkyle en (C₃-C₁₂) substitué ou non substitué ;
R_{c} et R_{d} qui peuvent être identiques ou différents à chaque occurrence, sont indépendamment un hydrogène ou un alkyle en (C₁-C₆) substitué ou non substitué ;
R₉ est indépendamment choisi parmi un hydrogène, un alkyle en (C₁-C₆) substitué ou non substitué, un halogénoalkyle en (C₁-C₆) substitué ou non substitué, un alcoxyalkyle en (C₁-C₆) substitué ou non substitué et un cycloalkyle en (C₃-C₁₂) substitué ou non substitué ;
R₁₀ est choisi parmi un hydrogène, un alkyle en (C₁-C₆) substitué ou non substitué et - (CRₐR_{b})₁₋₂phényle ;
R₁₁ est un phényle substitué ou non substitué, lesdits substituants étant choisis parmi un halogène, un alkyle en (C₁-C₆) et -OR₉ ;
R₁₂ est un hydrogène ou un alkyle en (C₁-C₆) substitué ou non substitué ;
« m » est un entier allant de 1 à 3, bornes incluses ; « n » est un entier allant de 1 à 3, bornes incluses ; « p » est un entier allant de 0 à 3, bornes incluses ; et « q » est un entier allant de 1 à 3, bornes incluses ;
ou des sels acceptables pharmaceutiquement de ceux-ci, ou une combinaison de ceux-ci, et au moins deux excipients acceptables pharmaceutiquement, lesdits au moins deux excipients acceptables pharmaceutiquement étant la cellulose microcristalline, la crospovidone, l'amidon, le stéarate de magnésium, le laurylsulfate de sodium, le dioxyde de silicium colloïdal ou une combinaison de ceux-ci ; et
ladite composition pharmaceutique solide étant appropriée pour une administration orale et se présentant sous la forme d'un comprimé ou d'une capsule.

2. Composition pharmaceutique solide de la revendication 1, dans laquelle le ou les composés sont :
l'acide 3-((S)-2-((((R)-1-(naphtalén-1-yl)éthyl)amino)méthyl)morpholino)-5-(trifluorométhyl)benzoïque, un sel interne ou un sel d'acide chlorhydrique de celui-ci (« Composé 1 ») ;
l'acide 2-méthyl-5-((S)-2-(2-(((R)-1-(naphtalén-1-yl)éthyl)amino)éthyl)-2H-benzo[b][1,4]oxazin-4(3H)-yl)benzoïque, un sel interne ou un sel d'acide chlorhydrique de celui-ci (« Composé 2 ») ;
le chlorhydrate de l'acide 2-méthyl-5-((2R,4S)-2-((((R)-1-(naphtalén-1-yl)éthyl)amino)méthyl)chroman-4-yl)benzoïque (« Composé 3 ») ;
l'acide 3-((1R,3S)-3-((((R)-1-(4-fluoro-3-méthoxyphényl)éthyl)amino)méthyl)-1,2,3,4-tétrahydronaphtalén-1-yl)-2,6-diméthylbenzoïque, un sel interne ou un sel d'acide chlorhydrique de celui-ci (« Composé 4 ») ;
l'acide (R)-3-(4-fluoro-3'-(2-((1-(3-méthoxyphényl)éthyl)amino)éthoxy)-5'-(trifluorométhyl)-[1,1'-biphényl]-3-yl)propanoïque, un sel interne ou un sel d'acide chlorhydrique de celui-ci (« Composé 5 ») ;
le chlorhydrate de (R)-N-((R)-1-(3-méthoxyphényl)éthyl)-1-(4-(4-(trifluorométhyl)phényl)naphtalén-2-yl)propan-1-amine (« Composé 6 ») ; ou
une combinaison de ceux-ci.

3. Composition pharmaceutique solide de la revendication 1 ou 2, dans laquelle le ou les composés sont le Composé 3.

4. Composition pharmaceutique solide de l'une quelconque des revendications 1 à 3, ladite composition se présentant sous la forme d'un comprimé, ledit comprimé étant enrobé d'un film.

5. Composition pharmaceutique solide de la revendication 4, dans laquelle le film est un amidon résistant, un amidon de maïs à haute teneur en amylose, le poly(acide méthacrylique-co-méthacrylate de méthyle) 1:2, le poly(acrylate d'éthyle-co-méthacrylate de méthyle-co-chlorure de triméthylammonioéthyl méthacrylate) 1:2:0,1, le poly(acrylate d'éthyle-co-méthacrylate de méthyle-co-chlorure de triméthylammonioéthyle méthacrylate) 1:2:0,2, l'acétate d'amidon, l'éthylcellulose, la croscarmellose sodique, le glycolate d'amidon sodique, un éther de cellulose, un polymère vinylique, un glycol, un polymère acrylique, la maltodextrine, la polydextrose, le phtalate d'acétate de cellulose, le trimellitate d'acétate de cellulose, le succinate d'acétate d'hydroxypropylméthylcellulose succinate d'acétate d'hypromellose, le phtalate d'hydroxypropylméthylcellulose, le phtalate d'acétate de polyvinyle, le poly(acide méthacrylique-co-méthacrylate de méthyle), la gomme laque, ou une combinaison de ceux-ci.

6. Composition pharmaceutique solide comprenant un ou plusieurs composés et un ou plusieurs ingrédients, dans laquelle le ou les composés sont le Composé 1, le Composé 2, le Composé 3, le Composé 4, le Composé 5, le Composé 6, un sel acceptable pharmaceutiquement de ceux-ci ou une combinaison de ceux-ci, dans laquelle le ou les ingrédients sont :
(a) une cellulose microcristalline en une quantité de 5 % à 50 % en poids du poids total de la composition pharmaceutique solide ;
(b) la crospovidone en une quantité de 5 % à 20 % en poids du poids total de la composition pharmaceutique solide, ou la povidone en une quantité de 3 % à 20 % en poids du poids total de la composition pharmaceutique solide, ou une combinaison de celles-ci ;
(c) un amidon prégélatinisé en une quantité de 20 % à 50 % en poids du poids total de la composition pharmaceutique solide ;
(d) le stéarate de magnésium en une quantité de 0,5 % à 5 % en poids du poids total de la composition pharmaceutique solide, ou le talc en une quantité de 0,5 % à 2 % en poids du poids total de la composition pharmaceutique solide, ou une combinaison de ceux-ci ;
(e) le laurylsulfate de sodium (SLS) en une quantité de 0,5 % à 3 % en poids du poids total de la composition pharmaceutique solide ;
(f) le dioxyde de silicium colloïdal en une quantité de 0,5 % à 3 % en poids du poids total de la composition pharmaceutique solide ; ou
(g) une combinaison de ceux-ci.

7. Composition pharmaceutique solide comprenant le chlorhydrate de l'acide 2-méthyl-5-((2R,4S)-2-((((R)-1-(naphtalén-1-yl)éthyl)amino)méthyl)chroman-4-yl)benzoïque et un ou plusieurs ingrédients, dans lequel le ou les ingrédients sont :
(a) une cellulose microcristalline en une quantité de 5 % à 50 % en poids du poids total de la composition pharmaceutique solide ;
(b) la crospovidone en une quantité de 5 % à 20 % en poids du poids total de la composition pharmaceutique solide, ou la povidone en une quantité de 3 % à 20 % en poids du poids total de la composition pharmaceutique solide, ou une combinaison de celles-ci ;
(c) un amidon prégélatinisé en une quantité de 20 % à 50 % en poids du poids total de la composition pharmaceutique solide ;
(d) le stéarate de magnésium en une quantité de 0,5 % à 5 % en poids du poids total de la composition pharmaceutique solide, ou le talc en une quantité de 0,5 % à 2 % en poids du poids total de la composition pharmaceutique solide, ou une combinaison de ceux-ci ;
(e) le laurylsulfate de sodium (SLS) en une quantité de 0,5 % à 3 % en poids du poids total de la composition pharmaceutique solide ;
(f) le dioxyde de silicium colloïdal en une quantité de 0,5 % à 3 % en poids du poids total de la composition pharmaceutique solide ; ou
(g) une combinaison de ceux-ci.

8. Composition pharmaceutique solide de l'une quelconque des revendications 1 à 6, dans laquelle le ou les composés sont en une quantité de 0,1 mg à 200 mg.

9. Composition pharmaceutique solide de l'une quelconque des revendications 1 à 6, dans laquelle le ou les composés sont en quantité de 1 mg à 50 mg, ou de 5 mg à 25 mg.

10. Composition pharmaceutique solide de la revendication 7, dans laquelle le chlorhydrate de l'acide 2-méthyl-5-((2R,4S)-2-((((R)-1-(naphtalén-1-yl)éthyl)amino)méthyl)chroman-4-yl)benzoïque est présent en une quantité de 0,1 mg à 200 mg, 1 mg à 50 mg ou 5 mg à 25 mg.

11. Composition pharmaceutique solide de l'une quelconque des revendications 1 à 3, ladite composition pharmaceutique étant une capsule, ladite capsule comprenant 35 % p/p à 39 % p/p du ou des composés.

12. Composition pharmaceutique solide telle que décrite dans l'une quelconque des revendications 1 à 11, destinée à être utilisée dans le traitement de l'hyperparathyroïdie secondaire associée à une maladie rénale chronique (MRC) chez un sujet en ayant besoin.

13. Composition pharmaceutique solide destinée à être utilisée selon la revendication 12, dans laquelle le composé dans la composition pharmaceutique solide est le chlorhydrate de l'acide 2-méthyl-5-((2R,4S)-2-((((R)-1-(naphtalène-1-yl)éthyl)amino)méthyl)chroman-4-yl)benzoïque.

14. Composition pharmaceutique solide destinée à être utilisée selon la revendication 12 ou la revendication 13, ladite utilisation comprenant l'administration du ou des composés à une dose quotidienne totale de 5 mg à 50 mg.

15. Composition pharmaceutique solide destinée à être utilisée selon l'une quelconque de la revendication 14, ladite administration étant de 25 mg une ou deux fois par jour, ou de 20 mg une ou deux fois par jour.

16. Composition pharmaceutique solide destinée à être utilisée selon l'une quelconque des revendications 12 à 15, ladite utilisation comprenant l'administration orale de ladite composition pharmaceutique solide.

17. Composition pharmaceutique solide destinée à être utilisée selon l'une quelconque des revendications 12 à 16, ladite utilisation ne comprenant pas de titration de dose.

18. Composition pharmaceutique solide destinée à être utilisée selon l'une quelconque des revendications 12 à 17, ledit sujet n'étant pas sous dialyse.

19. Composition pharmaceutique solide destinée à être utilisée selon l'une quelconque des revendications 12 à 17, ledit sujet étant sous dialyse.

20. Composition pharmaceutique solide destinée à être utilisée selon l'une quelconque des revendications 12 à 19, ladite hyperparathyroïdie secondaire étant traitée ou prise en charge sans induire d'hyperphosphatémie ou d'hypocalcémie chez le sujet.

21. Composition pharmaceutique solide destinée à être utilisée selon l'une quelconque des revendications 12 à 20, ledit sujet ayant été préalablement diagnostiqué ou présentant une MRC de stade 3b, une MRC de stade 4 ou une MRC de stade 5.

22. Composition pharmaceutique solide telle que décrite dans l'une quelconque des revendications 1 à 11, destinée à être utilisée pour abaisser ou supprimer un ou plusieurs niveaux d'hormone parathyroïdienne intacte (iPTH) chez un sujet souffrant d'hyperparathyroïdie secondaire associée à une MRC.

23. Composition pharmaceutique solide destinée à être utilisée selon la revendication 22, dans laquelle le composé dans la composition pharmaceutique solide est le chlorhydrate de l'acide 2-méthyl-5-((2R,4S)-2-((((R)-1-(naphtalén-1-yl)éthyl)amino)méthyl)chroman-4-yl)benzoïque.

24. Composition pharmaceutique solide destinée à être utilisée selon la revendication 22 ou 23, ledit sujet n'étant pas sous dialyse.

25. Composition pharmaceutique solide destinée à être utilisée selon la revendication 22 ou 23, ledit sujet étant sous dialyse.

26. Composition pharmaceutique solide destinée à être utilisée selon l'une quelconque des revendications 22 à 25, ladite utilisation comprenant l'administration du ou des composés en une quantité de dose quotidienne totale de 5 mg à 50 mg.

27. Composition pharmaceutique solide destinée à être utilisée selon la revendication 26, ladite dose quotidienne totale étant comprise entre 10 mg et 40 mg.

28. Composition pharmaceutique solide destinée à être utilisée selon la revendication 26, ladite dose quotidienne totale étant administrée à 25 mg une ou deux fois par jour, ou à 20 mg une ou deux fois par jour.

29. Composition pharmaceutique solide destinée à être utilisée selon l'une quelconque des revendications 22 à 27, ladite utilisation comprenant l'administration orale de ladite composition pharmaceutique solide.

30. Composition pharmaceutique solide destinée à être utilisée selon l'une quelconque des revendications 22 à 29, ladite administration ne nécessitant pas d'ajustement de dose pour le sujet.

31. Composition pharmaceutique solide destinée à être utilisée selon l'une quelconque des revendications 22 à 30, ledit niveau d'iPTH du sujet étant réduit d'au moins 30 % par rapport au niveau de concentration d'iPTH de référence avant traitement.

32. Composition pharmaceutique solide destinée à être utilisée selon l'une quelconque des revendications 22 à 30, ledit niveau d'iPTH du sujet étant réduit d'au moins 15 % par rapport au niveau de concentration d'iPTH de référence avant traitement.

33. Composition pharmaceutique solide d'acide 2-méthyl-5-((2R,4S)-2-((((R)-1-(naphtalén-1-yl)éthyl)amino)méthyl)chroman-4-yl)benzoïque, sel de chlorhydrate pour une utilisation dans le traitement ou la prise en charge de l'hyperparathyroïdie secondaire associée à une MRC.

34. Composition pharmaceutique solide d'acide 2-méthyl-5-((2R,4S)-2-((((R)-1-(naphthalén-1-yl)éthyl)amino)méthyl)chroman-4-yl)benzoïque, sel de chlorhydrate pour une utilisation dans le traitement ou la prise en charge de l'hyperparathyroïdie secondaire associée à une MRC en abaissant ou en supprimant un pour plusieurs niveaux d'iPTH chez un sujet.

35. Composition pharmaceutique solide d'acide 2-méthyl-5-((2R,4S)-2-((((R)-1-(naphtalén-1-yl)éthyl)amino)méthyl)chroman-4-yl)benzoïque, sel de chlorhydrate pour une utilisation selon la revendication 34, ledit niveau d'iPTH du sujet étant réduit d'au moins 30 % par rapport au niveau de concentration d'iPTH de référence avant traitement.

36. Composition pharmaceutique solide d'acide 2-méthyl-5-((2R,4S)-2-((((R)-1-(naphtalén-1-yl)éthyl)amino)méthyl)chroman-4-yl)benzoïque, sel de chlorhydrate pour une utilisation selon la revendication 34, ledit niveau d'iPTH du sujet étant réduit d'au moins 15 % par rapport au niveau de concentration d'iPTH de référence avant traitement.
